# EUROPEAN PATENT APPLICATION

(11) **EP 4 527 838 A1**
(43) Date of publication of application: **26.03.2025**
(21) Application number: 23806749.0
(22) Date of filing: 08.05.2023
(51) Int. Cl.: C07D 491/052, C07D 519/00, A61K 31/407, A61P 7/02

(54) **HETEROCYCLIC COMPOUND, PREPARATION METHOD THEREFOR AND APPLICATION THEREOF**

(30) Priority: 18.05.2022 CN 202210547956; 30.09.2022 CN 202211216556
(71) Applicant: Sichuan Kelun-Biotech Biopharmaceutical Co., Ltd., Chengdu, Sichuan 611138 (CN)
(72) Inventor: TIAN, Qiang, Chengdu, Sichuan 611138 (CN); ZHANG, Yitao, Chengdu, Sichuan 611138 (CN); YE, Jian, Chengdu, Sichuan 611138 (CN); LIAO, Menchang, Chengdu, Sichuan 611138 (CN); SU, Donghai, Chengdu, Sichuan 611138 (CN); WANG, Haitao, Chengdu, Sichuan 611138 (CN); SONG, Hongmei, Chengdu, Sichuan 611138 (CN); GE, Junyou, Chengdu, Sichuan 611138 (CN)
(74) Representative: Hoffmann Eitle
(86) International application number: PCT/CN2023/092727
(87) International publication number: WO 2023/221797

(57) **Abstract**

The present invention relates to a heterocyclic compound, a pharmaceutical composition comprising same, and a preparation method for the heterocyclic compound, and further relates to a use of the compound in the preparation of a drug for treating thromboembolic diseases.

## Description

### Technical Field

The invention belongs to the pharmaceutical field, in particular to a heterocyclic compound, a pharmaceutical composition comprising the same, and a process for preparing the same, and to the use of such a compound in the manufacture of a medicament for the treatment of thromboembolic diseases.

### Background of the Invention

Stroke is an acute cerebrovascular disease, including ischemic stroke and hemorrhagic stroke, in which the the incidence of ischemic stroke accounts for approximately 80% of all stroke cases (International Journal of Cardiology, 2016, 222, 441-447).

Intravenous tissue plasminogen activator (t-PA), as a high molecular weight compound, has been the only recommended therapy for ischemic stroke for nearly two decades, but t-PA has a short therapeutic window (< 4.5 h) and there is a risk of promoting intracerebral hemorrhage (New England Journal of Medicine, 2008, 48 (13), 1634-1635). Recombinant tissue plasminogen activator (rt-PA) has a half-life of only 3-5 minutes and requires continuous intravenous infusion, and for stroke resulting from acute occlusion of large vessels, the recanalization rate after intravenous thrombolysis with rt-PA does not exceed 25% (Stroke, 2010, 41, 2254-2258).

SMTP series compounds are a series of compounds with a triisoprenylphenol (TP) structure produced by microbial fermentation from the filamentous fungus *Stachybotrys microspora* and purification (JP2002065288A). This class of compounds, such as orniplabin, are known as plasminogen modulators that, by relaxing the plasminogen conformation, can accelerate plasminogen activation mediated by urokinase-type plasminogen activator (u-PA) or t-PA, rapidly elevate plasmin levels and promote thrombolysis by plasmin (The FEBS Journal, 2010, 277, 3675-3687). Because SMTP series compounds do not directly activate plasminogen and do not affect u-PA or t-PA levels, the potential adverse risk of intracerebral hemorrhage which may occur in thrombolytic agents with traditional mechanisms of action can be mechanistically avoided (Naunyn-Schmiedeberg's Archives of Pharmacology, 2010, 382, 245-253). In addition to the plasminogen conformational regulatory function, SMTP series compounds have soluble epoxide hydrolase (sEH) inhibitory activity independent of their thrombolytic activity. sEH is a bifunctional enzyme involved in important physiological activities such as inflammatory responses and lipid metabolism (Journal of Biological Chemistry, 2014, 289 (52), 35826-35838). The inhibitory effect of SMTP series compounds on sEH may be related to their *in vivo* anti-inflammatory activity (International Journal of Molecular Sciences, 2021, 22, 954). Plasminogen modulators with better biological activity improved based on SMTP series compounds worth further investigation, and it is necessary to develop thrombolytic drugs having new mechanisms, prolonged therapeutic time windows, and reduced risk of bleeding.

### Summary of the Invention

Through a large number of studies, we surpringly find a class of heterocyclic compounds and their preparation methods. Such compounds have good plasminogen regulatory activity, can exert thrombolysis promotion and anti-inflammatory effects, and are used for the treatment of various thromboembolic diseases, such as ischemic stroke, etc.

In one aspect, the present invention relates to a compound of Formula I or a pharmaceutically acceptable salt, an ester, a solvate, a stereoisomer, a tautomer, a polymorph, an isotopically labeled product, a metabolite, a prodrug, or a mixture thereof: 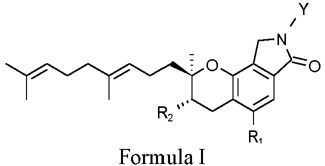 wherein,
Y is selected from the group consisting of 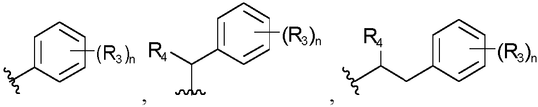 and 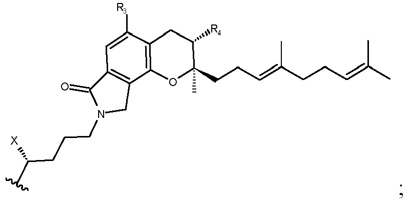
R₁ and R₃ are each independently selected from the group consisting of carboxyl, hydroxy, C₁₋₆ alkyl, C₁₋₆ alkoxy, C₁₋₆ alkoxyacyloxy, C₁₋₆ alkylacyloxy, C₁₋₆ alkoxyalkylaminoacyloxy, C₁₋₆ alkylaminoacyloxy, C₄₋₁₀ heterocyclylacyloxy, -O-C₄₋₁₀ heterocycyl, C₁₋₆ alkylsulfamoyloxy, C₄₋₁₀ heterocycylsulfonyloxy, glycosyl, -O-P(O)(OH)₂, -O-P(O)₂OH, -O-S(O)₂OH, amino, C₁₋₆ alkylamino, C₁₋₆ alkylamido, C₄₋₁₀ heterocyclylamido, halogen, cyano, C₂₋₆ alkenyl, and C₂₋₆ alkynyl; the C₄₋₁₀ heterocyclylacyloxy, -O-C₄₋₁₀ heterocycyl, C₁₋₆ alkylacyloxy, C₁₋₆ alkyl, C₁₋₆ alkoxy, or C₁₋₆ alkylaminoacyloxy being optionally substituted with one or more substituents selected from the group consisting of hydroxy, carboxyl, C₁₋₆ alkyl, C₁₋₆ alkoxy, C₁₋₆ alkylcarbonyl, C₁₋₆ alkoxycarbonyl, C₁₋₆ haloalkyl, C₁₋₆ alkoxyacyloxy, 4-10-membered heterocyclyl, C₁₋₆ hydroxyalkyl, and -O-P(O)(OH)₂;
R₂ and R₄ are each independently selected from the group consisting of hydrogen, hydroxyl, carboxyl, ester group, C₁₋₆ alkoxy, carboxyl-substituted C₁₋₆ alkylacyloxy, -OC(=O)-C₄₋₁₀ heterocyclyl, -O-P(O)₂OH, -O-S(O)₂OH, and -O-S(O)₂NH₂, the C₄₋₁₀ heterocycyl being optionally substituted with one or more substituents selected from C₁₋₆ alkyl;
R₁ and R₂ are not simultaneously hydroxyl when Y is selected from 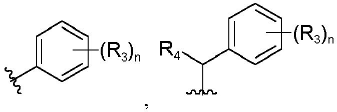 or 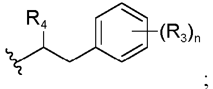
n = 0, 1, 2, 3, 4, or 5;
X is selected from the group consisting of -CO₂H and its carboxylic acid isosteres, -CO₂C₁₋₆ alkyl, -C(=O)SH, 4-10-membered heteroaryl, choline carboxylate, -C(=O)NHC₁₋₆ alkyl, -C(=O)NHS(=O)₂C₁₋₆ alkylamino and -C(=O)NHS(=O)₂C₁₋₆ alkyl, the -CO₂C₁₋₆ alkyl, 4-10 membered heteroaryl, choline carboxylate, -C(=O)NHC₁₋₆ alkyl, -C(=O)NHS(=O)₂C₁₋₆ alkylamino and -C(=O)NHS(=O)₂C₁₋₆ alkyl being optionally substituted with one or more substituents selected from the group consisting of C₄₋₁₀ heterocyclyl, C₁₋₆ alkoxy, 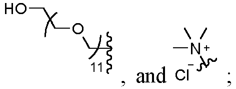 and
when X is -CO₂H, R₁, R₂, R₃ and R₄ are not simultaneously hydroxyl;
where the wavy line " 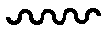 " represents the attachment point to the remainder of the molecule.

In another aspect, the present invention relates to a pharmaceutical composition comprising a therapeutically effective amount of the compound of the invention, or a pharmaceutically acceptable salt, an ester, a solvate, a stereoisomer, a tautomer, a polymorph, an isotopically labeled product, a metabolite, a prodrug, or a mixture thereof, and one or more pharmaceutically acceptable carriers thereof, and optionally further comprising one or more additional drugs for the treatment of thromboembolic diseases.

In another aspect, the present invention relates to use of the compound of the invention, or a pharmaceutically acceptable salt, an ester, a solvate, a stereoisomer, a tautomer, a polymorph, an isotopically labeled product, a metabolite, a prodrug, or a mixture thereof, or the pharmaceutical composition of the present invention in the manufacture of a medicament for the treatment of thromboembolic diseases.

In another aspect, the present invention relates to the compound of the invention, or a pharmaceutically acceptable salt, an ester, a solvate, a stereoisomer, a tautomer, a polymorph, an isotopically labeled product, a metabolite, a prodrug, or a mixture thereof, or the pharmaceutical composition of the invention, for use in the treatment of thromboembolic diseasess.

In another aspect, the invention relates to a method of treating thromboembolic diseases comprising administering to a subject in need thereof a therapeutically effective amount of the compound of the invention, or a pharmaceutically acceptable salt, an ester, a solvate, a stereoisomer, a tautomer, a polymorph, an isotopically labeled product, a metabolite, a prodrug, or a mixture thereof, or the pharmaceutical composition of the invention.

In another aspect, the present invention relates to a process for preparing the compound of the invention comprising the steps as shown in the following scheme: 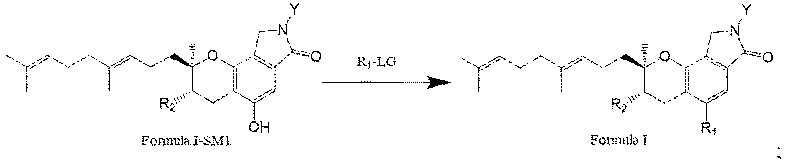 wherein:
R₁, R₂ and Y are as defined above; and
LG is aleaving group.

### Brief Description of the Drawings

Figure 1: a thromboelastogram of the compound 2-1 and compound 2-2 groups.
Figure 2: a thromboelastogram of the compound 2-5 group.
Figure 3: a thromboelastogram of the compound 2-5 and compound 2-6 groups.

### Detailed Description of the Invention

### Compound and preparation method

An object of the present invention is to provide a compound of Formula I or a pharmaceutically acceptable salt, an ester, a solvate, a stereoisomer, a tautomer, a polymorph, an isotopically labeled product, a metabolite, a prodrug, or a mixture thereof: 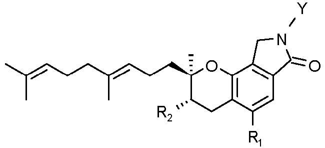 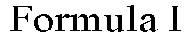 wherein,
Y is selected from the group consisting of 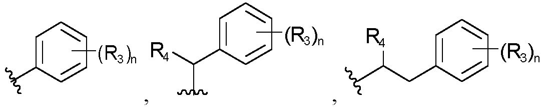 and 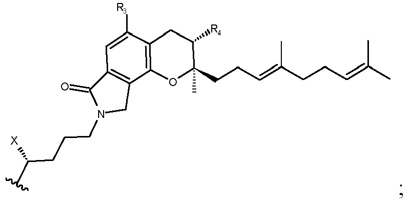
R₁ and R₃ are each independently selected from the group consisting of carboxyl, hydroxy, C₁₋₆ alkyl, C₁₋₆ alkoxy, C₁₋₆ alkoxyacyloxy, C₁₋₆ alkylacyloxy, C₁₋₆ alkoxyalkylaminoacyloxy, C₁₋₆ alkylaminoacyloxy, C₄₋₁₀ heterocyclylacyloxy, -O-C₄₋₁₀ heterocycyl, C₁₋₆ alkylsulfamoyloxy, C₄₋₁₀ heterocycylsulfonyloxy, glycosyl, -O-P(O)(OH)₂, -O-P(O)₂OH, -O-S(O)₂OH, amino, C₁₋₆ alkylamino, C₁₋₆ alkylamido, C₄₋₁₀ heterocyclylamido, halogen, cyano, C₂₋₆ alkenyl, and C₂₋₆ alkynyl; the C₄₋₁₀ heterocyclylacyloxy, -O-C₄₋₁₀ heterocycyl, C₁₋₆ alkylacyloxy, C₁₋₆ alkyl, C₁₋₆ alkoxy, or C₁₋₆ alkylaminoacyloxy being optionally substituted with one or more substituents selected from the group consisting of hydroxy, carboxyl, C₁₋₆ alkyl, C₁₋₆ alkoxy, C₁₋₆ alkylcarbonyl, C₁₋₆ alkoxycarbonyl, C₁₋₆ haloalkyl, C₁₋₆ alkoxyacyloxy, 4-10-membered heterocyclyl, C₁₋₆ hydroxyalkyl, and -O-P(O)(OH)₂;
R₂ and R₄ are each independently selected from the group consisting of hydrogen, hydroxyl, carboxyl, ester group, C₁₋₆ alkoxy, carboxyl-substituted C₁₋₆ alkylacyloxy, -OC(=O)-C₄₋₁₀ heterocyclyl, -O-P(O)₂OH, -O-S(O)₂OH, and -O-S(O)₂NH₂, the C₄₋₁₀ heterocycyl being optionally substituted with one or more substituents selected from C₁₋₆ alkyl;
R₁ and R₂ are not simultaneously hydroxyl when Y is selected from 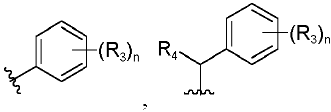 or 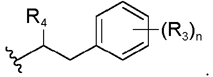
n = 0, 1, 2, 3, 4, or 5;
X is selected from the group consisting of -CO₂H and its carboxylic acid isosteres, -CO₂C₁₋₆ alkyl, -C(=O)SH, 4-10-membered heteroaryl, choline carboxylate, -C(=O)NHC₁₋₆ alkyl, -C(=O)NHS(=O)₂C₁₋₆ alkylamino and -C(=O)NHS(=O)₂C₁₋₆ alkyl, the -CO₂C₁₋₆ alkyl, 4-10 membered heteroaryl, choline carboxylate, -C(=O)NHC₁₋₆ alkyl, -C(=O)NHS(=O)₂C₁₋₆ alkylamino and -C(=O)NHS(=O)₂C₁₋₆ alkyl being optionally substituted with one or more substituents selected from the group consisting of C₄₋₁₀ heterocyclyl, C₁₋₆ alkoxy, 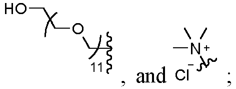 and
when X is -CO₂H, R₁, R₂, R₃ and R₄ are not simultaneously hydroxyl;
where the wavy line " 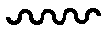 " represents the attachment point to the remainder of the molecule.

In some embodiments, R₁ and R₃ are each independently selected from the group consisting of carboxyl, hydroxy, C₁₋₆ alkyl, C₁₋₆ alkoxy, C₁₋₆ alkoxyacyloxy, C₁₋₆ alkylacyloxy, C₁₋₆ alkoxyalkylaminoacyloxy, C₁₋₆ alkylaminoacyloxy, C₄₋₁₀ heterocyclylacyloxy, -O-C₄₋₁₀ heterocycyl, C₁₋₆ alkylsulfamoyloxy, C₄₋₁₀ heterocycylsulfonyloxy, glycosyl, -O-P(O)(OH)₂, -O-P(O)₂OH, -O-S(O)₂OH, amino, C₁₋₆ alkylamino, C₁₋₆ alkylamido, C₄₋₁₀ heterocyclylamido, halogen, cyano, C₂₋₆ alkenyl, and C₂₋₆ alkynyl; the C₄₋₁₀ heterocyclylacyloxy, -O-C₄₋₁₀ heterocycyl, C₁₋₆ alkylacyloxy, C₁₋₆ alkyl, C₁₋₆ alkoxy, or C₁₋₆ alkylaminoacyloxy being optionally substituted with one or more substituents selected from the group consisting of hydroxy, carboxyl, C₁₋₆ alkyl, C₁₋₆ alkoxy, C₁₋₆ alkylcarbonyl, C₁₋₆ haloalkyl, C₁₋₆ alkoxyacyloxy, C₁₋₆ alkyl-C(=O)-, 4-10-membered heterocyclyl, C₁₋₆ hydroxyalkyl, and -O-P(O)(OH)₂.

In some embodiments, n = 1, 2, 3, 4, or 5.

In some embodiments, R₁ and R₂ are not simultaneously hydroxyl.

Another object of the invention is to provide a compound of Formula I or a pharmaceutically acceptable salt, an ester, a solvate, a stereoisomer, a tautomer, a polymorph, an isotopically labeled product, a metabolite, a prodrug, or a mixture thereof: 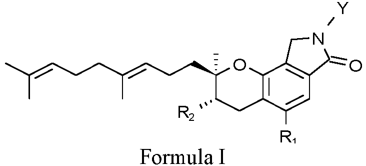 wherein,
Y is selected from the group consisting of 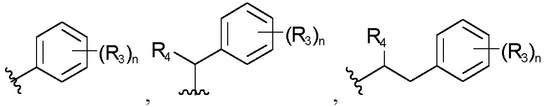 and 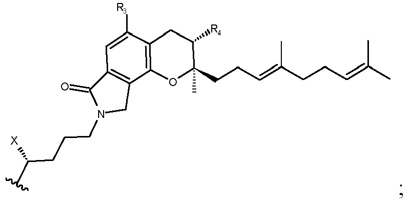
R₁ and R₃ are each independently selected from the group consisting of hydroxy, C₁₋₆ alkoxy, C₁₋₆ alkoxyacyloxy, C₁₋₆ alkylacyloxy, C₁₋₆ alkoxyalkylaminoacyloxy, C₁₋₆ alkylaminoacyloxy, C₄₋₁₀ heterocyclylacyloxy, C₁₋₆ alkylsulfamoyloxy, C₄₋₁₀ heterocycylsulfonyloxy, glycosyl, -O-P(O)₂OH, -O-S(O)₂OH, amino, C₁₋₆ alkylamino, C₁₋₆ alkylamido, C₄₋₁₀ heterocyclylamido, halogen, cyano, C₂₋₆ alkenyl, and C₂₋₆ alkynyl; preferably, R₁ is selected from the group consisting of C₁₋₆ alkylaminoacyloxy and C₄₋₁₀ heterocyclylacyloxy; and R₃ is selected from the group consisting of hydrogen, hydroxy, C₁₋₆ alkoxy, and carboxyl;
R₂ and R₄ are each independently selected from the group consisting of hydrogen, hydroxyl, carboxyl, ester group, C₁₋₆ alkoxy, carboxyl-substituted C₁₋₆ alkylacyloxy, -O-P(O)₂OH and -O-S(O)₂OH; preferably, R₂ is hydroxyl; and R₄ is selected from the group consisting of carboxyl and ester group;
R₁ and R₂ are not simultaneously hydroxyl;
n = 0, 1, 2, 3, 4, or 5; and
X is selected from the group consisting of -CO₂H and its carboxylic acid isosteres, -CO₂C₁₋₆ alkyl, choline carboxylate, -C(=O)NHC₁₋₆ alkyl, and -C(=O)NHS(=O)₂C₁₋₆ alkyl; preferably, X is -CO₂H;
where the wavy line " 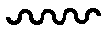 " represents the attachment point to the remainder of the molecule.

Another object of the invention is to provide a compound of Formula I or a pharmaceutically acceptable salt, an ester, a solvate, a stereoisomer, a tautomer, a polymorph, an isotopically labeled product, a metabolite, a prodrug, or a mixture thereof: 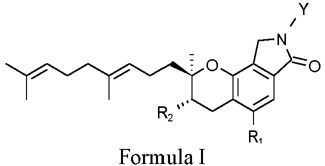 wherein,
Y is selected from the group consisting of 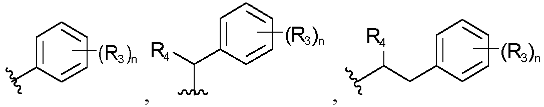 and 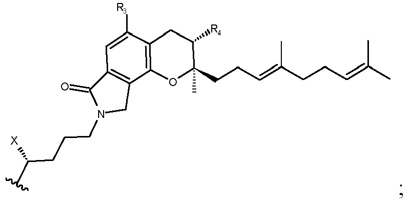
R₁ and R₃ are each independently selected from the group consisting of carboxyl, hydroxy, C₁₋₆ alkyl, C₁₋₆ alkoxy, C₁₋₆ alkoxyacyloxy, C₁₋₆ alkylacyloxy, C₁₋₆ alkoxyalkylaminoacyloxy, C₁₋₆ alkylaminoacyloxy, C₄₋₁₀ heterocyclylacyloxy, -O-C₄₋₁₀ heterocycyl, C₁₋₆ alkylsulfamoyloxy, C₄₋₁₀ heterocycylsulfonyloxy, glycosyl, -O-P(O)(OH)₂, -O-P(O)₂OH, -O-S(O)₂OH, amino, C₁₋₆ alkylamino, C₁₋₆ alkylamido, C₄₋₁₀ heterocyclylamido, halogen, cyano, C₂₋₆ alkenyl, and C₂₋₆ alkynyl; the C₄₋₁₀ heterocyclylacyloxy, -O-C₄₋₁₀ heterocycyl, C₁₋₆ alkylacyloxy, C₁₋₆ alkyl, C₁₋₆ alkoxy, or C₁₋₆ alkylaminoacyloxy being optionally substituted with one or more substituents selected from the group consisting of hydroxy, carboxyl, C₁₋₆ alkyl, C₁₋₆ alkoxy, C₁₋₆ alkylcarbonyl, C₁₋₆ haloalkyl, C₁₋₆ alkoxyacyloxy, C₁₋₆ alkyl-C(=O)-, 4-10-membered heterocyclyl, C₁₋₆ hydroxyalkyl, and -O-P(O)(OH)₂;
R₂ and R₄ are each independently selected from the group consisting of hydrogen, hydroxyl, carboxyl, ester group, C₁₋₆ alkoxy, carboxyl-substituted C₁₋₆ alkylacyloxy, -OC(=O)-C₄₋₁₀ heterocyclyl, -O-P(O)₂OH, -O-S(O)₂OH, and -O-S(O)₂NH₂, the C₄₋₁₀ heterocycyl being optionally substituted with one or more substituents selected from C₁₋₆ alkyl;
R₁ and R₂ are not simultaneously hydroxyl;
n = 1, 2, 3, 4, or 5; and
X is selected from the group consisting of -CO₂H and its carboxylic acid isosteres, -CO₂C₁₋₆ alkyl, 4-10-membered heteroaryl, choline carboxylate, -C(=O)NHC₁₋₆ alkyl, -C(=O)NHS(=O)₂C₁₋₆ alkylamino and -C(=O)NHS(=O)₂C₁₋₆ alkyl, the -CO₂C₁₋₆ alkyl, 4-10 membered heteroaryl, choline carboxylate, -C(=O)NHC₁₋₆ alkyl, -C(=O)NHS(=O)₂C₁₋₆ alkylamino and -C(=O)NHS(=O)₂C₁₋₆ alkyl being optionally substituted with one or more substituents selected from the group consisting of C₄₋₁₀ heterocyclyl, C₁₋₆ alkoxy, 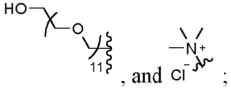
where the wavy line " 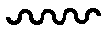 " represents the attachment point to the remainder of the molecule.

In some embodiments, the compound of Formula I of the present invention is a compound of Formula II: 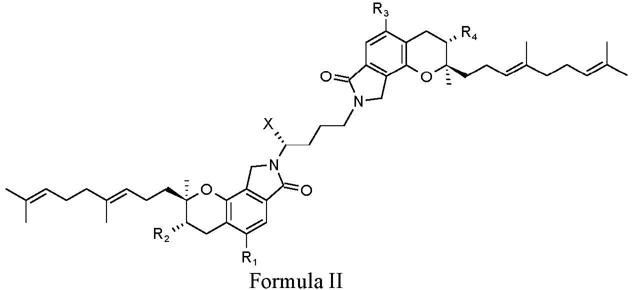 wherein,
R₁, R₂, R₃, R₄, and X are as defined above for the compound of Formula I, and
when X is -CO₂H, R₁, R₂, R₃ and R₄ are not simultaneously hydroxyl.

In some embodiments, in the compounds having the structures of Formulae I and II, R₁ and R₃ are each independently selected from the group consisting of carboxyl, hydroxy, C₁₋₆ alkoxy, C₁₋₆ alkoxyacyloxy, C₁₋₆ alkylaminoacyloxy, C₄₋₁₀ heterocyclylacyloxy, -O-C₄₋₁₀ heterocycyl, C₄₋₁₀ heterocycylsulfonyloxy, -O-P(=O)(OH)₂, -O-S(O)₂OH, and C₄₋₁₀ heterocyclylamido; the C₄₋₁₀ heterocyclylacyloxy, -O-C₄₋₁₀ heterocycyl, or C₁₋₆ alkylaminoacyloxy being optionally substituted with one or more substituents selected from the group consisting of hydroxy, carboxyl, C₁₋₆ alkyl, C₁₋₆ alkoxy, C₁₋₆ alkoxyacyloxy, C₁₋₆ alkoxycarbonyl, C₁₋₆ alkyl-C(=O)-, 4-10-membered heterocyclyl, C₁₋₆ hydroxyalkyl, and -O-P(=O)(OH)₂.

In some embodiments, in the compounds having the structures of Formulae I and II, R₁ and R₃ are each independently selected from the group consisting of carboxyl, hydroxy, C₁₋₃ alkoxy, C₁₋₃ alkoxyacyloxy, C₁₋₃ alkylaminoacyloxy, C₄₋₈ heterocyclylacyloxy, -O-C₄₋₈ heterocyclyl, C₄₋₈ heterocycylsulfonyloxy, -O-P(=O)(OH)₂, -O-S(O)₂OH, and C₄₋₈ heterocyclylamido; the C₄₋₈ heterocyclylacyloxy, -O-C₄₋₈ heterocyclyl, or C₁₋₃ alkylaminoacyloxy being optionally substituted with one or more substituents selected from the group consisting of hydroxy, carboxyl, C₁₋₃ alkyl, C₁₋₃ alkoxy, C₁₋₃ alkoxyacyloxy, C₁₋₃ alkoxycarbonyl, C₁₋₃ alkyl-C(=O)-, 4-8-membered heterocyclyl, C₁₋₃ hydroxyalkyl, and -O-P(=O)(OH)₂.

In some embodiments, in the compounds having the structures of Formulae I and II, R₁ and R₃ are each independently selected from the group consisting of carboxyl, hydroxy, -OC(=O)N(CH₃)₂, -OC(=O)-morpholinyl, -OC(=O)NHCH(CH₃)₂, -OC(=O)OCH₃, -OC(=O)-thiomorpholine dioxide, -OC(=O)-thiomorpholine monoxide, -OC(=O)-thiomorpholine, -OC(=O)N(CH₃)-tetrahydropyran, -OC(=O)-piperidine, -OSO₂-morpholine, -OC(=O)-piperazine, 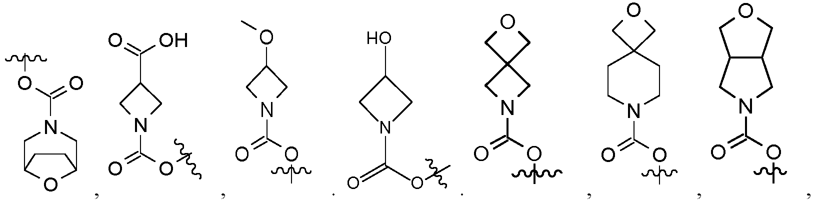 -O-tetrahydropyran, -O-P(=O)(OH)₂, methoxy, glucopyranosyl and -O-S(O)₂OH, the -OC(=O)-piperidine, -OC(=O)-piperazine, -O-tetrahydropyran, or methoxy is optionally substituted with one or more substituents selected from the group consisting of hydroxyl, carboxyl, CH₃OC(=O)-, -C(=O)CH₃, -OCH₃, piperidinyl, morpholinyl, hydroxy, -CH₂OH, -O-P(=O)(OH)₂, and oxo, where the wavy line " 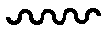 " represents the attachment point to the remainder of the molecule.

In some embodiments, in the compounds having the structures of Formulae I and II, R₁ and R₃ are each independently selected from the group consisting of carboxyl, hydroxy, -OC(=O)N(CH₃)₂, -OC(=O)-morpholinyl, -OC(=O)NHCH(CH₃)₂, -OC(=O)OCH₃, -OC(=O)-thiomorpholine dioxide, -OC(=O)-thiomorpholine monoxide, -OC(=O)-thiomorpholine, -OC(=O)N(CH₃)-tetrahydropyran, -OC(=O)-piperidine, -OSO₂-morpholine, -OC(=O)-piperazine, 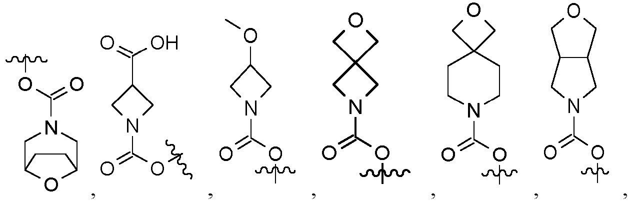 -O-tetrahydropyran, -P(=O)(OH)₂, methoxy and -O-S(O)₂OH, the -OC(=O)-piperidine, -OC(=O)-piperazine, -O-tetrahydropyran, or methoxy is optionally substituted with one or more substituents selected from the group consisting of hydroxy, carboxyl, CH₃OC(=O)-, -C(=O)CH₃, -OCH₃, piperidinyl, morpholinyl, hydroxy, -CH₂OH, and -O-P(=O)(OH)₂.

In some embodiments, in the compounds having the structures of Formulae I and II, R₁ and R₃ are each independently selected from the group consisting of carboxyl, hydroxy, -OC(=O)N(CH₃)₂, -OC(=O)NHCH(CH₃)₂, -OC(=O)OCH₃, -O-P(=O)(OH)₂, methoxy, -O-S(O)₂OH, 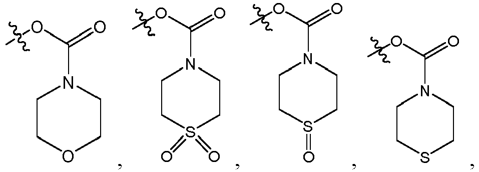 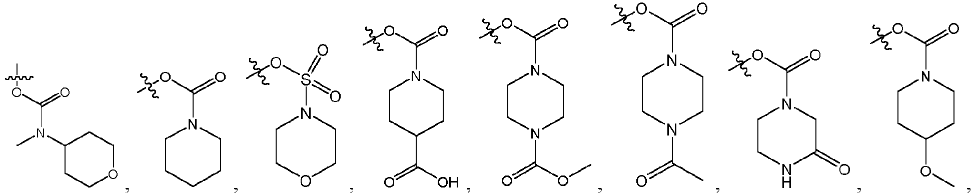 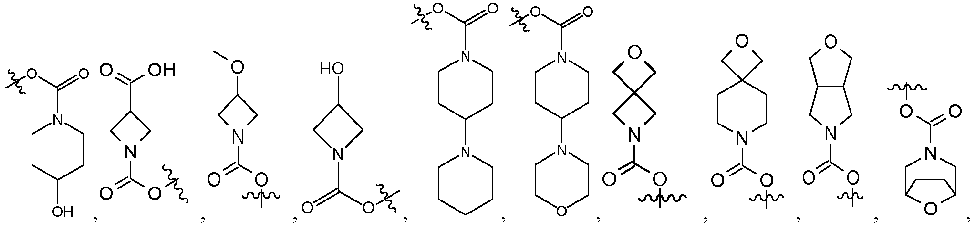 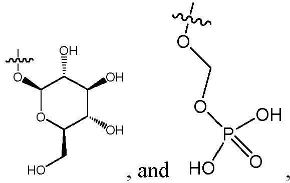 where the wavy line " 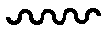 " represents the attachment point to the remainder of the molecule.

In some embodiments, in the compounds having the structures of Formulae I and II, R₁ and R₃ are each independently selected from the group consisting of hydroxyl, C₁₋₆ alkoxy, C₁₋₆ alkoxyacyloxy, C₁₋₆ alkylacyloxy, C₁₋₆ alkoxyalkylaminoacyloxy, C₁₋₆ alkylaminoacyloxy, C₄₋₆ heterocyclylacyloxy, C₁₋₆ alkylsulfamoyloxy, C₄₋₆ heterocycylsulfonyloxy, glycosyl, -O-P(O)₂OH, -O-S(O)₂OH, amino, C₁₋₆ alkylamino, C₁₋₆ alkylamido, C₄₋₆ heterocyclylamido, halogen, cyano, C₂₋₆ alkenyl, and C₂₋₆ alkynyl.

In some embodiments, in the compounds having the structures of Formulae I and II, R₁ and R₃ are each independently selected from the group consisting of C₁₋₆ alkoxy, C₁₋₆ alkoxyacyloxy, C₁₋₆ alkylacyloxy, C₁₋₆ alkoxyalkylaminoacyloxy, C₁₋₆ alkylaminoacyloxy, C₄₋₆ heterocyclylacyloxy, C₁₋₆ alkylsulfamoyloxy, C₄₋₆ heterocycylsulfonyloxy, glycosyl, -O-P(O)₂OH, -O-S(O)₂OH, amino, C₁₋₆ alkylamino, C₁₋₆ alkylamido, C₄₋₆ heterocyclylamido, halogen, cyano, C₂₋₆ alkenyl, and C₂₋₆ alkynyl.

In some embodiments, in the compounds having the structures of Formulae I and II, R₁ and R₃ are each independently selected from the group consisting of C₁₋₆ alkylaminoacyloxy and C₄₋₆ heterocyclylacyloxy.

In some embodiments, in the compounds having the structures of Formulae I and II, R₁ and R₃ are each independently selected from the group consisting of -OC(=O)N(CH₃)₂, -OC(=O)-piperidine ring, -OC(=O)-morpholine ring, -OC(=O)-thiomorpholine, -OC(=O)-thiomorpholine dioxide, -OC(=O)-thiomorpholine monoxide, -OC(=O)NHCH(CH₃)₂, and -OC(=O)CH₃.

In some embodiments, in the compounds having the structures of Formulae I and II, R₁ and R₃ are each independently selected from the group consisting of -OC(=O)N(CH₃)₂, -OC(=O)-morpholine ring, -OC(=O)NHCH(CH₃)₂, and -OC(=O)CH₃.

In some embodiments, in the compounds having the structures of Formulae I and II, R₂ and R₄ are each independently selected from the group consisting of hydrogen, carboxyl, hydroxy, C₁₋₆ alkoxy, carboxyl-substituted C₁₋₆ alkylacyloxy, -OC(=O)-C₄₋₁₀ heterocyclyl, -O-P(O)₂OH, -O-S(O)₂OH, and -O-S(O)₂NH₂, the C₄₋₁₀ heterocyclyl being optionally substituted with one or more substituents selected from C₁₋₆ alkyl.

In some embodiments, in the compounds having the structures of Formulae I and II, R₂ and R₄ are each independently selected from the group consisting of hydrogen, hydroxy, C₁₋₆ alkoxy, carboxyl-substituted C₁₋₆ alkylacyloxy, -OC(=O)-C₄₋₁₀ heterocyclyl, -O-P(O)₂OH, -O-S(O)₂OH, and -O-S(O)₂NH₂, the C₄₋₁₀ heterocyclyl being optionally substituted with one or more substituents selected from C₁₋₆ alkyl.

In some embodiments, in the compounds having the structures of Formulae I and II, R₂ and R₄ are each independently selected from the group consisting of carboxyl, hydroxy, C₁₋₆ alkoxy, carboxyl-substituted C₁₋₆ alkylacyloxy, -OC(=O)-piperazinyl, -O-P(O)₂OH, -O-S(O)₂OH, and -O-S(O)₂NH₂, the piperazinyl being optionally substituted with one or more substituents selected from C₁₋₆ alkyl.

In some embodiments, in the compounds having the structures of Formulae I and II, R₂ and R₄ are each independently selected from the group consisting of carboxyl, hydroxy, 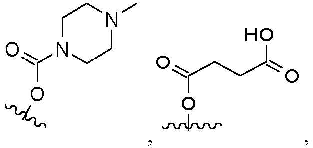 and -O-S(O)₂NH₂, where the wavy line " 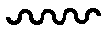 " represents the attachment point to the remainder of the molecule.

In some embodiments, in the compounds having the structures of Formulae I and II, R₂ and R₄ are each independently selected from the group consisting of hydroxy, 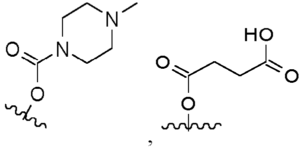 , and -O-S(O)₂NH₂.

In some embodiments, in the compounds having the structures of Formulae I and II, R₂ and R₄ are each independently selected from the group consisting of hydrogen, hydroxy, C₁₋₆ alkoxy, carboxyl-substituted C₁₋₆ alkylacyloxy, -O-P(O)₂OH, -O-S(O)₂OH, and -O-S(O)₂NH₂; preferably, R₂ and R₄ are each independently hydroxyl.

In some embodiments, in the compounds having the structures of Formulae I and II, R₂ and R₄ are each independently selected from the group consisting of hydrogen, hydroxy, C₁₋₆ alkoxy, carboxyl-substituted C₁₋₆ alkylacyloxy, -O-P(O)₂OH, and -O-S(O)₂OH; preferably, R₂ and R₄ are each independently hydroxyl.

In some embodiments, in the compounds having the structures of Formulae I and II, X is selected from the group consisting of -CO₂H and its carboxylic acid isosteres, -CO₂C₁₋₆ alkyl, -C(=O)SH, 4-10-membered heteroaryl, choline carboxylate, -C(=O)NHC₁₋₆ alkyl, -C(=O)NHS(=O)₂C₁₋₆ alkylamino and -C(=O)NHS(=O)₂C₁₋₆ alkyl, the -CO₂C₁₋₆ alkyl, 4-10-membered heteroaryl, choline carboxylate, -C(=O)NHC₁₋₆ alkyl, -C(=O)NHS(=O)₂C₁₋₆ alkylamino and -C(=O)NHS(=O)₂C₁₋₆ alkyl being optionally substituted with one or more substituents selected from the group consisting of 4-10 membered heterocyclyl, 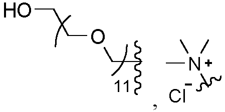 and -C₁₋₆ alkoxy, where the wavy line " 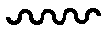 " represents the attachment point to the remainder of the molecule. In some embodiments, in the compounds having the structures of Formulae I and II, X is selected from the group consisting of -CO₂H and its carboxylic acid isosteres, -CO₂C₁₋₆ alkyl, 4-10-membered heteroaryl, choline carboxylate, -C(=O)NHC₁₋₆ alkyl, -C(=O)NHS(=O)₂C₁₋₆ alkylamino and -C(=O)NHS(=O)₂C₁₋₆ alkyl, the -CO₂C₁₋₆ alkyl, 4-10-membered heteroaryl, choline carboxylate, -C(=O)NHC₁₋₆ alkyl, -C(=O)NHS(=O)₂C₁₋₆ alkylamino and -C(=O)NHS(=O)₂C₁₋₆ alkyl being optionally substituted with one or more substituents selected from the group consisting of 4-10 membered heterocyclyl, 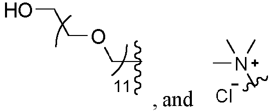 , where the wavy line " 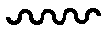 " represents the attachment point to the remainder of the molecule.

In some embodiments, in the compounds having the structures of Formulae I and II, X is selected from the group consisting of -CO₂H, -C(=O)SH, -CO₂C₁₋₆ alkyl, -C(=O)SH, tetrazolyl, -C(=O)NHC₁₋₆ alkyl, -C(=O)NHS(=O)₂C₁₋₆ alkyl and -C(=O)NHS(=O)₂N(C₁₋₆ alkyl)₂, the -CO₂C₁₋₆ alkyl and -C(=O)NHC₁₋₆ alkyl being each optionally substituted with a substituent selected from the group consisting of morpholinyl, 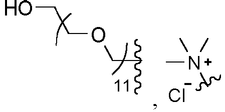 and -C₁₋₆ alkoxy, where the wavy line " 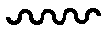 " represents the attachment point to the remainder of the molecule.

In some embodiments, in the compounds having the structures of Formulae I and II, X is selected from the group consisting of -CO₂H, -C(=O)NHCH₃, -C(=O)SH, 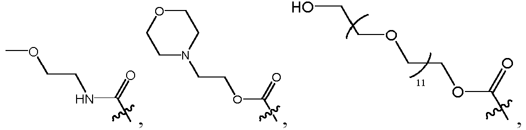 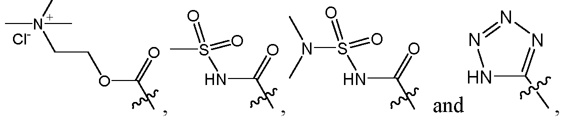 where the wavy line " 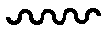 " represents the attachment point to the remainder of the molecule.

In some embodiments, in the compounds having the structures of Formulae I and II, X is selected from the group consisting of -CO₂H, -CO₂C₁₋₃ alkyl, 5-6-membered nitrogen-containing heterocyclyl, choline carboxylate, -C(=O)NHC₁₋₆ alkyl, -C(=O)NHS(=O)₂C₁₋₃ alkylamino and -C(=O)NHS(=O)₂C₁₋₃ alkyl, the -C(=O)₂C₁₋₃ alkyl, -C(=O)NHC₁₋₆ alkyl, -C(=O)NHS(=O)₂C₁₋₃ alkylamino and -C(=O)NHS(=O)₂C₁₋₃ alkyl are optionally substituted with one or more substituents selected from the group consisting of 4-6-membered heterocyclyl, 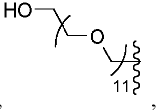 and

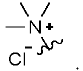

In some embodiments, in the compounds having the structures of Formulae I and II, X is selected from the group consisting of -CO₂H, a 5-6-membered nitrogen-containing heterocyclyl, -C(=O)NHS(=O)₂N(CH₃)₂, -C(=O)NHS(=O)₂CH₃, -CO₂CH₂CH₃, -CO₂CH₃, -CO₂CH₂CH₃, -C(=O)NHCH₂CH₂OCH₃, -C(=O)NHCH₃, and tetrazole, the -CO₂CH₂CH₃, -CO₂CH₃ or -CO₂CH₂CH₃ is optionally substituted with one or more substituents selected from the group consisting of morpholino,

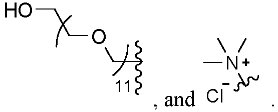

In some embodiments, in the compounds having the structures of Formulae I and II, n = 1, 2, 3, or 4.

In some embodiments, in the compounds having the structures of Formulae I and II, n = 1, 2, or 3.

In some embodiments, in the compounds having the structures of Formulae I and II, n = 1 or 2.

In some embodiments, in the compounds having the structures of Formulae I and II, R₁ and R₃ are each independently selected from the group consisting of hydroxyl, -OC(=O)N(CH₃)₂, 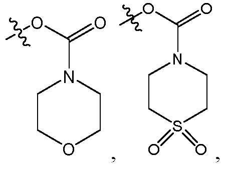 and 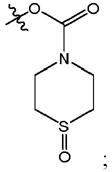
R₂ and R₄ are selected from hydroxyl;
X is -CO₂H, and R₁, R₂, R₃ and R₄ are not simultaneously hydroxyl.

In some embodiments, the compound of formula I is selected from the group consisting of:

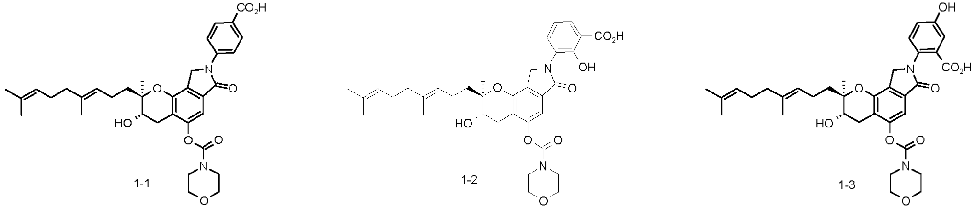

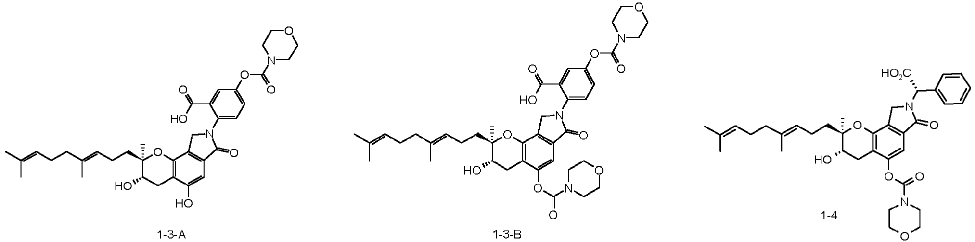

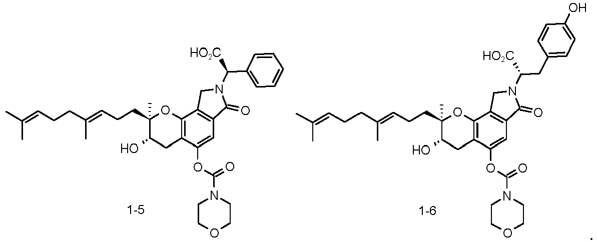

In some embodiments, the compound of formula II is selected from the group consisting of:

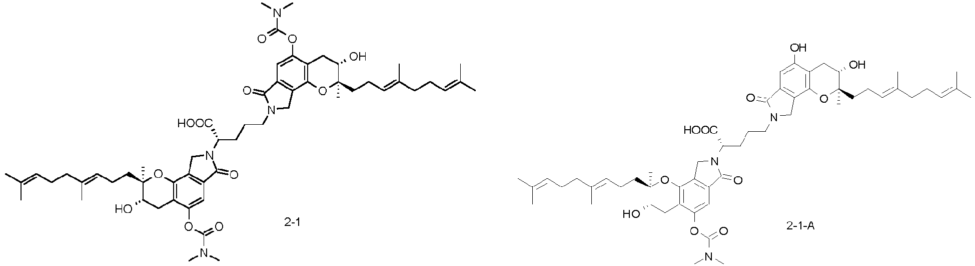

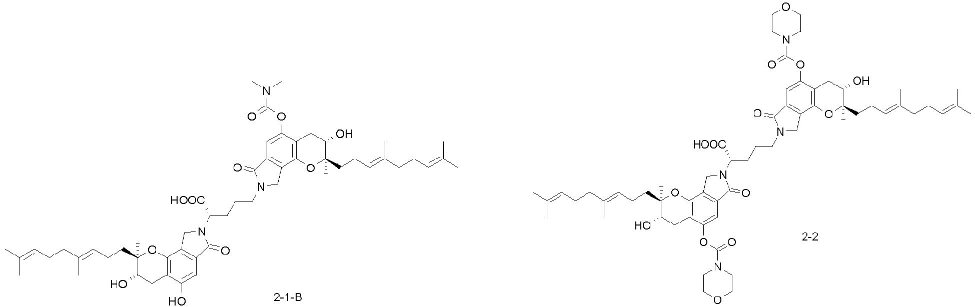

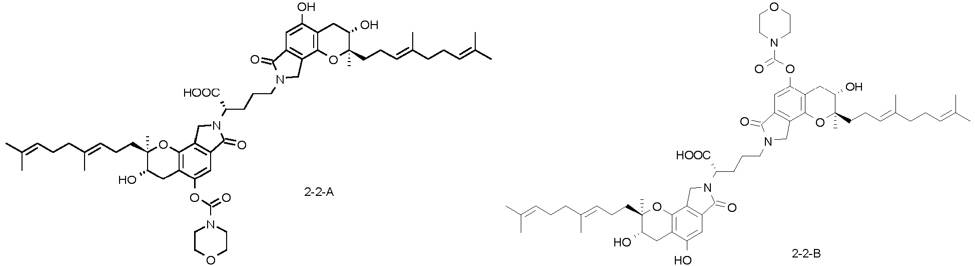

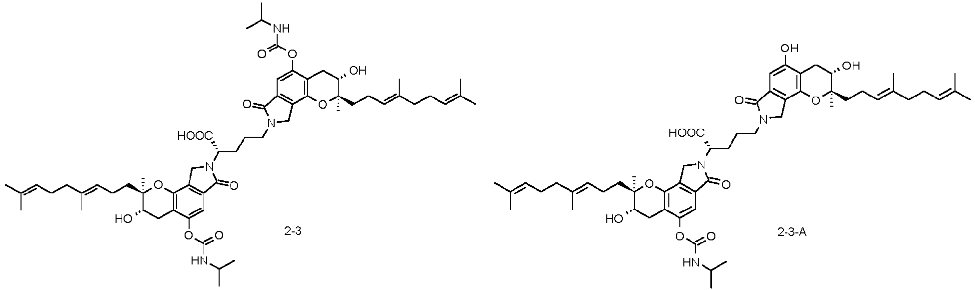

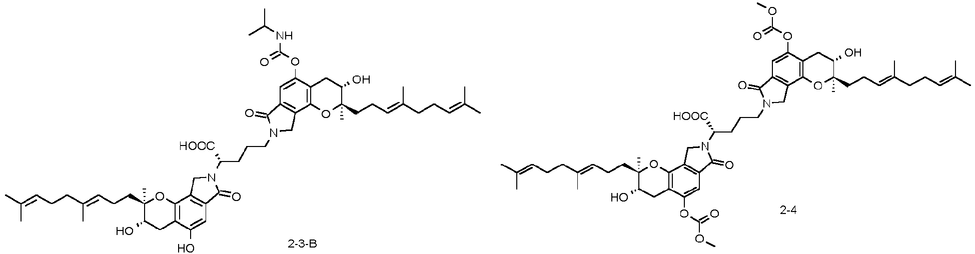

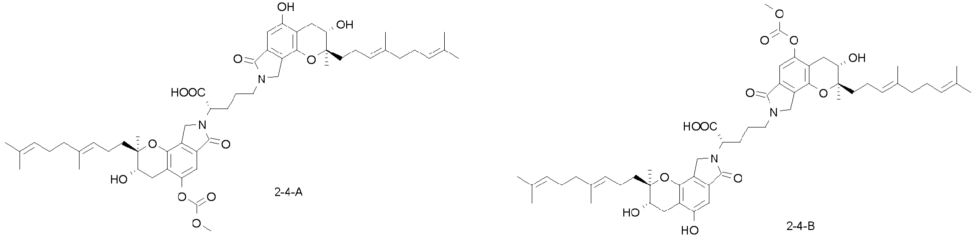

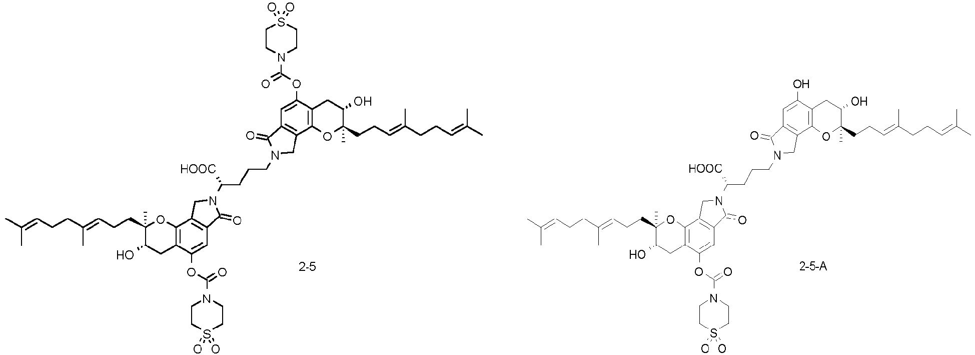

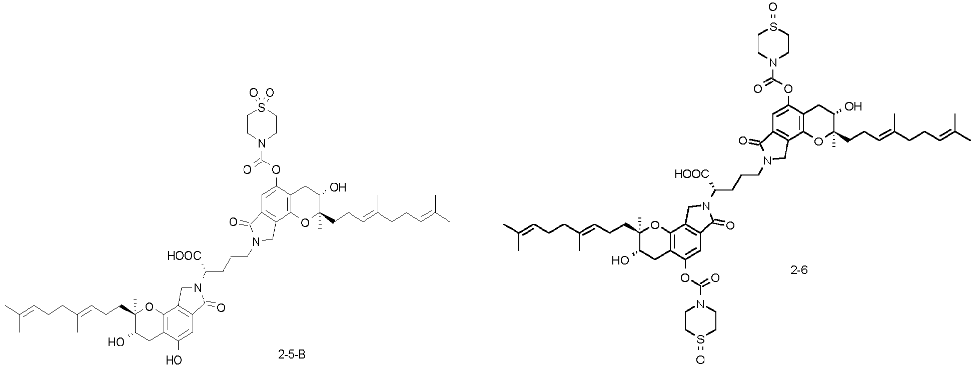

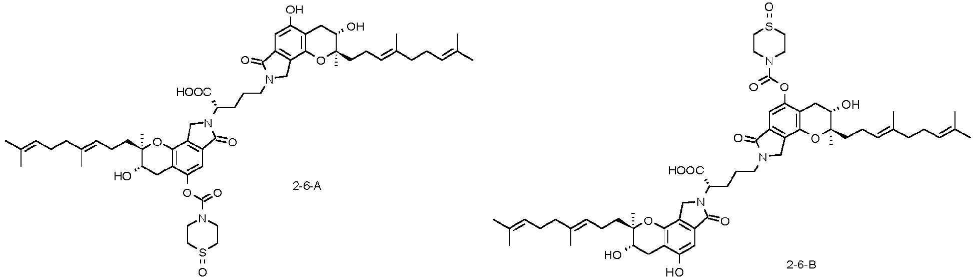

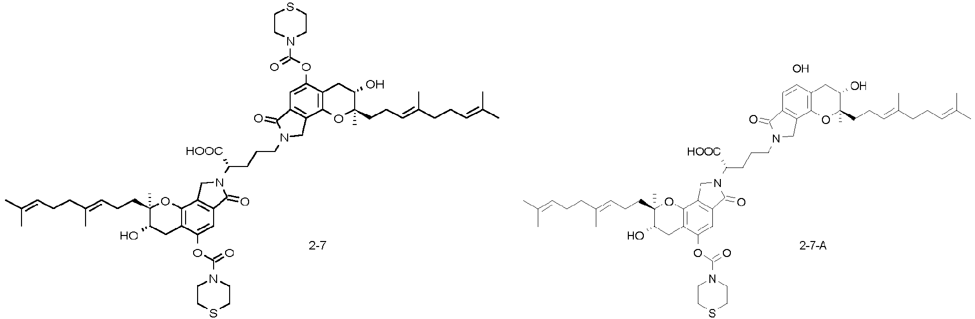

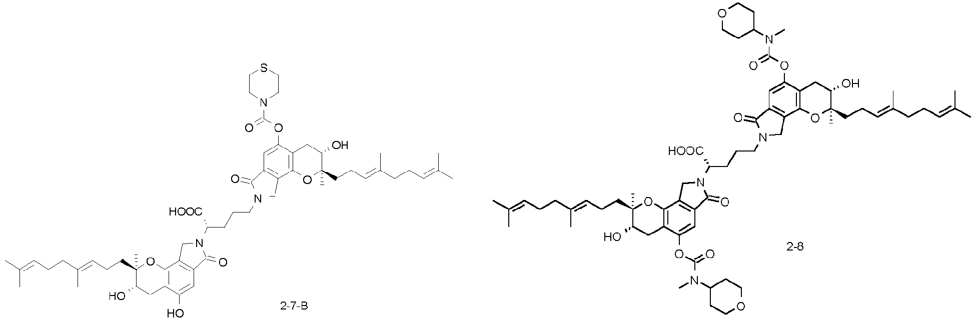

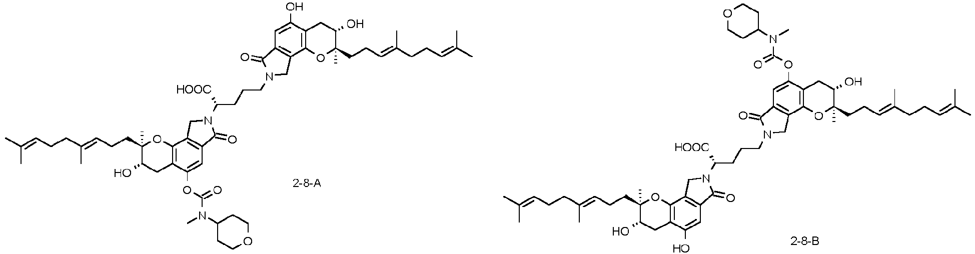

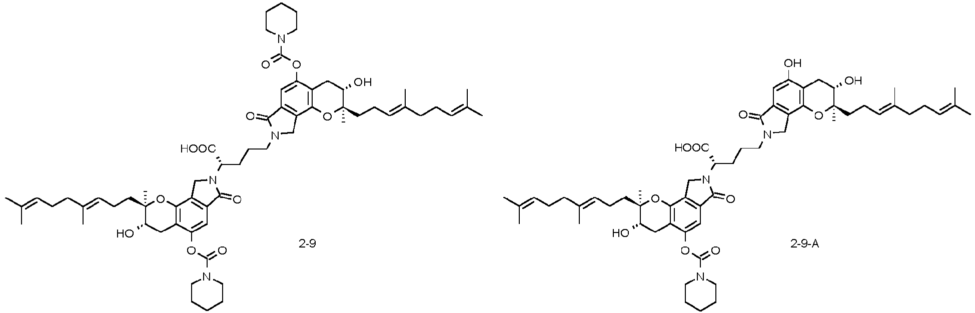

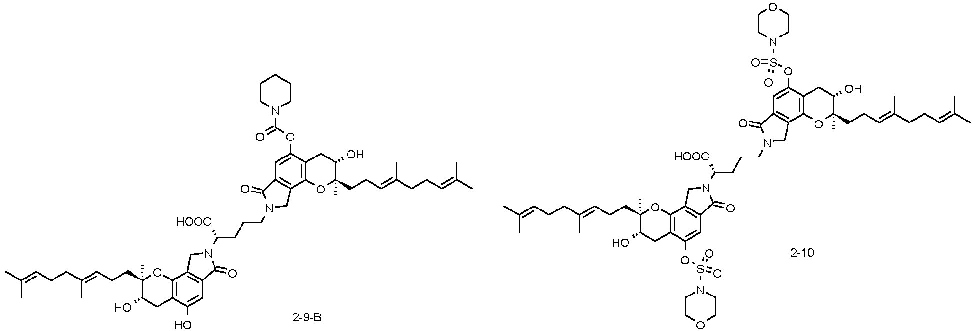

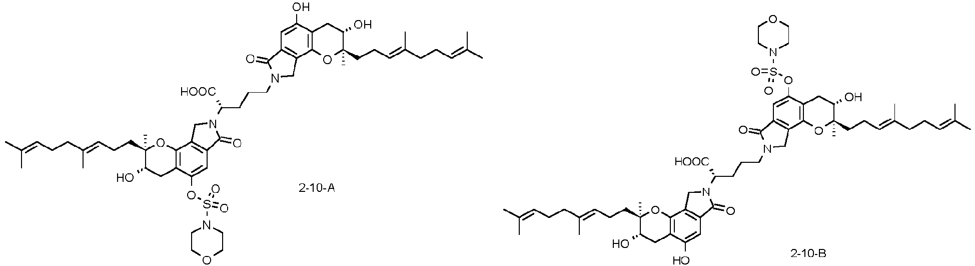

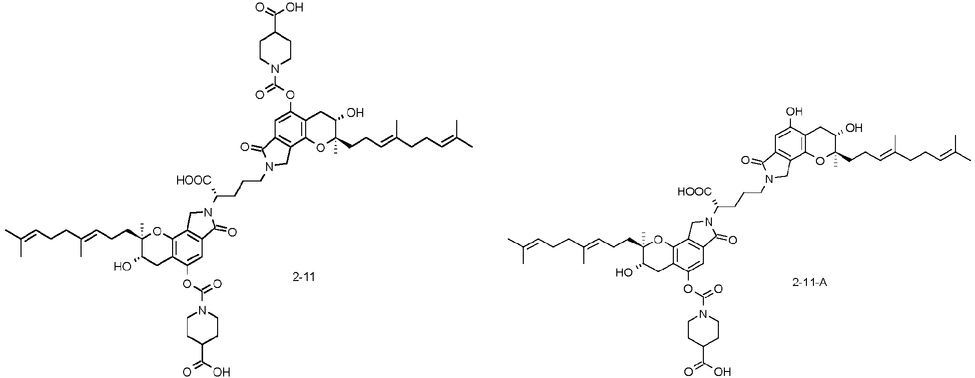

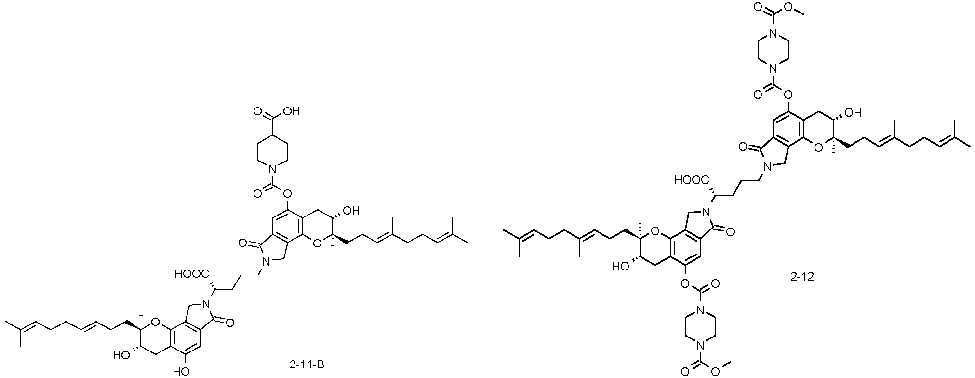

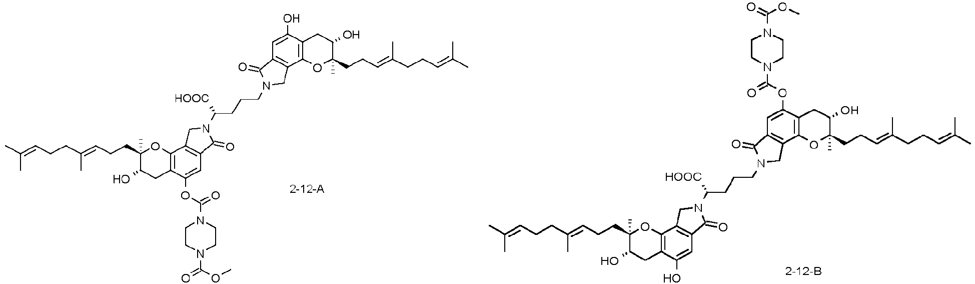

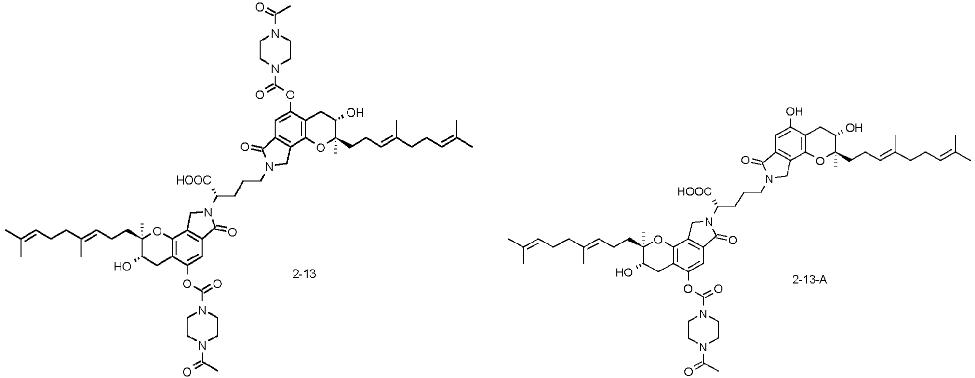

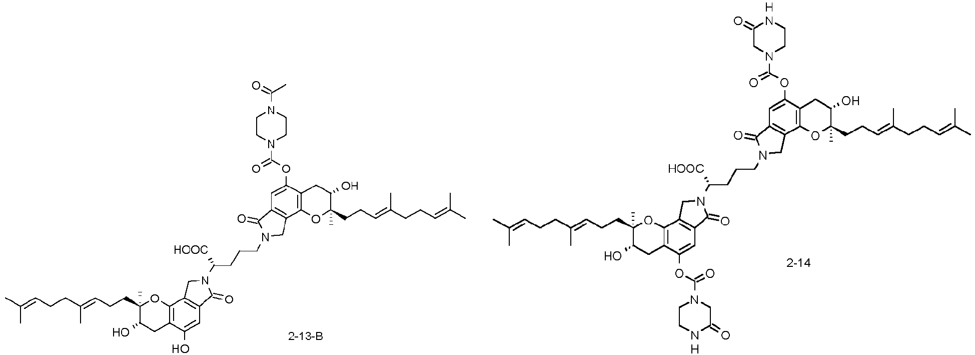

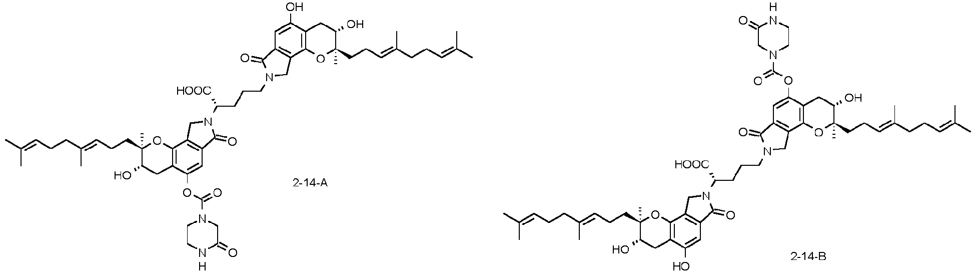

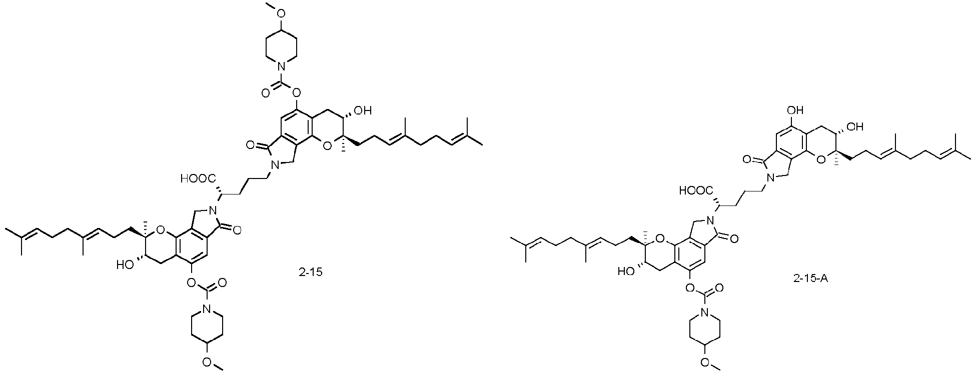

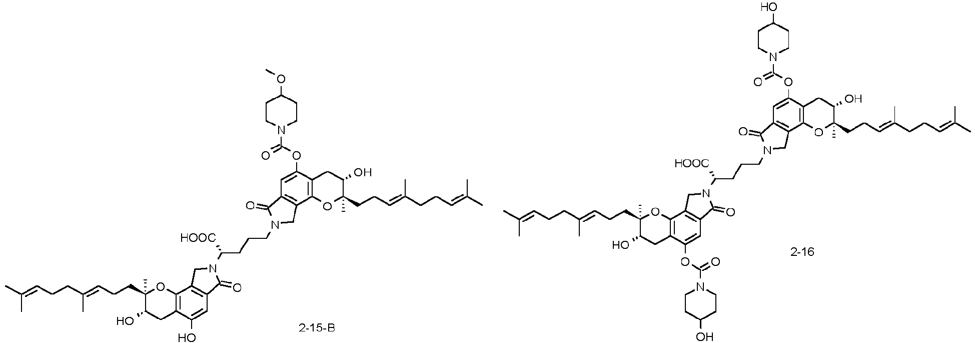

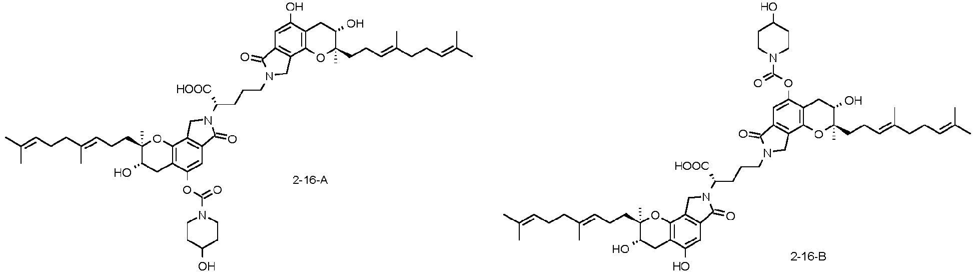

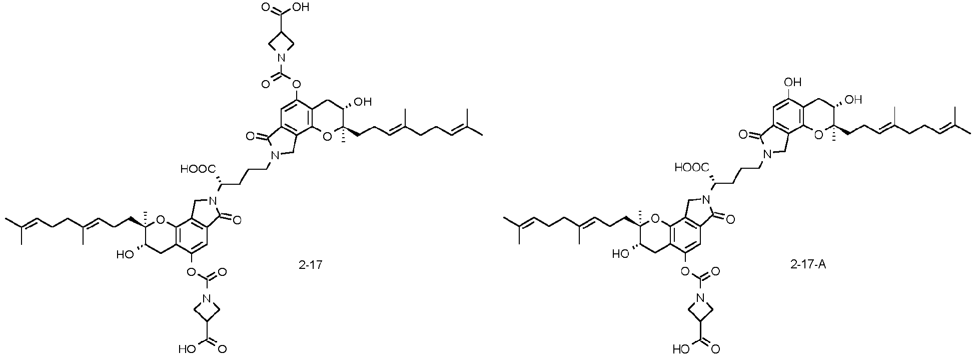

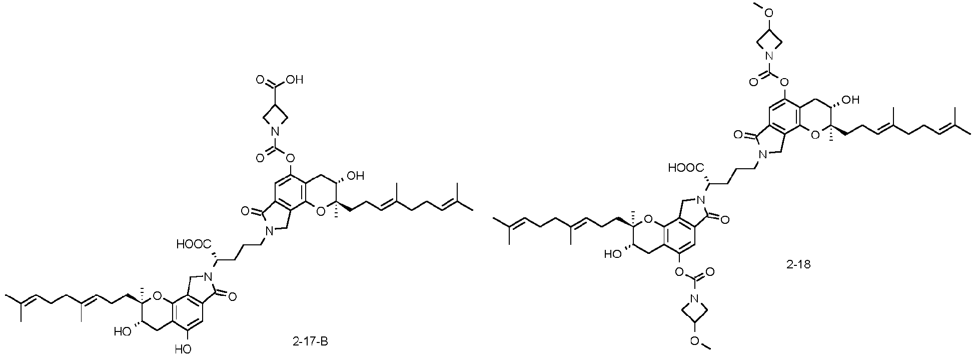

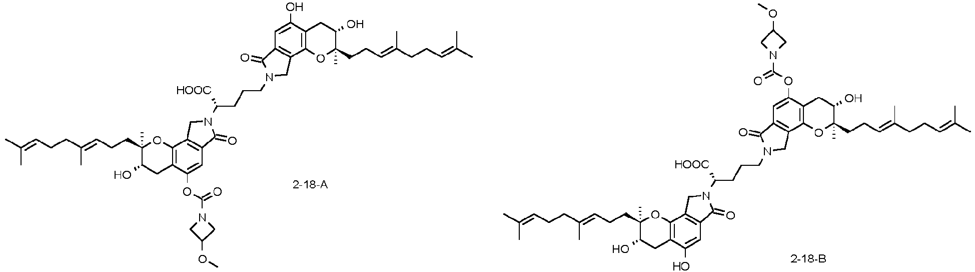

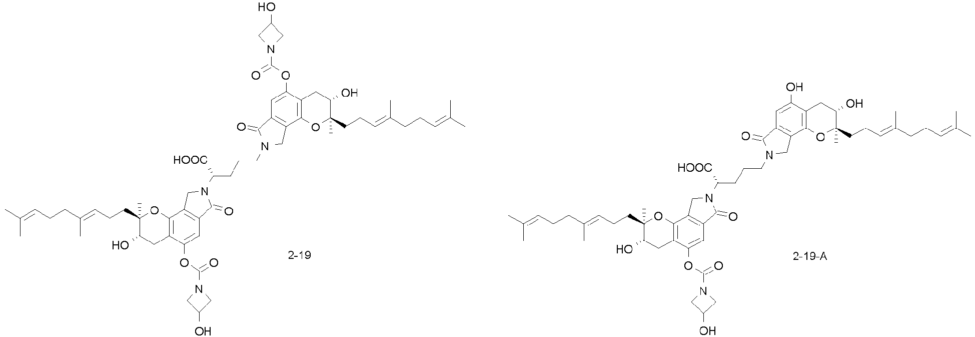

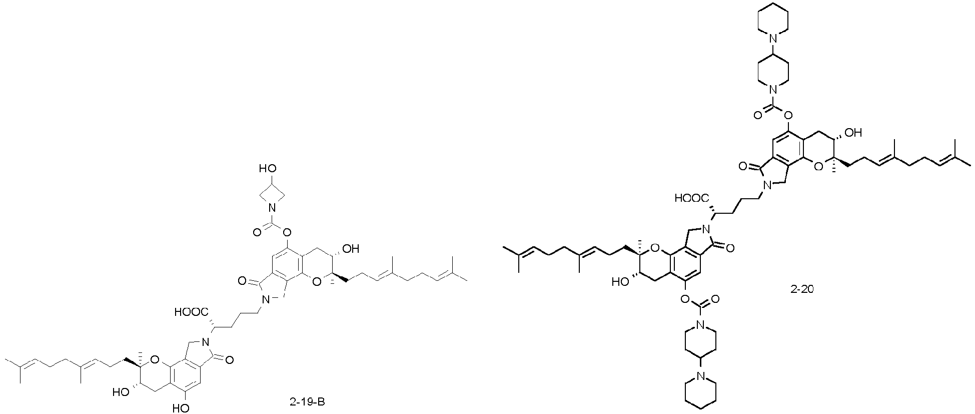

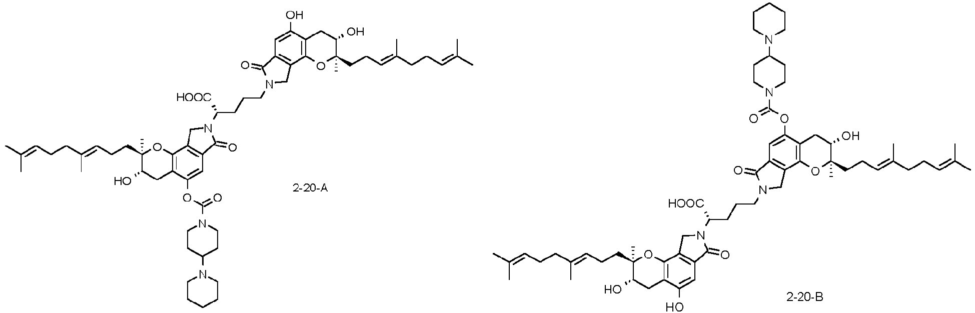

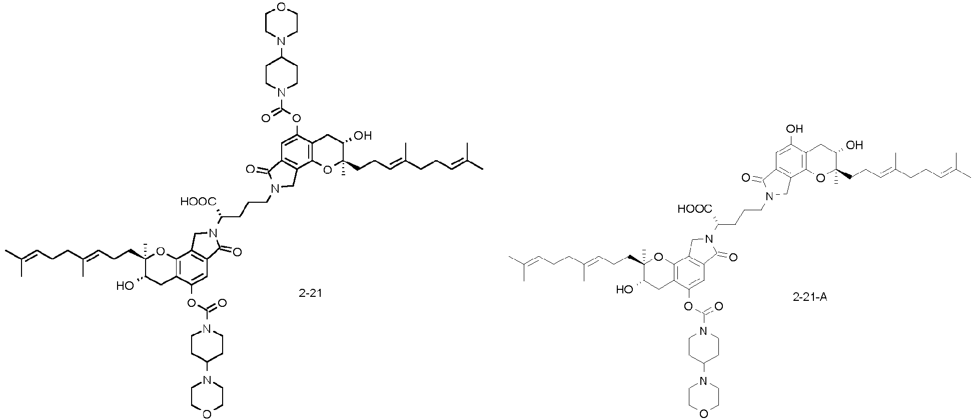

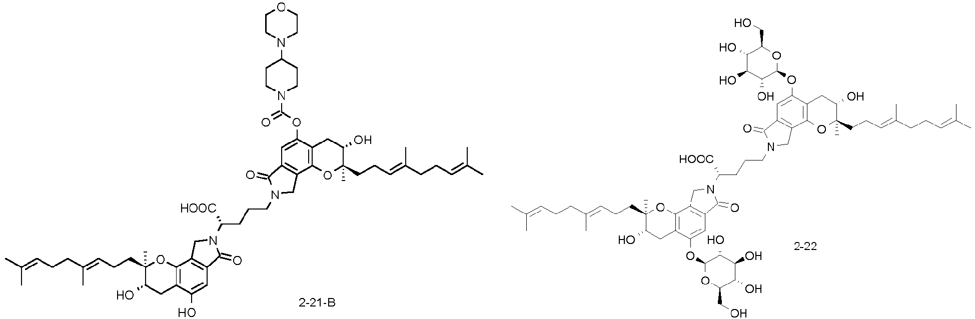

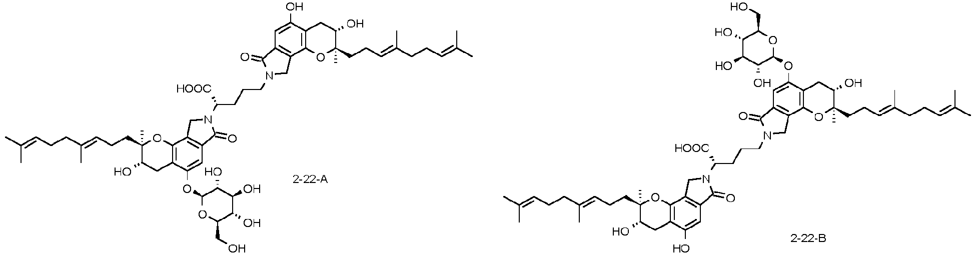

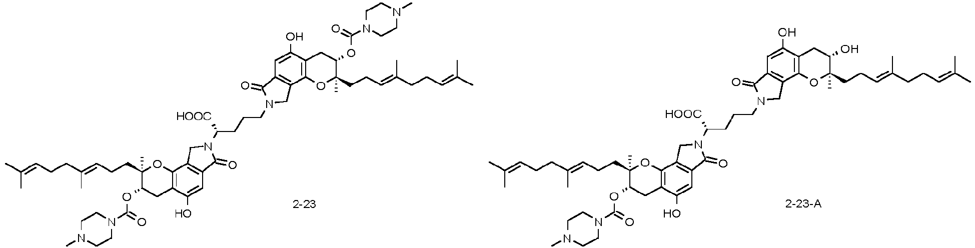

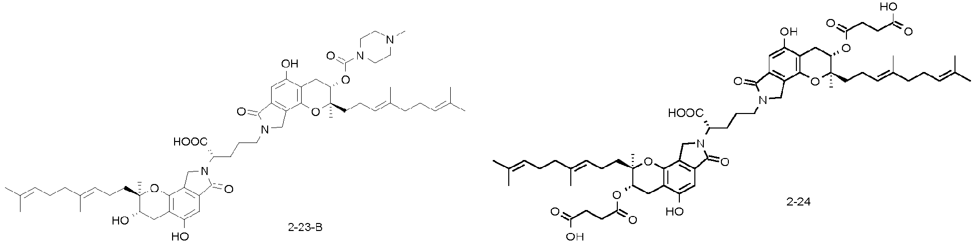

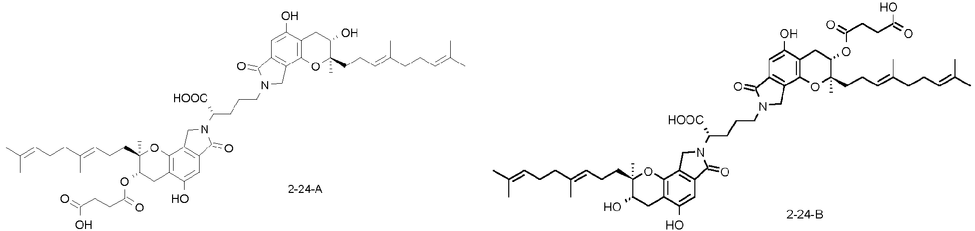

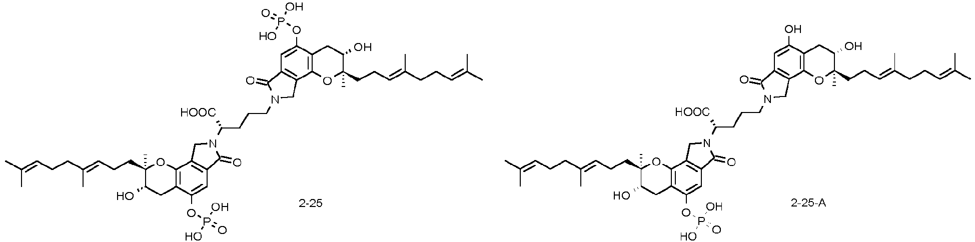

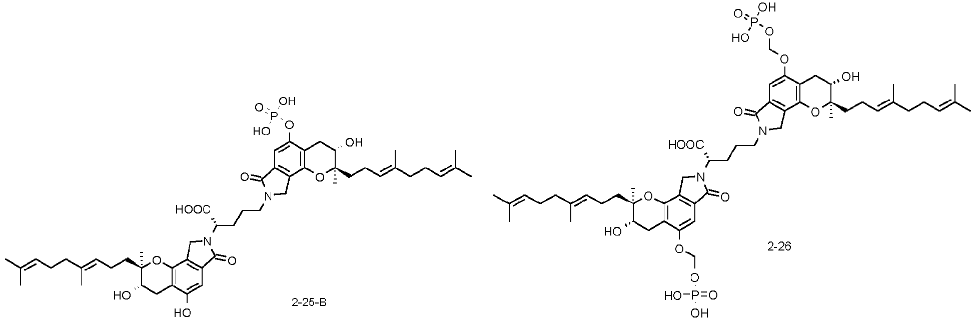

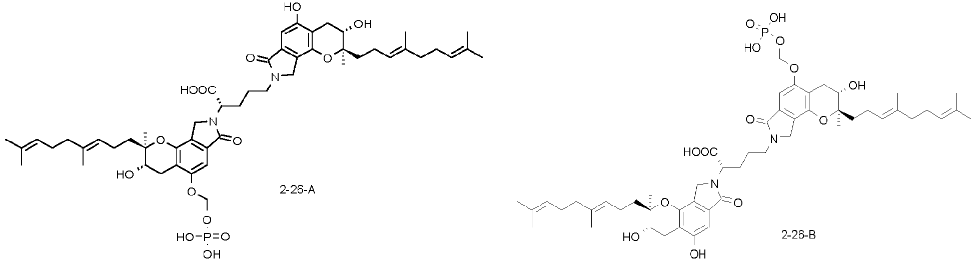

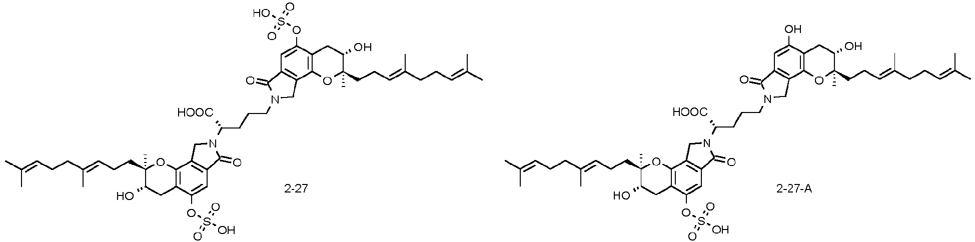

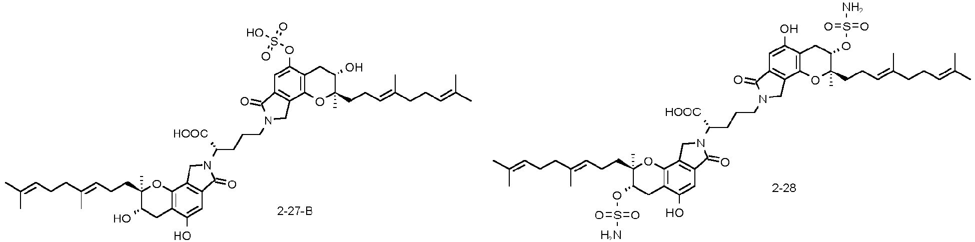

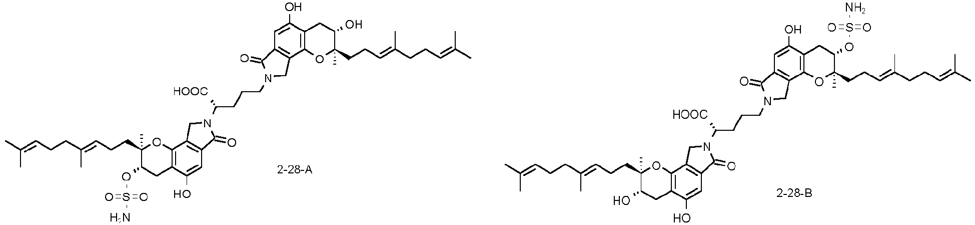

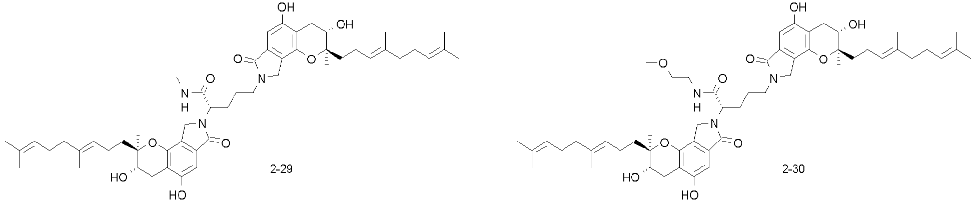

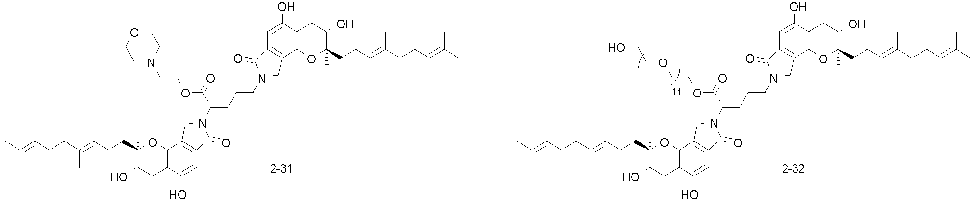

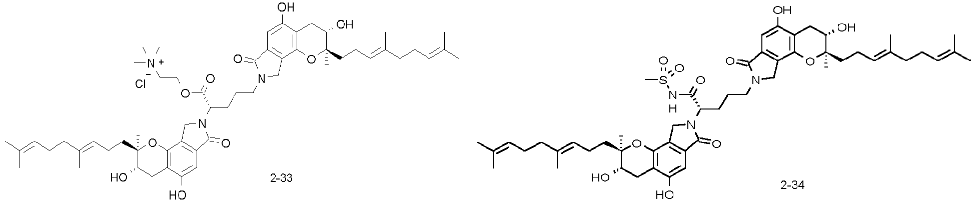

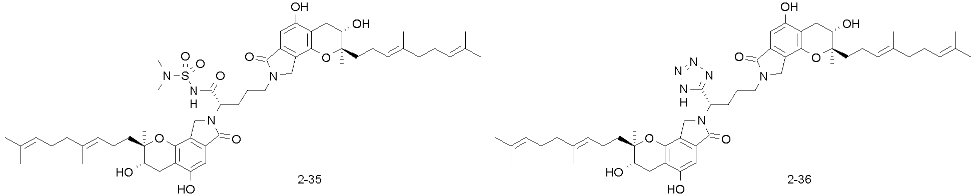

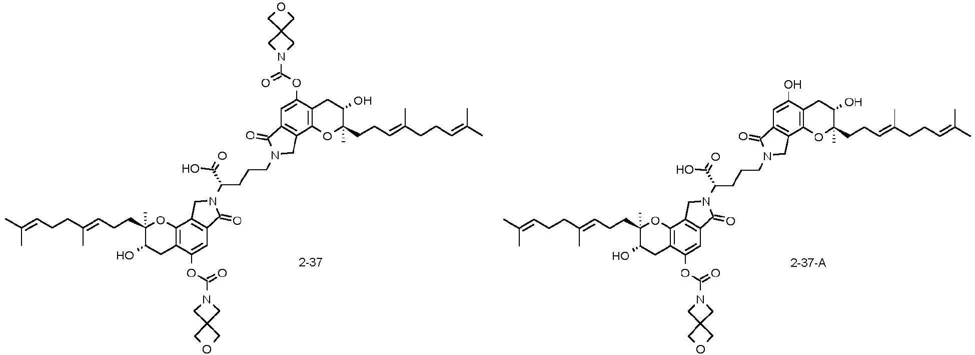

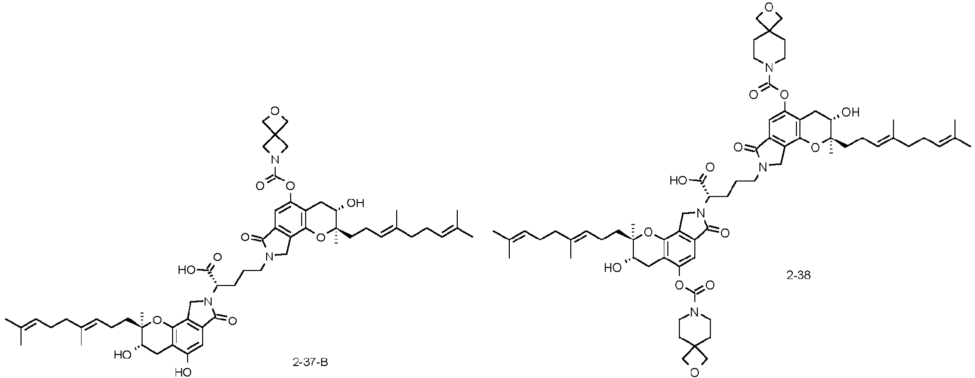

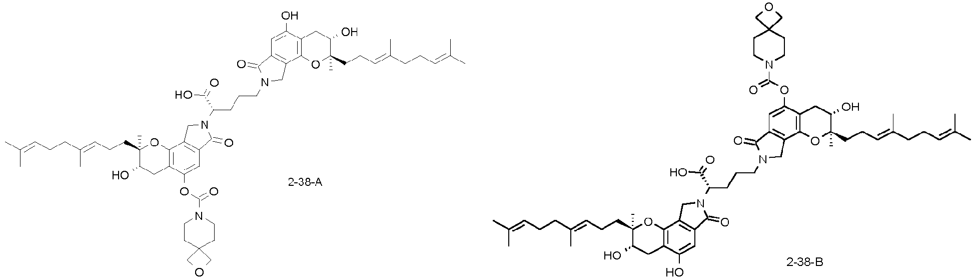

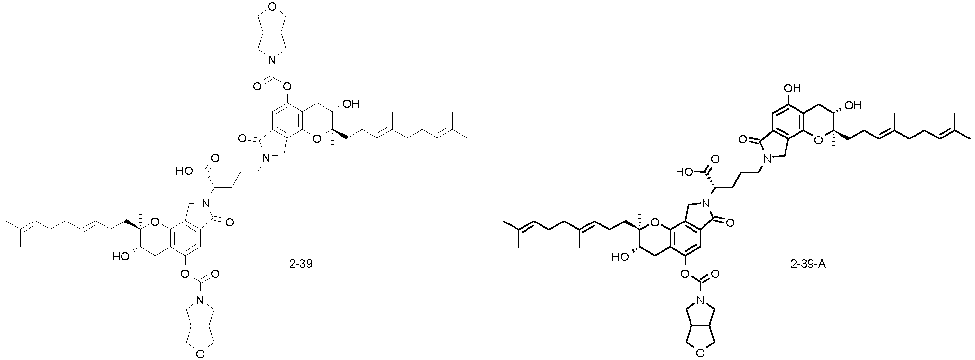

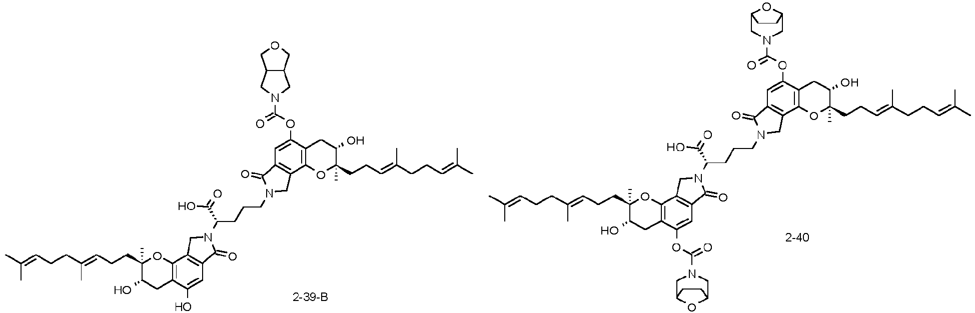

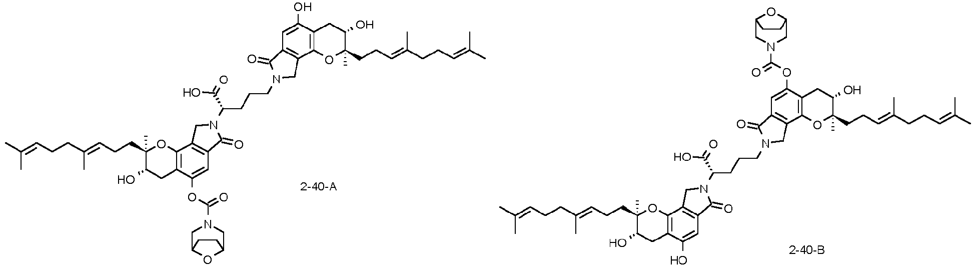

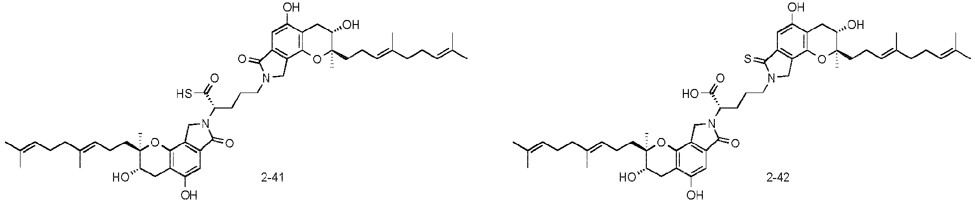

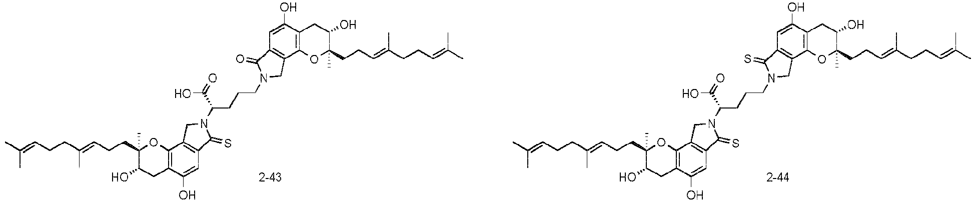

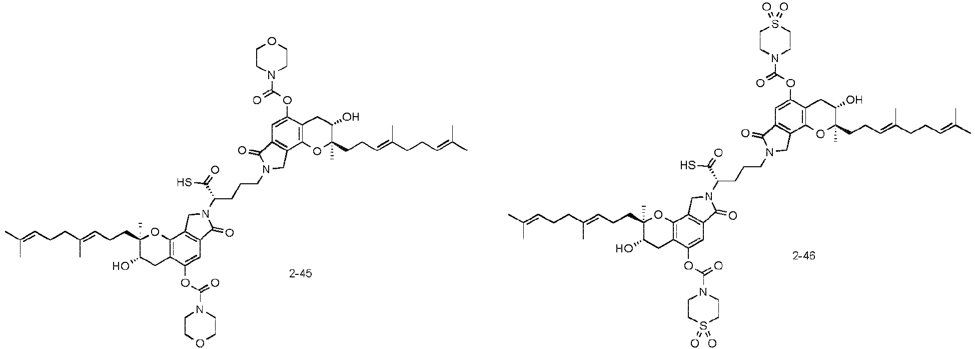

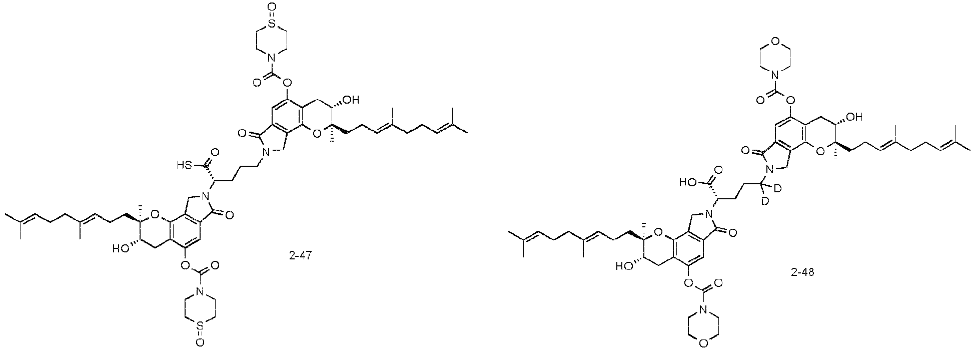

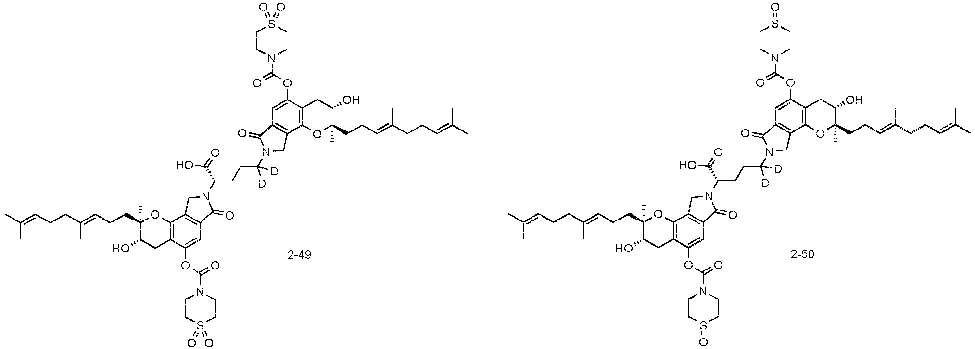

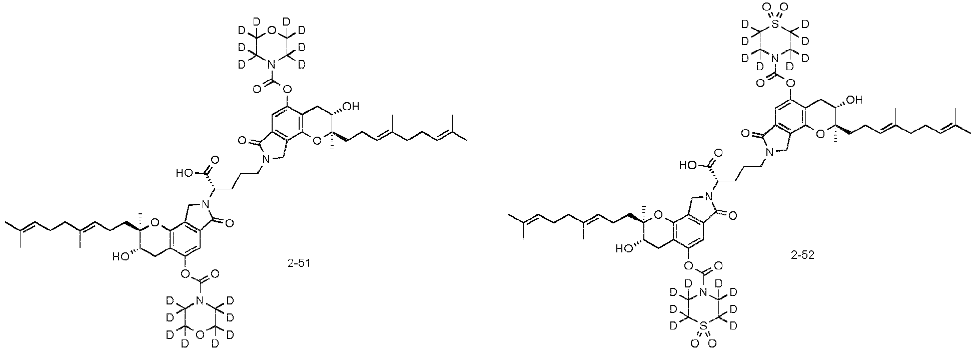

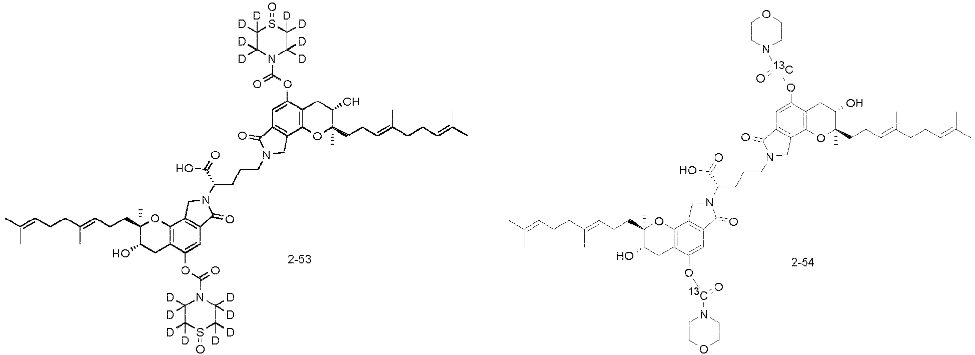

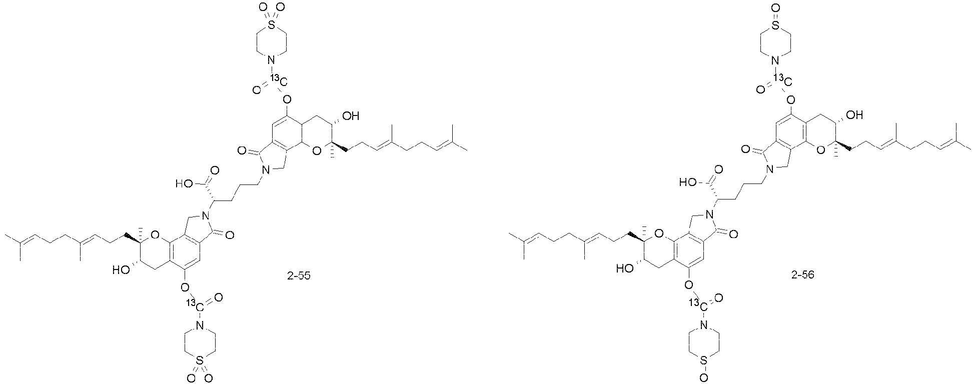

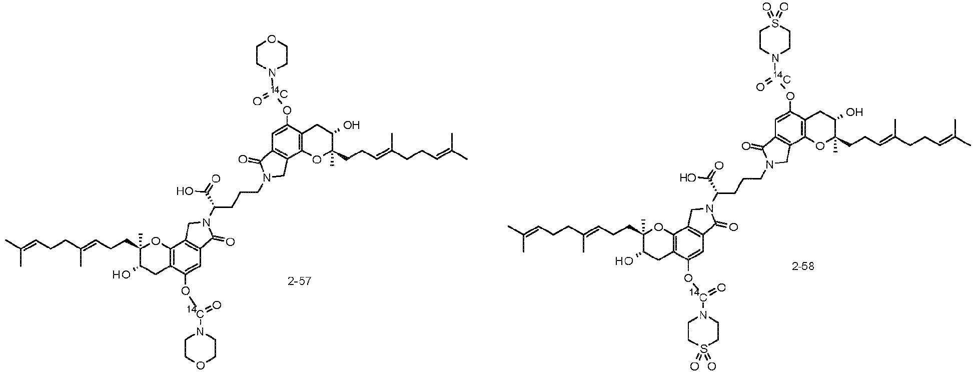

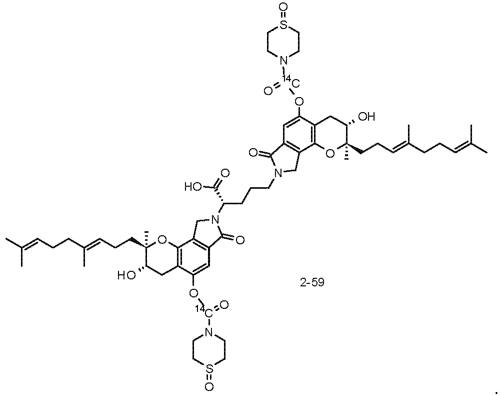

Another object of the present invention is to provide a process for preparing the compound of formula I of the present invention comprising the steps shown in the following scheme: 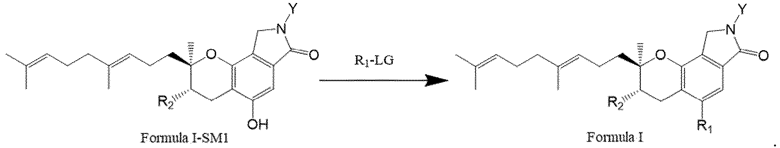 wherein:
R₁, R₂ and Y are as defined above for the compound of formula I; and
LG is a leaving group.

**In** some embodiments, the leaving group includes, but is not limited to, halogen, C₁₋₆ alkylacyloxy, nitrophenoxy, and fluorophenoxy.

**In** some embodiments, the leaving group includes, but is not limited to, fluorine, chlorine, bromine, iodine, acetoxy, p-nitrophenoxy, and p-fluorophenoxy.

When R₁ is selected from C₁₋₆ alkylaminoacyloxy or C₄₋₆ heterocyclylacyloxy, the compound of formula I of the present invention can be synthesized and prepared by the following synthetic route.

Route 1: the compound of Formula I-SM-1 is condensed with an alkylaminoacyl chloride or a heterocyclylacyl chloride to obtain the compound of formula I.

In some embodiments, the reaction is carried out at a temperature of 0-140 °C, e.g., 0 °C, 20 °C, 25 °C, 40 °C, 50 °C, 60 °C, 100 °C, and 140 °C, preferably 0 to 35 °C.

In some embodiments, the reaction is carried out in a suitable organic solvent selected from the group consisting of halogenated hydrocarbons (e.g., dichloromethane (DCM), chloroform (TCM), 1,2-dichloroethane (1,2-DCE), etc.), nitriles (e.g., acetonitrile (AN), etc.), N-methylpyrrolidone (NMP), N,N-dimethylformamide (DMF), N,N-dimethylacetamide (DMA), tetrahydrofuran (THF), 1,4-dioxane (Dioxane), dimethyl sulfoxide (DMSO), and any combinations thereof, preferably tetrahydrofuran (THF).

In some embodiments, the reaction is carried out in the presence of a suitable base including an organic base or an inorganic base. The organic base is selected from the group consisting of N,N-diisopropylethylamine (DIPEA), triethylamine (TEA), potassium tert-butoxide (t-BuOK) and pyridine (Py). The inorganic base is selected from the group consisting of potassium phosphate (K₃PO₄), sodium hydride (NaH), potassium carbonate (K₂CO₃), sodium carbonate (Na₂CO₃), sodium bicarbonate (NaHCO₃), cesium carbonate (Cs₂CO₃), and NaOH, preferably NaH.

Route II: the compound of Formula I-SM-1 is reacted with an isocyanate to give the compound of Formula I.

In some embodiments, the reaction is carried out at a temperature of 0-140 °C, e.g., 0 °C, 20 °C, 25 °C, 40 °C, 50 °C, 60 °C, 100 °C, and 140 °C, preferably 25 °C.

In some embodiments, the reaction is carried out in a suitable organic solvent selected from the group consisting of halogenated hydrocarbons (e.g., dichloromethane (DCM), chloroform (TCM), 1,2-dichloroethane (1,2-DCE), etc.), nitriles (e.g., acetonitrile (AN), etc.), N-methylpyrrolidone (NMP), N,N-dimethylformamide (DMF), N,N-dimethylacetamide (DMA), tetrahydrofuran (THF), 1,4-dioxane (Dioxane), dimethyl sulfoxide (DMSO) and any combinations thereof, preferably N,N-dimethylformamide (DMF).

In some embodiments, the reaction is carried out in the presence of a suitable base including an organic base or an inorganic base. The organic base is selected from the group consisting of N,N-diisopropylethylamine (DIPEA), triethylamine (TEA), potassium tert-butoxide (t-BuOK) and pyridine (Py). The organic base is selected from the group consisting of potassium phosphate (K₃PO₄), sodium hydride (NaH), potassium carbonate (K₂CO₃), sodium carbonate (Na₂CO₃), sodium bicarbonate (NaHCO₃), cesium carbonate (Cs₂CO₃) and NaOH. The base is preferably N,N-diisopropylethylamine (DIPEA) or triethylamine (TEA).

Another object of the present invention is to provide a process for preparing the compound of formula II of the present invention comprising the steps shown in the following scheme: 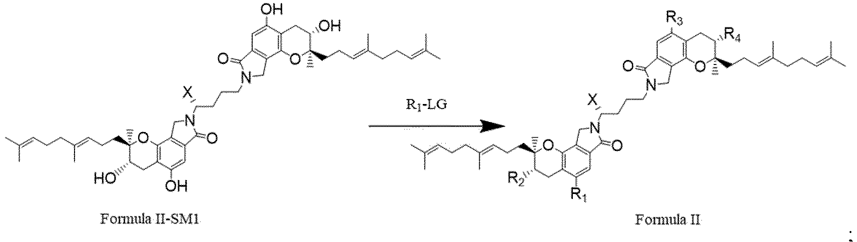 wherein:
R₁, R₃ and X are as defined above for the compound of formula II, and R₁ and R₃ are identical;
R₂ and R₄ are hydroxyl; and
LG is a leaving group.

In some embodiments, the leaving group includes, but is not limited to, halogen, C₁₋₆ alkylacyloxy, nitrophenoxy, and fluorophenoxy.

In some embodiments, the leaving group includes, but is not limited to, fluorine, chlorine, bromine, iodine, acetoxy, p-nitrophenoxy, and p-fluorophenoxy.

When R₁ and R₃ are each independently selected from C₁₋₆ alkylaminoacyloxy or C₄₋₆ heterocyclylacyloxy, the compound of formula II of the present invention can be synthesized and prepared by the following synthetic routes.

Route 1: the compound of Formula II-SM1 is condensed with an alkylaminoacyl chloride or a heterocyclylacyl chloride to obtain the compound of formula II.

In some embodiments, the reaction is carried out at a temperature of 0-140 °C, e.g., 0 °C, 20 °C, 25 °C, 40 °C, 50 °C, 60 °C, 100 °C, and 140 °C, preferably 0 to 35 °C.

In some embodiments, the reaction is carried out in a suitable organic solvent selected from the group consisting of halogenated hydrocarbons (e.g., dichloromethane (DCM), chloroform (TCM), 1,2-dichloroethane (1,2-DCE), etc.), nitriles (e.g., acetonitrile (AN), etc.), N-methylpyrrolidone (NMP), N,N-dimethylformamide (DMF), N,N-dimethylacetamide (DMA), tetrahydrofuran (THF), 1,4-dioxane (Dioxane), dimethyl sulfoxide (DMSO), and any combinations thereof, preferably tetrahydrofuran (THF).

In some embodiments, the reaction is carried out in the presence of a suitable base including an organic base or an inorganic base. The organic base is selected from the group consisting of N,N-diisopropylethylamine (DIPEA), triethylamine (TEA), potassium tert-butoxide (t-BuOK) and pyridine (Py). The inorganic base is selected from the group consisting of potassium phosphate (K₃PO₄), sodium hydride (NaH), potassium carbonate (K₂CO₃), sodium carbonate (Na₂CO₃), sodium bicarbonate (NaHCO₃), cesium carbonate (Cs₂CO₃), and NaOH, preferably NaH.

Route II: the compound of Formula II-SM1 is reacted with an isocyanate to give the compound of formula II.

In some embodiments, the reaction is carried out at a temperature of 0-140 °C, which is 0 °C, 20 °C, 25 °C, 40 °C, 50 °C, 60 °C, 100 °C, and 140 °C, preferably 25 °C.

In some embodiments, the reaction is carried out in a suitable organic solvent selected from the group consisting of halogenated hydrocarbons (e.g., dichloromethane (DCM), chloroform (TCM), 1,2-dichloroethane (1,2-DCE), etc.), nitriles (e.g., acetonitrile (AN), etc.), N-methylpyrrolidone (NMP), N,N-dimethylformamide (DMF), N,N-dimethylacetamide (DMA), tetrahydrofuran (THF), 1,4-dioxane (Dioxane), dimethyl sulfoxide (DMSO) and any combinations thereof, preferably N,N-dimethylformamide (DMF).

In some embodiments, the step is carried out in the presence of a suitable base including an organic base or an inorganic base. The organic base is selected from the group consisting of N,N-diisopropylethylamine (DIPEA), triethylamine (TEA), potassium tert-butoxide (t-BuOK) and pyridine (Py). The inorganic base is selected from the group consisting of potassium phosphate (K₃PO₄), sodium hydride (NaH), potassium carbonate (K₂CO₃), sodium carbonate (Na₂CO₃), sodium bicarbonate (NaHCO₃), cesium carbonate (Cs₂CO₃) and NaOH. The base is preferably N,N-diisopropylethylamine (DIPEA) or triethylamine (TEA).

### Definition

Unless otherwise defined in the context, all technical and scientific terms used herein are intended to have the same meaning as commonly understood by a person skilled in the art. References to techniques employed herein are intended to refer to the techniques as commonly understood in the art, including variations on those techniques or substitutions of equivalent techniques which would be apparent to a person skilled in the art. While it is believed that the following terms will be readily understood by a person skilled in the art, the following definitions are nevertheless put forth to better illustrate the present invention.

The terms "comprising", "including, "having", "involving", or any other variant thereof are intended to cover non-exclusive or open-ended content of inclusion. For example, a composition, method, or device comprising a series of elements is not necessarily limited to the elements that are explicitly listed, but may also contain other elements that are not explicitly listed or elements inherent to the composition, method, or device described above.

The term "pharmaceutically acceptable salt" refers to a salt of the compound of the invention that is substantially nontoxic to an organism. Pharmaceutically acceptable salts generally include (but are not limited to) salts formed by the reaction of the compounds of the invention with pharmaceutically acceptable inorganic/organic acids or inorganic/organic bases, also referred to as acid addition salts or base addition salts.

For a review on suitable salts, see "Hand book of Pharmaceutical Salts: Properties, Selection, and Use" by Stahl and Wermuth (Wiley-VCH, 2002). The method for preparing a pharmaceutically acceptable salt of the compound of the present invention is known to a person skilled in the art.

The term "pharmaceutically acceptable ester" refers to an ester of the compound of the invention or a salt thereof that is substantially nontoxic to an organism and hydrolyzes in the organism to the compound of the invention or a salt thereof. Pharmaceutically acceptable esters generally include (but are not limited to) esters formed from the compounds of the invention with pharmaceutically acceptable carboxylic or sulfonic acids, also referred to as carboxylate esters or sulfonate esters.

The term "isomer" refers to compounds having the same molecular weight due to the same number and type of atoms, but with different spatial arrangements or configurations of atoms.

The term "stereoisomer"(or "optically active isomer") refers to a stable isomer having a vertical asymmetric plane due to having at least one chiral factor (including a chiral center, a chiral axis, a chiral plane, etc.), thereby enabling rotation of plane-polarized light. The present invention also includes these stereoisomers and mixtures thereof due to the presence of asymmetric centers and other chemical structures in the compounds of the invention that may lead to stereoisomers. Since the compounds of the invention (or pharmaceutically acceptable salts thereof) comprise asymmetric carbon atoms, they can be present in the form of single stereoisomers, racemates, or mixtures of enantiomers and diastereomers. Typically, these compounds can be prepared as racemates. However, if required, such compounds can be prepared or isolated to yield pure stereoisomers, i.e., single enantiomers or diastereoisomers, or mixtures enriched with single stereoisomers (≥ 98%, ≥ 95%, ≥ 93%, ≥ 90%, ≥ 88%, ≥ 85%, or ≥ 80% purity). As described below, a single stereoisomer of a compound is obtained by synthesis from an optically active starting material containing the desired chiral center, or by preparing a mixture of enantiomeric products followed by separation or resolution, e.g. conversion to a mixture of diastereomers followed by separation or recrystallization, chromatographic treatment, use of chiral resolution reagents, or direct separation of the enantiomers on a chiral column. Starting compounds with specific stereochemistry can be commercially available or prepared as described below followed by resolution via methods well known in the art. The term "enantiomer" refers to a pair of stereoisomers that have mirror images that cannot overlap with each other. The term "diastereomer" refers to optically active isomers that do not constitute mirror images of each other. The term "racemic mixture" or "racemate" refers to a mixture containing an equal amount of single enantiomers (i.e., an equimolar mixture of the two R and S enantiomers). The term "non-racemic mixture" refers to a mixture containing unequal amounts of single enantiomers. Unless otherwise indicated, all stereoisomeric forms of the compounds of the invention are within the scope of the invention.

The term "tautomer"(or "tautomeric form") refers to structural isomers with different energies that can be interconverted through low energy barriers. If tautomerism is possible (e.g. in solution), the chemical equilibrium of the tautomers can be achieved. For example, proton tautomers (or proton transfer tautomers) include, but are not limited to, interconversion by proton migration, such as keto-enol isomerization, imine-enamine isomerization, amide-iminol isomerization, etc. Unless otherwise indicated, all tautomeric forms of the compounds of the invention are within the scope of the invention.

The term "polymorph" (or "polymorphic form") refers to the solid crystalline form of a compound or complex. Polymorphs of molecules can be obtained by those skilled in the art via many known methods. These methods include, but are not limited to, melt recrystallization, melt cooling, solvent recrystallization, desolvation, rapid evaporation, rapid cooling, slow cooling, gas phase diffusion, and sublimation. In addition, polymorphs can be detected, classified and identified using well-known techniques including, but not limited to, differential scanning calorimetry (DSC), thermogravimetric analysis (TGA), X-ray powder diffraction (XRPD), single crystal X-ray diffraction (SCXRD), solid-state nuclear magnetic resonance (NMR), infrared spectroscopy (IR), Raman spectroscopy, and scanning electron microscopy (SEM), etc.

The term "solvate" refers to a substance formed by combining the compound of the invention (or a pharmaceutically acceptable salt thereof) with at least one solvent molecule by noncovalent intermolecular forces. Common solvates include, but are not limited to, hydrates (including hemihydrates, monohydrates, dihydrates, trihydrates, etc.), ethanolates, acetonates, etc.

The term "isotopically labeled product" refers to a derivatized compound formed by replacing a specific atom in a compound of the invention with an isotopic atom thereof. Unless otherwise indicated, the compounds of the invention include various isotopes of H, C, N, O, F, P, S, and Cl, such as ²H (D), ³H (T), ¹³C, ¹⁴C, ¹⁵N, ¹⁷O, ¹⁸O, ¹⁸F, ³¹P, ³²P, ³⁵S, ³⁶S, and ³⁷Cl. For example, ¹²C may be replaced with ¹²C, ¹³C, or ¹⁴C; ¹H may be replaced with ²H (D, deuterium) or ³H (T, tritium); ¹⁶O may be replaced with ¹⁸O, etc. The invention comprises isotopically labeled compounds obtained by replacement of any atom in the structure with its isotope.

The term "metabolite" refers to a derivatized compound formed after metabolism of the compound of the invention. Further information on metabolism can be found in Goodman and Gilman' s: The Pharmacological Basis of Therapeutics (9th ed.) [M], McGraw-Hill International Editions, 1996.

The term "prodrug" refers to a derivatized compound capable of providing the compound of the invention directly or indirectly after administration to a subject. Particularly preferred derivatized compounds or prodrugs are compounds that can improve the bioavailibility of the compounds of the invention (e.g., more readily absorbed into blood) or compounds that facilitate delivery of the parent compound to the site of action (e.g., the lymphatic system) when administered to a subject. Unless otherwise indicated, all prodrug forms of the compounds of the invention are within the scope of the invention, and various prodrug forms are well known in the art.

The term "each independently" means that at least two groups (or ring systems) present in a structure with the same or similar ranges of values may have the same or different meanings in a particular case. For example, substituent X and substituent Y are each independently hydrogen, halogen, hydroxyl, cyano, alkyl or aryl, which indicates that when substituent X is hydrogen, substituent Y may be either hydrogen or halogen, hydroxy, cyano, alkyl, or aryl; similarly, when substituent Y is hydrogen, substituent X may be either hydrogen or halogen, hydroxyl, cyano, alkyl, or aryl.

The term "alkyl" refers to a linear or branched saturated aliphatic hydrocarbon group. For example, the term "C₁₋₆ alkyl" as used in the present invention refers to an alkyl having 1 to 6 carbon atoms (e.g., methyl, ethyl, n-propyl, isopropyl, n-butyl, sec-butyl, isobutyl, tert-butyl, n-pentyl, n-hexyl, etc.) optionally substituted with one or more (e.g., 1 to 3) substituents described in the invention (e.g., when substituted with halogen, the group is "C₁₋₆ haloalkyl", e.g., -CF₃, -C₂F₅, -CHF₂, -CH₂F, -CH₂CF₃, -CH₂Cl, -CH₂CH₂CF₃, etc.).

The term "C₁₋₆ alkylamino" refers to a group formed by substitution of -NH₂ with 1 or 2 C₁₋₆ alkyl groups, i.e., -NH-C₁₋₆ alkyl or -N (C₁₋₆ alkyl)₂.

The term "acyloxy" refers to -O-C(=O)-.

The term "C₁₋₆ alkylacyloxy" refers to -O-C(=O)-C₁₋₆ alkyl.

The term "C₁₋₆ alkylaminoacyloxy" refers to -O-C(=O)-NH-C₁₋₆ alkyl or -O-C(=O)-N(C₁₋₆ alkyl)₂.

The term "C₁₋₆ alkoxyacyloxy" refers to -O-C(=O)-C₁₋₆ alkoxy.

The term "C₁₋₆ alkoxyalkylaminoacyloxy" refers to -O-C(=O)-NH-alkyl-O-alkyl.

The term "C₄₋₁₀ heterocyclylacyloxy" refers to -O-C(=O)-C₄₋₁₀ heterocyclyl.

The term "sulfonyloxy" refers to -O-S(=O)₂-.

The term "C₁₋₆ hydroxyalkyl" refers to -C₁₋₆ alkylene-OH.

The term "alkoxy" refers to an "alkyl" or a "cycloalkyl" as defined above that is linked to the parent molecule moiety via an oxygen atom (e.g., C₁₋₆ alkoxy, C₃₋₈ cycloalkoxy, etc., including methoxy, ethoxy, n-propoxy, isopropoxy, cyclopropoxy, n-butoxy, isobutoxy, tert-butoxy, sec-butoxy, cyclobutoxy, n-pentoxy, isopentoxy, n-hexoxy, or isomers thereof). For example, the term "C₁₋₆ alkoxy" as used in the present invention refers to an alkoxy group having 1 to 6 carbon atoms (e.g., methoxy, ethoxy, tert-butoxy, etc.) optionally substituted with one or more (e.g., 1 to 3) substituents described in the present invention (e.g., when substituted with halogen, the group is "C₁₋₆ haloalkoxy", e.g. -OCF₃, -OC₂F₅, etc.).

The term "C₁₋₆ alkoxycarbonyl" refers to -C(=O)-OC₁₋₆ alkyl.

The term "aryl" refers to a monocyclic or fused polycyclic aromatic hydrocarbon group having a conjugated π-electron system optionally substituted with one or more substituents described in the present invention (e.g., substituted with oxo, C₁₋₆ alkyl (methyl, ethyl) or halogen (fluorine, chlorine, bromine)). For example, the term "C₆₋₁₀ aryl" as used in the present invention refers to an aryl group having 6 to 10 carbon atoms (e.g., phenyl, naphthyl, etc.).

The term "heteroaryl" refers to a monocyclic or fused polycyclic aromatic group having a conjugated π-electron system having one or more carbon atoms (e.g., 1, 2, 3, 4, 5, 6, 9 or 10 carbon atoms) and one or more (e.g., 2, 3 or 4) heteroatoms or heteroatomic groups each independently selected from O, S, N, and NH in the ring, optionally substituted with one or more substituents described in the present invention (e.g., substituted with oxo, C₁₋₆ alkyl (methyl, ethyl) or halogen (fluorine, chlorine, bromine)). If the valence bond requirements are met, the heteroaryl group can be linked to the parent molecule moiety by any ring atom. For example, the term "4-10-membered heteroaryl" as used in the present invention refers to a heteroaryl having 4 to 10 ring atoms (e.g., thienyl), furyl, pyrrolyl, oxazolyl, thiazolyl, imidazolyl, pyrazolyl, isoxazolyl, isothiazolyl, oxadiazolyl, triazolyl, tetrazolyl, thiadiazolyl, pyridyl (pyridinyl), pyridazinyl, pyrimidinyl, pyrazinyl, triazinyl, or benzo derivatives thereof).

The term "alkenyl" refers to a linear or branched aliphatic hydrocarbon group containing one or more carbon-carbon double bonds, typically containing from about 2 to 20 carbon atoms. For example, the term "C₂₋₆ alkenyl" as used in the present invention refers to an alkenyl group containing from 2 to 6 carbon atoms (e.g., vinyl, propenyl, n-butenyl, 3-methylbut-2-enyl, n-pentenyl, n-hexyl, etc.) optionally substituted with one or more (e.g., 1 to 3) substituents (e.g., halogen, alkyl, alkenyl, alkynyl, aryl, cycloalkyl, cyano, hydroxy, alkoxy, aryloxy, alkoxyalkyl, alkylthio, amino, alkylacyloxy, arylacyloxy, cycloalkylacyloxy, carboxyl, alkoxycarbonyl, etc.).

The term "C₂₋₆ alkynyl" refers to a linear or branched monovalent hydrocarbon group comprising one or more triple bonds and having 2, 3, 4, 5 or 6 carbon atoms, in particular 2 or 3 carbon atoms ("C₂₋₃ alkynyl"). The C₂₋₆ alkynyl group is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or 3,3-dimethylbut-1-ynyl. In particular, the alkynyl group is ethynyl group, a prop-1-ynyl group or prop-2-ynyl group.

The term "heterocyclyl" means a 3- to 18-membered nonaromatic cyclic group consisting of 2 to 12 carbon atoms and 1 to 6 heteroatoms selected from nitrogen, oxygen, and sulfur, such as a C₄₋₁₀ heterocyclyl, a C₄₋₈ heterocyclyl, a 4-12-membered heterocyclyl, a 4-8-membered heterocyclyl, and a 5-6-membered heterocyclyl. The heterocyclyl may be saturated or unsaturated. Unless otherwise specified in this specification, the heterocyclyl may be a ring system of monocyclic, bicyclic, tricyclic or more rings, which may include a fused ring system, an condensed ring system, a bridged ring system, or a spiro ring system. For the purposes of the present application, the heterocyclyl is preferably a 4- to 12-membered nonaromatic monocyclic or bicyclic group comprising 1 to 3 heteroatoms selected from nitrogen, oxygen, and sulfur, such as a 4- to 7-membered nonaromatic monocyclic group comprising 1 to 3 heteroatoms selected from nitrogen, oxygen, and sulfur, or a 5- to 6-membered nonaromatic monocyclic group comprising 1 to 2 heteroatoms selected from nitrogen and oxygen; or a 5-12-membered nonaromatic bicyclic group, including a fused bicyclic ring, a condensed bicyclic ring, a bridged bicyclic ring, or a spiro ring. The nitrogen, carbon or sulfur atoms in the heterocyclyl may optionally be oxidized, e.g., substituted with oxo; the nitrogen atom may optionally be quaternized; and the heterocyclyl may be partially unsaturated or completely saturated. The heterocyclyl may be attached to the remainder of the molecule via a carbon atom or a heteroatom and via a single bond. Examples of heterocyclyls include, but are not limited to, azetidinyl, pyranyl, tetrahydropyranyl, thiapyranyl, tetrafuryl, morpholinyl, thiomorpholinyl, pyrazolyl, dihydropyrazolyl, piperazinyl, piperazonyl, piperidinyl, pyrrolyl, pyrrolidinyl, oxazinyl, dioxolyl, tetrahydroisoquinolinyl, decahydroisoquinolinyl, imidazolinyl, imidazolidinyl, quinazinyl, thiazolidinyl, isothiazolidinyl, isooxazolidinyl, dihydroindolyl, octahydroindolyl, octahydroisoindolyl, pyrazolidinyl, phthalimido, azabicyclo[3.1.0]hexyl, azaspiro[2.4]heptyl, and groups of the following formulae: 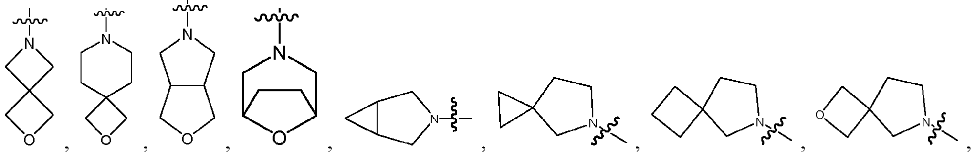 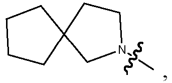 and preferably morpholinyl, thiomorpholinyl, thiomorpholinyl dioxide, thiomorpholinyl monoxide, piperidinyl, pyrrolidinyl, azetidinyl, dihydropyrazolyl, tetrahydropyranyl, piperazinyl, piperazinonyl, azabicyclo[3.1.0]hexyl, azaspiro[2.4]heptyl, and

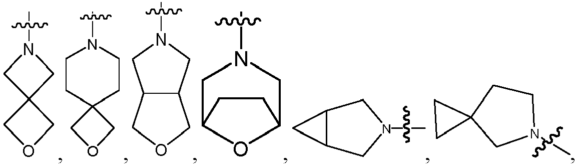

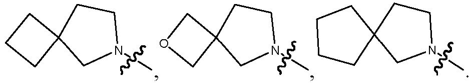

The term "halogen" refers to fluorine (F), chlorine (Cl), bromine (Br) and iodine (I).

The term "cyano" refers to a "-CN" group.

The term "hydroxy" refers to a "-OH" group.

The term "carboxyl" refers to a "-CO₂H" group.

The term "substitution" or "substituted" means that one or more (e.g., 1, 2, 3, or 4) atoms (e.g., hydrogen atoms) or atomic groups (e.g., trifluoromethanesulfonate groups) **in** the designated group are replaced with other atoms or atomic groups, provided that the assigned group satisfies the valence bond requirements **in** the present case and forms a stable compound after substitution. Combinations of substituents and/or variables are allowed only if the combination is capable of forming a stable compound. If the substituent is described as "optionally substituted with ...", the substituent may be unsubstituted or substituted. If the first substituent is described as optionally substituted with one or more groups in the second list of substituents, one or more hydrogen atoms in the first substituent may be replaced, individually or each independently, with one or more groups in the second list of substituents, or not.

When the bond of a substituent is shown to pass through a bond linking two atoms in a ring, such a substituent may be linked to any of the ring-forming atoms in the ring.

The term "one or more" refers to 1 or more, e.g., 2, 3, 4, 5, 6, 7, 8, 9, or 10, under reasonable conditions.

The carbon-carbon bonds of the compound of the present invention may be depicted herein using a solid line ( 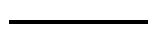 ), a solid wedge ( 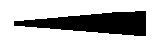 ), or a dotted wedge ( 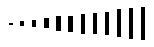 ). The use of a solid line to depict bonds to asymmetric carbon atoms is meant to indicate that all possible stereoisomers (e.g., specific enantiomers, racemic mixtures, etc.) at that carbon atom are included. The use of either a solid or dotted wedge to depict bonds to asymmetric carbon atoms is meant to indicate that the stereoisomer shown is present. When present in racemic compounds, solid and dotted wedges are used to define relative stereochemistry, rather than absolute stereochemistry. Unless stated otherwise, it is intended that the compound of the present invention can exist as stereoisomers, which include *cis* and *trans* isomers, optical isomers such as R and S enantiomers, diastereomers, geometric isomers, rotational isomers, conformational isomers, atropisomers, and mixtures thereof. The compound of the present invention may exhibit more than one type of isomerism, and consist of mixtures thereof (such as racemates and diastereomeric pairs).

The term "carboxylic acid electronic isostere" refers to a group or substituent having physical and chemical properties similar to the carboxyl group that produces generally similar, related, or opposite biological activity. These include, but are not limited to, hydroxamic acid, hydroxamate, phosphonic acid, hypophosphinic acid, sulfonic acid, sulfinic acid, sulfonamide, sulfonylurea, ureide, tetrazole, thiazolidinone, and oxazolidinone.

The term "glycosyl" refers to one or more compounds of a class containing a tetrahydropyran or tetrahydrofuran structure, and this class of compounds is substituted with one or more -O-, hydroxy, hydroxymethyl, carboxyl, amino, acetamido, such as glucopyranosyl, glucofuranosyl, and the like.

### Pharmaceutical composition, use and therapeutic method

It is another object of the present invention to provide a pharmaceutical composition comprising a therapeutically effective amount of the compound of the invention, or a pharmaceutically acceptable salt, an ester, a solvate, a stereoisomer, a tautomer, a polymorph, an isotopically labeled product, a metabolite, a prodrug, or a mixture thereof, and one or more pharmaceutically acceptable carriers thereof, and optionally further comprising one or more additional drugs for the treatment of thromboembolic diseases.

It is a further object of the present invention to provide a method of treating thromboembolic diseases comprising administering to a subject in need thereof a therapeutically effective amount of the compound of the invention, or a pharmaceutically acceptable salt, a stereoisomer, a polymorph, a solvate, a metabolite, or a prodrug, or a pharmaceutical composition of the invention.

It is a further object of the present invention to provide use of the compound of the invention, or a pharmaceutically acceptable salt, an ester, a solvate, a stereoisomer, a tautomer, a polymorph, an isotopically labeled product, a metabolite, a prodrug, or a mixture thereof, or the pharmaceutical composition of the present invention in the manufacture of a medicament for the treatment of thromboembolic diseases.

It is a further object of the present invention to provide the compound of the invention, or a pharmaceutically acceptable salt, an ester, a solvate, a stereoisomer, a tautomer, a polymorph, an isotopically labeled product, a metabolite, a prodrug, or a mixture thereof, or the pharmaceutical composition of the present invention, for use in the treatment of thromboembolic diseasess.

The term "pharmaceutically acceptable carrier" in the present invention refers to a diluent, auxiliary material, excipient, or vehicle with which a therapeutic is administered, and it is, within the scope of sound medical judgment, suitable for contact with the tissues of human beings and animals without excessive toxicity, irritation, allergic response, or other problem or complication, commensurate with a reasonable benefit/risk ratio.

The pharmaceutically acceptable carrier which can be employed in the pharmaceutical composition of the present invention includes, but is not limited to sterile liquids, such as water and oils, including those of petroleum, animal, vegetable or synthetic origin, such as peanut oil, soybean oil, mineral oil, sesame oil and the like. Water is an exemplary carrier when the pharmaceutical composition is administered intravenously. Physiological salines as well as aqueous dextrose and glycerol solutions can also be employed as liquid carriers, particularly for injectable solutions. Suitable pharmaceutical excipients include starch, glucose, lactose, sucrose, gelatin, maltose, chalk, silica gel, sodium stearate, glycerol monostearate, talc, sodium chloride, dried skim milk, glycerol, propylene glycol, water, ethanol and the like. The pharmaceutical composition, if desired, can also contain minor amounts of wetting or emulsifying agents, or pH buffering agents. Oral formulations can include standard carriers such as pharmaceutical grades of mannitol, lactose, starch, magnesium stearate, sodium saccharine, cellulose, magnesium carbonate, etc. Examples of suitable pharmaceutical carriers are described in e.g. Remington's Pharmaceutical Sciences (1990).

The pharmaceutical composition of the present invention can act systemically and/or topically. To this end, it can be administered through a suitable route, such as through injection (intravenous, intraarterial, subcutaneous, intraperitoneal, or intramuscular injection), or transdermal administration, or administered via oral, buccal, nasal, transmucosal, topical, as an ophthalmic formulation, or via inhalation.

For these routes of administration, the pharmaceutical composition of the present invention can be administered in a suitable dosage form.

Such dosage forms include, but are not limited to tablets, capsules, lozenges, hard candies, powders, sprays, creams, salves, suppositories, gels, pastes, lotions, ointments, aqueous suspensions, injectable solutions, elixirs, and syrups.

As used herein, the term "therapeutically effective amount" refers to the amount of a compound being administered which will relieve to some extent one or more of the symptoms of the disorder being treated.

Dosage regimens may be adjusted to provide the optimum desired response. For example, a single bolus may be administered, several divided doses may be administered over time, or the dose may be proportionally reduced or increased as indicated by the exigencies of the therapeutic situation. It is to be noted that dosage values may vary with the type and severity of the condition to be alleviated, and may include single or multiple doses. It is to be further understood that for any particular subject, specific dosage regimens should be adjusted over time according to the individual need and the professional judgment of the person administering or supervising the administration of the composition.

The amount of the compound of the present invention administered will be dependent on the subject being treated, the severity of the disorder or condition, the rate of administration, the disposition of the compound and the discretion of the prescribing physician. Generally, an effective dosage is in the range of about 0.0001 to about 50 mg per kg body weight per day, for example about 0.01 to about 10 mg/kg/day, in single or divided doses. For a 70 kg human, this would amount to about 0.007 mg to about 3500 mg/day, for example about 0.7 mg to about 700 mg/day. In some instances, dosage levels below the lower limit of the aforesaid range may be more than adequate, while in other cases, still larger doses may be employed without causing any harmful side effect, provided that such larger doses are first divided into several small doses for administration throughout the day.

The content or dosage of the compound of the present invention in the pharmaceutical composition is about 0.01 mg to about 1000 mg, suitably 0.1-500 mg, preferably 0.5-300 mg, more preferably 1-150 mg, particularly preferably 1-50 mg, e.g., 1.5 mg, 2 mg, 4 mg, 10 mg, 25 mg, etc.

Unless otherwise indicated, the term "treating" or "treatment", as used herein, means reversing, alleviating, inhibiting the progress of, or preventing the disorder or condition to which such term applies, or one or more symptoms of such disorder or condition.

As used herein, the term "subject" includes a human or non-human animal. An exemplary human subject includes a human subject having a disease (such as one described herein) (referred to as a patient), or a normal subject. The term "non-human animal" as used herein includes all vertebrates, such as non-mammals (e.g. birds, amphibians, reptiles) and mammals, such as non-human primates, livestock and/or domesticated animals (such as sheep, dog, cat, cow, pig and the like).

### Examples

In order to make the object and technical solutions of the present invention more clear, the embodiments of the present invention will be described in detail with reference to the following examples. However, a person skilled in the art will understand that the following examples are only for illustrating the invention and should not be construed as limiting the scope of the invention. Where specific conditions are not specified **in** the examples, they shall be carried out according to the conventional conditions or the conditions suggested by the manufacturer. If the manufacturer is not indicated on the reagents or instruments used, they are all conventional products that can be purchased **in** the market.

**In** this application, when the chemical name and structural formula are inconsistent, the structural formula shall prevail unless it can be inferred from the context that the chemical name rather than the structural formula is correct.

**In** the conventional synthesis methods as well as examples and intermediate synthesis examples, the meanings of each abbreviation are shown **in** the following table. The structure of the compound was confirmed by nuclear magnetic resonance spectroscopy (¹H NMR) or mass spectrometry (MS).

If not specified, the reaction temperature is room temperature (20 °C ~ 30 °C).

| Abbreviations | Meanings | Abbreviations | Meanings |
|---|---|---|---|
| THF | Tetrahydrofuran | N | mol/L |
| DMF | Dimethylformamide | EA | Ethyl acetate |
| DMSO | Dimethyl sulfoxide | NMP | N-Methylpyrrolidone |
| NaOH | Sodium hydroxide | LiOH.H₂O | Lithium hydroxide monohydrate |
| NaH | Sodium hydride | Cs₂CO₃ | Cesium carbonate |
| DIPEA | N,N-diisopropylethylamine | TEA | Triethylamine |
| SOCl₂ | Dichlorosulfoxide | DCC | N,N' -dicyclohexylcarbodiimide |
| HATU | O-(7-azabenzotriazol-1-yl) -N,N,N',N'-tetramethylurea hexafluorophosphate | DMA | N,N-Dimethylacetamide |
| DCC | Dicyclohexylcarbodiimide | | |

### Example 1: preparation of 2-(2R,3S)-2-(E)-4,8-dimethylnona-3,7-dien-1-yl)-3,5-dihydroxy-2-methyl-7-oxo-3,4,7,9-tetrahydropyrano[2,3 -E]isoindol-8(2H)-yl)-5-(morpholino-4-carbonyl)oxy)benzoic acid (1-3-A); 2-(2R,3S)-2-(E)-4,8-dimethylnona-3,7-dien-1-yl)-3-hydroxy-2-methyl-5-(morpholino-4-carbonyl)oxy)-7-oxo-3 ,4,7,9-tetrahydropyrano[2,3-E]isoindol-8(2H)-yl)-5-hydroxybenzoic acid (1-3); 2-((2R,3S)-2-(E)-4,8-dimethylnona-3,7-dien-1-yl)-3-hydroxy-2-methyl-5-((morpholino-4-carbonyl)oxy)-7-oxo -3,4,7,9-tetrahydropyrano[2,3-E]isoindol-8(2H)-yl)-5-(morpholino-4-carbonyl)oxy)benzoic acid (1-3-B)

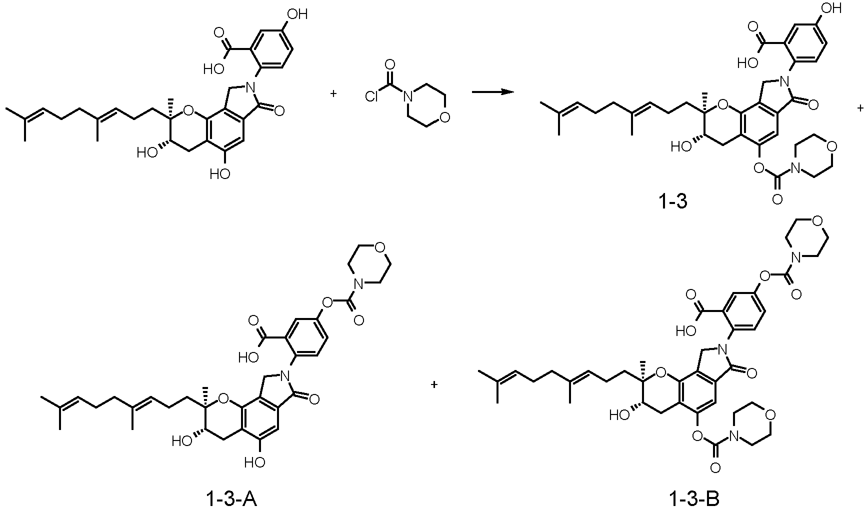

(2-(2R,3S)-2-(E)-4,8-dimethylnona-3,7-dien-1-yl)-3,5-dihydroxy-2-methyl-7-oxo-3,4,7,9-tetrahydropyrano[2, 3-E]isoindol-8(2H)-yl)-5-hydroxybenzoic acid (obtained by microbial fermentation) (20 mg, 0.038 mmol, FR) was dissolved **in** dry THF (2 mL), cooled to 0 °C with stiring. Then NaH (9.20 mg, 0.383 mmol, 60% purity) was added, and stirred for 0.5 hours. 4-morpholinoformyl chloride (17.21 mg, 0.115 mmol) was added, and the mixture was allowed to react at room temperature for 2 hours. The reaction was monitored by high performance liquid chromatography coupled with mass spectrometry. The reaction was quenched by pouring into cooled aqueous ammonium chloride solution, neutralized by dropping 3N aqueous hydrochloric acid solution, and extracted with ethyl acetate. The organic phase was dried and concentrated, followed by purification by high performance liquid chromatography to obtain compounds 1-3-A (2.34 mg), 1-3 (2.44 mg) and 1-3-B (11.63 mg).

Column: SunFire Prep C18 OBD 19 mm × 150 mm × 5.0 µm.

Mobile phase A: acetonitrile; mobile phase B: water (containing 0.05% formic acid).

| Time [min] | Mobile phase A [%] | Mobile phase B [%] | Flow rate [mL/min] |
|---|---|---|---|
| 0.00 | 0 | 100 | 28 |
| 2.00 | 30 | 70 | 28 |
| 16.00 | 90 | 10 | 28 |

1-3:
   ESI-MS (m/z): 634.7[M+H]⁺.
   ¹H NMR (400 MHz, DMSO) δ 12.71 (s, 1H), 9.95 (s, 1H), 7.34 (d, *J* = 8.6 Hz, 1H), 7.26 (d, *J* = 2.6 Hz, 1H), 7.00 (d, *J=* 6.8 Hz, 2H), 5.34 (d, *J=* 4.8 Hz, 1H), 5.13 (t, *J=* 6.7 Hz, 1H), 5.03 (t, *J=* 6.9 Hz, 1H), 4.63 (q, *J=* 16.9 Hz, 2H), 3.79 (dd, *J* = 12.0, 5.3 Hz, 1H), 3.67 (s, 6H), 3.44 (s, 2H), 2.85 (dd, *J=* 17.3, 5.1 Hz, 1H), 2.63 - 2.53 (m, 1H), 2.13 (d, *J* = 7.5 Hz, 2H), 2.04 - 1.97 (m, 2H), 1.94 - 1.86 (m, 2H), 1.58 (dd, *J=* 35.1, 11.0 Hz, 11H), 1.22 (d, *J=* 12.2 Hz, 3H).
1-3-A:
   ESI-MS (m/z): 634.7[M+H]⁺.
   ¹H NMR (400 MHz, DMSO) δ 12.71 (s, 1H), 9.95 (s, 1H), 7.34 (d, *J* = 8.6 Hz, 1H), 7.26 (d, *J* = 2.6 Hz, 1H), 7.00 (d, *J=* 6.8 Hz, 2H), 5.34 (d, *J=* 4.8 Hz, 1H), 5.13 (t, *J=* 6.7 Hz, 1H), 5.03 (t, *J=* 6.9 Hz, 1H), 4.63 (q, *J=* 16.9 Hz, 2H), 3.79 (dd, *J* = 12.0, 5.3 Hz, 1H), 3.67 (s, 6H), 3.44 (s, 2H), 2.85 (dd, *J=* 17.3, 5.1 Hz, 1H), 2.63 - 2.53 (m, 1H), 2.13 (d, *J* = 7.5 Hz, 2H), 2.04 - 1.97 (m, 2H), 1.94 - 1.86 (m, 2H), 1.58 (dd, *J=* 35.1, 11.0 Hz, 11H), 1.22 (d, *J=* 12.2 Hz, 3H).
1-3-B:
   ESI-MS (m/z): 747.8[M+H]⁺.
   ¹H NMR (400 MHz, DMSO) δ 12.71 (s, 1H), 9.95 (s, 1H), 7.34 (d, *J* = 8.6 Hz, 1H), 7.26 (d, *J* = 2.6 Hz, 1H), 7.00 (d, *J* = 6.8 Hz, 2H), 5.34 (d, *J* = 4.8 Hz, 1H), 5.13 (t, *J* = 6.7 Hz, 1H), 5.03 *(t, J=* 6.9 Hz, 1H), 4.63 (q, *J* = 16.9 Hz, 2H), 3.79 (dd, *J* = 12.0, 5.3 Hz, 1H), 3.67 (s, 6H), 3.44 (s, 2H), 2.85 (dd, *J* = 17.3, 5.1 Hz, 1H), 2.63 - 2.53 (m, 1H), 2.13 (d, *J* = 7.5 Hz, 2H), 2.04 - 1.97 (m, 2H), 1.94 - 1.86 (m, 2H), 1.58 (dd, *J* = 35.1, 11.0 Hz, 11H), 1.22 (d, *J=* 12.2 Hz, 3H).

### Example 2: preparation of (S)-2,5-bis((2R,3S)-5-((dimethylcarbamoyl)oxy)-2-((E)-4,8-dimethylnona-3,7-dien-1-yl)-3-hydroxy-2-methyl-7-oxo-3,4,7,9-tetrahydropyrano[2,3-E]isoindol-8(2H)-yl)pentanoic acid (2-1); (S)-5-((2R,3S)-5-((dimethylcarbamoyl)oxy)-2-((E)-4,8-dimethylnona-3,7-dien-1-yl)-3-hydroxy-2-methyl-7-oxo -3,4,7,9-tetrahydropyrano[2,3-E]isoindol-8(2H)-yl)-2-(((E)-4,8-dimethylnona-3,6-dien-yl)-3,5-dihydroxy-2-m ethyl-7-oxo-4,7,9-tetrahydroxypyrano[2,3-E]isoindol-8(2H)-yl)pentanoic acid (2-1-A); (S)-2-((2R,3S)-5-((dimethylcarbamoyl)oxy)-2-(((E)-4,8-dimethylnona-3,7-dien-1-yl)-3-hydroxy-2-methyl-7-ox o-3,4,7,9-tetrahydropyrano[2,3-E]isoindol-8(2H)-yl)-5-(2R,3S)-2-(E)-4,8-dimethylnona-2,7-dien-1-yl)-3,5-dih ydroxy-2-methyl-7-oxo-4,4,4,7,9-tetrahydropyrano[2,3-E]isoindol-8(2H)-yl)pentanoic acid (2-1-B)

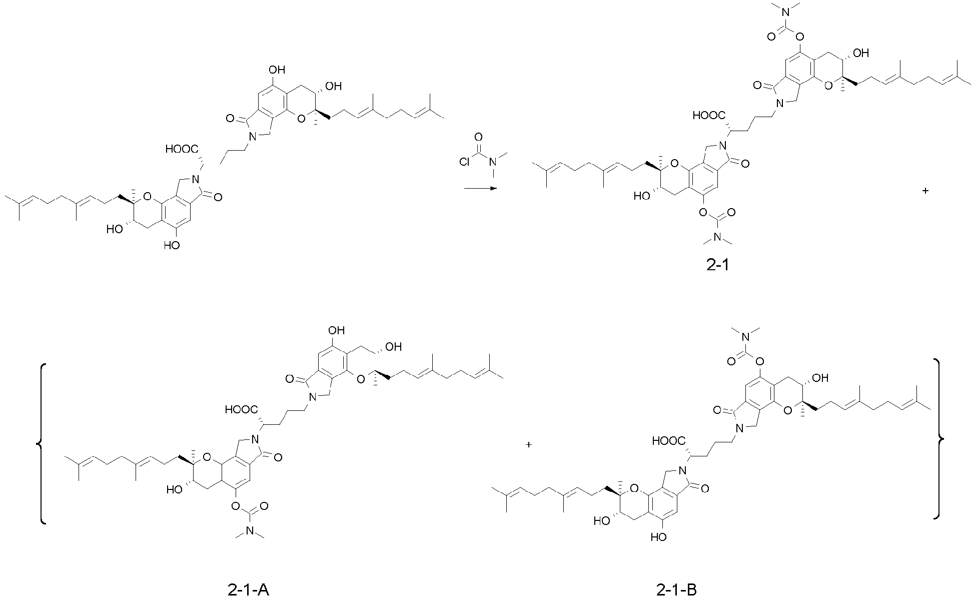

(S)-2,5-bis((2R,3S)-2-((E)-4,8-dimethylnona-3,7-dien-1-yl)-3,5-dihydroxy-2-methyl-7-oxo-3,4,7,9-tetrahydro pyrano[2,3-E]isoindol-8(2H)-yl)pentanoic acid (obtained by microbial fermentation) (200 mg, 0.230 mmol, FR) was dissolved **in** dry tetrahydrofuran (4 mL), cooled to 0 °C with stiring. Then NaH (55.23 mg, 2.3 mmol) was added, and the mixture was stirred at room temperature for 0.5 hours. Dimethylcarbamoyl chloride (123.74 mg, 1.15 mmol) was added dropwise, and the temperature was slowly raised to 40 °C for 3 hours. The reaction was monitored by high performance liquid chromatography coupled with mass spectrometry. The reaction was quenched by dropping water under ice water cooling with stirring, neutralized by dropping into 3N aqueous hydrochloric acid solution, and purified by high performance liquid chromatography to obtain 30.79 mg of a monosubstituted mixture (2-1-A and 2-1-B) and 9.94 mg of a disubstituted compound (2-1), respectively.

Column: SunFire Prep C18 OBD 19 mm × 150 mm × 5.0 µm.

Mobile phase A: acetonitrile; mobile phase B: water (containing 0.05% formic acid).

| Time [min] | Mobile phase A [%] | Mobile phase B [%] | Flow rate [mL/min] |
|---|---|---|---|
| 0.00 | 0 | 100 | 28 |
| 2.00 | 60 | 40 | 28 |
| 20.00 | 90 | 10 | 28 |

### Compound 2-1:

ESI-MS (m/z) 1011.3 [M+H]⁺.

¹H NMR (400 MHz, DMSO) δ 13.05 (s, 1H), 6.95 (s, 1H), 6.91 (s, 1H), 5.29 (t, *J* = 4.2 Hz, 2H), 5.11 (t, J = 6.6 Hz, 2H), 5.04 (t, *J* = 6.9 Hz, 2H), 4.73 (s, 1H), 4.39 - 4.23 (m, 4H), 3.78 (d, *J* = 5.0 Hz, 2H), 3.52 (t, *J=* 6.6 Hz, 2H), 3.08 (d, *J* = 2.0 Hz, 6H), 2.93 (s, 6H), 2.83 (t, *J* = 5.0 Hz, 1H), 2.78 (t, *J=* 4.9 Hz, 1H), 2.54 (d, *J* = 10.2 Hz, 1H), 2.47 (s, 1H), 2.10 (s, 4H), 2.00 (dd, *J* = 14.6, 7.3 Hz, 5H), 1.94 - 1.85 (m, 5H), 1.64 - 1.47 (m, 24H), 1.19 (d, *J* = 14.7 Hz, 6H).

### Mixture 2-1-A & 2-1-B:

ESI-MS (m/z): 940.3[M+H]+.

¹H NMR (400 MHz, DMSO) δ 12.97 (s, 2H), 9.78 (d, *J* = 21.9 Hz, 2H), 6.93 (d, *J* = 18.6 Hz, 2H), 6.64 (d, *J* = 13.0 Hz, 2H), 5.29 (t, *J* = 4.8 Hz, 2H), 5.16 (t, *J =* 4.6 Hz, 2H), 5.11 (t, *J =* 6.6 Hz, 4H), 5.04 (t, *J =* 6.3 Hz, 4H), 4.74 (d, *J=* 5.9 Hz, 2H), 4.31 (d, *J=* 8.3 Hz, 4H), 4.19 (d, *J* = 11.6 Hz, 4H), 3.80 - 3.69 (m, 4H), 3.49 (dd, *J* = 12.3, 5.9 Hz, 4H), 3.08 (d, *J=* 2.2 Hz, 6H), 2.93 (s, 6H), 2.81 (dd, *J* = 16.8, 3.7 Hz, 4H), 2.54 (s, 2H), 2.43 (d, *J* = 7.5 Hz, 2H), 1.98 (ddd, *J=* 33.5, 25.8, 18.2 Hz, 30H), 1.57 (d, *J* = 33.6 Hz, 46H), 1.18 (dd, *J* = 14.3, 11.7 Hz, 12H).

### Example 3: preparation of (S)-2,5-bis((2R,3S)-2-((E)-4,8-dimethylnona-3,7-dien-1-yl)-3-hydroxy-2-methyl-5-((morpholino-4-carbonyl)o xy)-7-oxo-3,4,7,9-tetrahydropyrano[2,3-e]isoindol-8(2H)-yl)pentanoic acid (2-2); (S)-5-((2R,3S)-2-((E)-4,8-dimethylnona-3,7-dien-1-yl)-3,5-dihydroxy-2-methyl-7-oxo-3,4,7,9-tetrahydropyran o[2,3-E]isoindol-8(2H)-yl)-2-((2R,3S)-2-(((E)-4,8-dimethylnona-3,7-dien-1-yl)-3-hydroxy-2-methyl-5-((morph olino-4-carbonyl)oxy)-7-oxo-3,4,7,9-tetrahydropyrano[2,3-E]isoindol-8(2H)-yl)pentanoic acid (2-2-A); (S)2-((2R,3S)-2-(((E)-4,8-dimethylnona-3,7-dien-1-yl)-3,5-dihydroxy-2-methyl-7-oxo-3,4,7,9-tetrahydropyran o[2,3-E]isoindol-8(2H)-yl)-5-((2R,3S)-2-((E)-4,8-dimethylnona-3,7-dien-1-yl)-3-hydroxy-2-methyl-5-((morpho lino-4-carbonyl)oxy)-7-oxo-3,4,7,9-tetrahydropyrano[2,3-E]isoindol-8(2H)-yl)pentanoic acid (2-2-B)

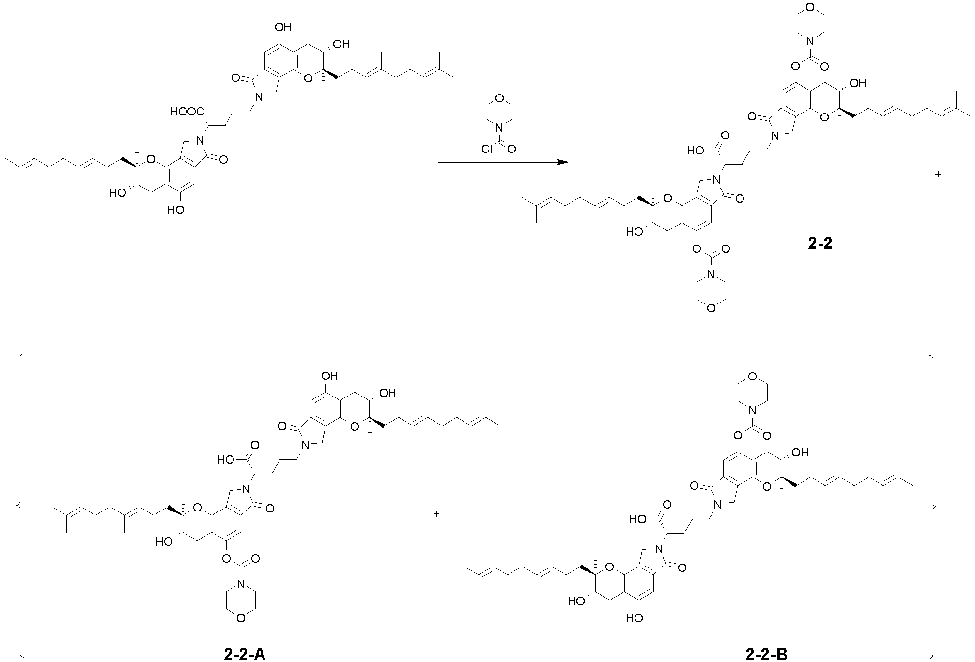

Under the protection of nitrogen, (S)-2,5-bis((2R,3S)-2-((E)-4,8-dimethylnona-3,7-dien-1-yl)-3,5-dihydroxy-2-methyl-7-oxy-3,4,7,9-tetrahydropyran o[2,3-e]isoindol-8(2H)-yl)pentanoic acid (200 mg, 0.23 mmol) was added to THF (5 mL), stirred to dissolution, and cooled to 0-5 °C with ice water. NaH (138.1 mg, 3.45 mmol, 60% content) was added, and the mixture was stirred for 10 minutes. 4-morpholinoformyl chloride (137.68 mg, 0.92 mmol) was added, the temperature was raised to 35 °C, and the mixture was allowed to react for 4 hours. The reaction solution was poured into an aqueous solution of ammonium chloride (10 ml), and the product was extracted with EA (20 ml), concentrated to give a crude product, and purified with preparative high-performance liquid chromatography to obtain the title compound 2-2 (28.0 mg) and a mixture of 2-2-A/2-2-B (21.0 mg).

Column: Waters SunFire Prep C18 OBD (5 µm * 19 mm * 150 mm).

Mobile phase A: acetonitrile; mobile phase B: water (containing 0.05% formic acid).

Retention time: 12.7-13.2 min

| Time [min] | Mobile phase A [%] | Mobile phase B [%] | Flow rate [mL/min] |
|---|---|---|---|
| 0.00 | 50 | 50 | 28 |
| 2.00 | 50 | 50 | 28 |
| 18.00 | 90 | 90 | 28 |

### Compound 2-2:

ESI- MS m/z (ESI): 1095.3 [M+H]⁺.

¹H NMR (400 MHz, DMSO-*d*₆) δ 6.99 (s, 1H), 6.95 (s, 1H), 5.29 (br, 2H), 5.11 (t, *J* = 6.4 Hz, 2H), 5.03 (t, *J* = 6.4 Hz, 2H), 4.75 - 4.72 (m, 1H), 4.40 - 4.30 (m,3H), 3.78 - 3.77(m, 2H), 3.68 - 3.58 (m, 12H), 3.52 - 3.51 (m, 2H), 3.46 - 3.42 (m, 3H), 2.85 - 2.78 (m, 2H), 2.57 - 2.54 (m, 1H), 2.16 - 2.06 (m, 4H), 2.02 - 1.89 (m, 11H), 1.65 - 1.56 (m, 13H), 1.55 -1.45 (m, 13H), 1.21 (s, 3H), 1.17 (s, 3H).

### Mixture 2-2-A & 2-2-B:

ESI- MS m/z (ESI): 982.6 [M+H]⁺.

¹H NMR (400 MHz, DMSO-*d*₆) δ 9.77 (s, 2H), 6.96 (s, 1H), 6.95 (s, 1H), 6.64 (s, 1H), 6.62 (s, 1H), 5.29 (br, 2H), 5.15 -5.04 (m, 8H), 4.63 - 4.54 (m, 2H), 4.53 - 4.43 (m, 2H), 4.37 - 4.06 (m, 8H), 3.80 - 3.72(m, 4H), 3.69 - 3.58 (m, 10H), 3.54 - 3.34 (m, 12H), 2.84 - 2.78 (m, 4H), 2.55 - 2.53 (m, 2H), 2.48 - 2.41 (m, 2H), 2.15 - 2.05 (s, 6H), 2.05 - 1.87 (m, 14H), 1.85 - 1.72 (m, 3H), 1.61 - 1.53 (m, 38H), 1.23 - 1.14 (m, 12H).

### Example 4: preparation of (S)-2,5-bis((2R,3S)-2-((E)-4,8-dimethylnona-3,7-dien-1-yl)-3-hydroxy-5-((isopropylcarbamoyl)oxy)-2-methyl-7-oxo-3,4,7,9-tetrahydropyrano[2,3-e]isoindol-8(2H)-yl)pentanoic acid (2-3)

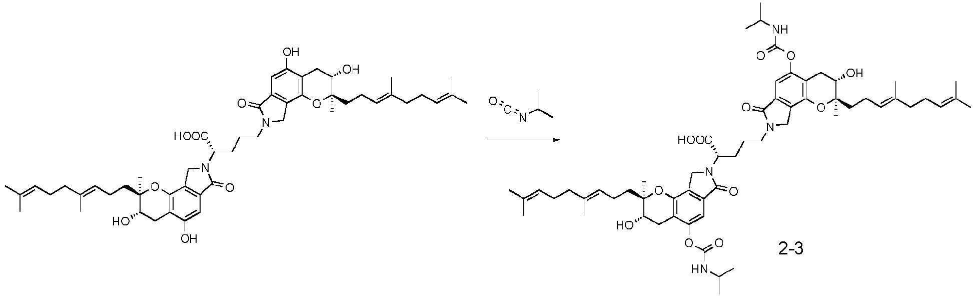

(S)-2,5-bis((2R,3S)-2-((E)-4,8-dimethylnona-3,7-dien-1-yl)-3,7-hydroxy-2-methyl-7-oxo-3,4,7,9-tetrahydropy rano[2,3-e]isoindol-8(2H)-yl)pentanoic acid (100 mg, 0.115 mmol) was dissolved **in** DMF (2 mL), then triethylamine (23.29 mg, 0.230 mmol) and isopropyl isocyanate (29.38 mg, 0.345 mmol) were added, and the reaction was stirred at 25 °C for 16 hours. The reaction was monitored by high performance liquid chromatography coupled with mass spectrometry. The reaction solution was purified by high performance liquid chromatography to obtain 48 mg of the title compound.

Column: SunFire Prep C18 OBD 19 mm × 150 mm × 5.0 µm.

Mobile phase A: acetonitrile; mobile phase B: water (containing 0.05% ammonium bicarbonate).

| Time [min] | Mobile phase A [%] | Mobile phase B [%] | Flow rate [mL/min] |
|---|---|---|---|
| 0.00 | 0 | 100 | 28 |
| 12.00 | 60 | 40 | 28 |
| 18.00 | 90 | 10 | 28 |

ESI-MS (m/z):1039.5[M+H]⁺.

¹H NMR (400 MHz, DMSO-*d*₆) δ 7.77 (dd, *J =* 7.6, 4.6 Hz, 2H), 6.87 (d, *J* = 11.7 Hz, 2H), 5.31 (s, 2H), 5.16 - 5.02 (m, 4H), 4.64 (s, 1H), 4.44 - 4.23 (m, 4H), 3.77 (s, 2H), 3.66 (dd, J = 12.5, 6.4 Hz, 2H), 3.51 (t, *J* = 6.5 Hz, 2H), 2.86 - 2.74 (m, 2H), 2.54 (s, 1H), 2.43 -2.41 (m, 1H), 2.11 (d, *J* = 8.3 Hz, 4H), 1.94 (tt, *J* = 25.2, 12.7 Hz, 10H), 1.57 (d, *J* = 31.6 Hz, 24H), 1.16 (dd, *J* = 17.0, 9.8 Hz, 18H).

### Example 5: preparation of (S)-2,5-bis((2R,3S)-2-((E)-4,8-dimethylnona-3,7-dien-1-yl)-3-hydroxy-5-((methoxycarbonyl)oxy)-2-methyl-7-oxo-3,4,7,9-tetrahydropyrano[2,3-E]isoindol-8(2H)-yl)pentanoic acid (2-4)

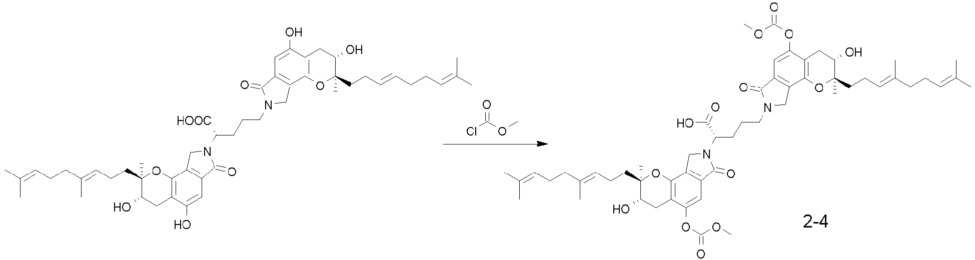

(S)-5-((2R,3S)-2-((E)-4,8-dimethylnona-3,7-dien-1-yl)-3,5-dihydroxy-2-methyl-7-oxo-3,4,7,9-tetrahydropyran o[2,3-E]isoindol-8(2H)-yl)-2-((2S,3S)-2-((E)-4,8-dimethylnona-3,7-dien-1-yl)-3,5-dihydroxy-2-methyl-7,9-tetrahy dropyrano[2,3-E]isoindol-8(2H)-pentanoic acid (250 mg, 0.288 mmol) was added to tetrahydrofuran (8 mL) and water (2 mL), stirred to dissolution, and was cooled to 0 to 5 °C with ice water (2 mL). 10% aqueous NaOH solution was dropped to adjust the pH to 11, methyl chloroformate (163.1 mg, 1.73 mmol) was dropped, and 10% aqueous NaOH solution was dropped at the same time to control the pH to 10 ~ 11. After dropping, the mixture was allowed to react at room temperature for 2 hours. The pH was adjusted to 3-4 with aqueous citric acid solution. The product was extracted with EA (30 ml), and the organic phase was concentrated to obtain the crude product, which was purified by preparative high performance liquid chromatography to obtain the title compound 2-4 (130.0 mg).

Column: Waters SunFire Prep C18 OBD (5 µm * 19 mm * 150 mm).

Mobile phase A: acetonitrile; mobile phase B: water (0.05% ammonium formate in water).

Retention time: 7-9 min

| Time [min] | Mobile phase A [%] | Mobile phase B [%] | Flow rate [mL/min] |
|---|---|---|---|
| 0.00 | 15 | 85 | 28 |
| 2.00 | 15 | 85 | 28 |
| 18.00 | 95 | 5 | 28 |

The structural characterization data are as follows:

MS m/z (ESI): 985.4 [M+H]⁺.

¹H NMR (400 MHz, DMSO-*d*₆) δ 8.29 (s, 1H), 7.05 (s, 1H), 7.05 (s, 1H), 5.32 (s, 2H), 5.14 - 5.11(m, 2H), 5.09 - 5.02 (m, 2H), 4.54 - 4.50 (m, 3H), 4.31 (s, 2H), 4.24 - 4.19 (m, 2H), 3.85 (d, *J=* 1.2 Hz, 6H), 3.81 - 3.76 (m, 2H), 3.54 - 3.48 (m, 4H), 2.84 - 2.78 (m, 2H), 2.55 - 2.51 (m, 2H), 2.14 - 2.08 (m, 4H), 2.13 - 1.96 (m, 4H), 1.94 - 1.88 (m, 4H), 1.79 -1.70 (m, 2H), 1.61-1.57 (m, 9H), 1.56 -1.50 (m, 13H), 1.21 (s, 3H) , 1.18 (s, 3H).

### Example 6: preparation of (S)-2,5-bis((2R,3S)-2-((E)-4,8-dimethylnona-3,7-dien-1-yl)-5-((1,1-dioxothiomorpholino-4-carbonyl)oxy)-3-hy droxy-2-methyl-7-oxo-3,4,7,9-tetrahydropyrano[2,3-E]isoindol-8(2H)-yl)pentanoic acid (2-5); (S)-5-((2R,3S)-2-((E)-4,8-dimethylnona-3,7-dien-1-yl)-3,5-dihydroxy-2-methyl-7-oxo-3,4,7,9-tetrahydropyran o[2,3-E]isoindol-8(2H)-yl)-2-(2R,3S)-3-(((E)-4,6-dimethylhept-3,7-dien-1-yl)-5-((1,1-dioxothiomorpholino-4-c arbonyl)oxy)-3-hydroxy-2-methyl-7-oxo-3,4,7,9-tetrahydropyrano[2,3-E]isoindol-8(2H)-yl)pentanoic acid (2 -5-A);

### (S)-2-(2R,3S)-2-(E)-4,8-dimethylnona-3,7-dien-1-yl)-3,5-dihydroxy-2-methyl-7-oxo-3,4,7,9-tetrahydropyrano [2,3-E]isoindol-8(2H)-yl)-5-((2R,3S)-2-((E)-4.8-dimethylnon-2,7-dien-1-yl)-5-(1,1-dioxothiomorpholino-4-car bonyl)oxy)-3-hydroxy-2-methyl-7-oxo-3,4,7,9-tetrahydropyrano[2,3-E]isoindol-8(2H)-yl)pentanoic acid (2-5-B)

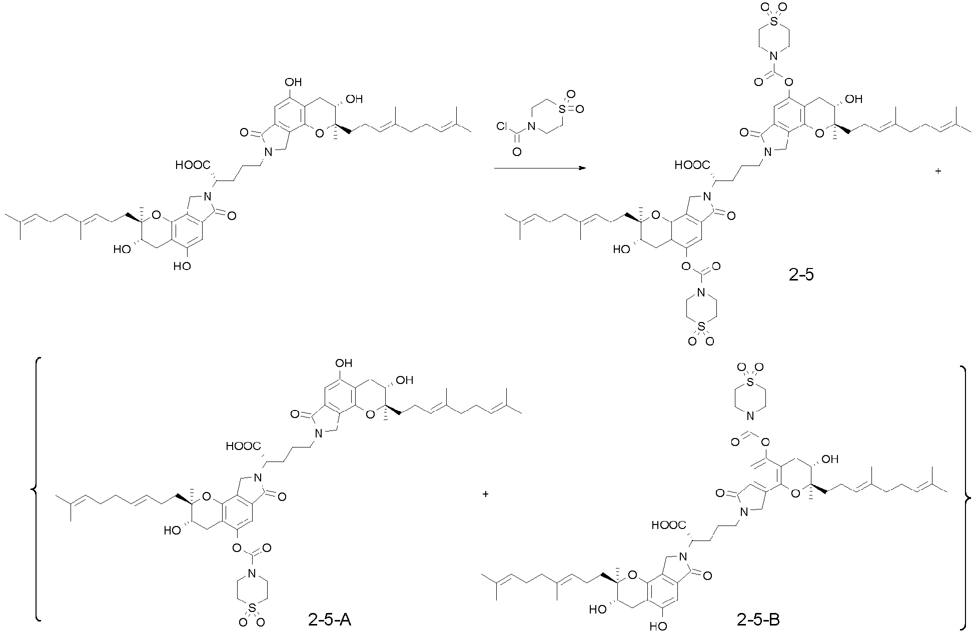

(S)-2,5-bis((2R,3S)-2-((E)-4,8-dimethylnona-3,7-dien-1-yl)-3,5-dihydroxy-2-methyl-7-oxo-3,4,7,9-tetrahydro pyrano[2,3-E]isoindol-8(2H)-yl)pentanoic acid (100 mg, 0.115 mmol, FR) was dissolved **in** dry THF (4 mL), and cooled to 0 °C with stiring. Then NaH (46.03 mg, 1.15 mmol, 60% purity) was added, and the reaction was stirred for 0.5 hours. Thiomorpholine-1,1-dioxide-4-carbonyl chloride (90.96 mg, 0.460 mmol) was added, the temperature was slowly raised to room temperature, and the mixture was allowed to react for 4 hours. The reaction was monitored by high performance liquid chromatography coupled with mass spectrometry. The reaction was quenched by pouring into cooled aqueous ammonium chloride solution, neutralized by dropping 3N aqueous hydrochloric acid solution, and extracted with ethyl acetate. The organic phase was dried and concentrated and purified by high performance liquid chromatography to obtain disubstituted compound 2-5 (54.46 mg) and a monosubstituted mixture of 2-5-A and 2-5-B (16.96 mg).

Column: SunFire Prep C18 OBD 19 mm × 150 mm × 5.0 µm.

Mobile phase A: acetonitrile; mobile phase B: water (containing 0.05% formic acid).

| Time [min] | Mobile phase A [%] | Mobile phase B [%] | Flow rate [mL/min] |
|---|---|---|---|
| 0.00 | 0 | 100 | 24 |
| 2.00 | 75 | 25 | 24 |
| 20.00 | 90 | 10 | 24 |

### Compounds 2-5:

ESI-MS (m/z): 1191.5[M+H]⁺.

¹H NMR (400 MHz, DMSO) δ 12.95 (s, 1H), 7.18 (d, *J* = 17.7 Hz, 2H), 5.34 - 5.23 (m, 2H), 5.08 (dt, *J* = 27.6, 6.9 Hz, 4H), 4.79 (s, 1H), 4.33 (d, *J =* 9.4 Hz, 4H), 4.03 (s, 4H), 3.93 - 3.74 (m, 6H), 3.53 (s, 2H), 3.37 (s, 4H), 3.27 (s, 4H), 3.07 (s, 1H), 2.91 - 2.80 (m, 2H), 2.55 (s, 2H), 2.11 (d, *J =* 8.4 Hz, 4H), 1.95 (dt, *J =* 15.0, 7.2 Hz, 10H), 1.73 - 1.41 (m, 23H), 1.20 (d, *J* = 16.0 Hz, 6H).

### Mixture 2-5-A & 2-5-B:

ESI-MS (m/z): 1030.3[M+H]⁺.

¹H NMR (400 MHz, DMSO) δ 12.91 (s, 2H), 9.78 (d, *J* = 22.7 Hz, 2H), 7.18 (d, *J* = 18.8 Hz, 2H), 6.64 (d, *J* = 13.3 Hz, 2H), 5.29 (s, 2H), 5.08 (d, *J* = 27.6 Hz, 8H), 4.80 - 4.71 (m, 2H), 4.32 (d, *J* = 7.6 Hz, 6H), 4.19 (d, *J* = 9.7 Hz, 2H), 4.03 (s, 6H), 3.86 (s, 6H), 3.77 (s, 4H), 3.55 - 3.47 (m, 4H), 3.28 (s, 6H), 2.85 (dd, *J=* 23.5, 11.6 Hz, 4H), 2.56 (d, *J* = 8.3 Hz, 4H), 2.27 - 1.78 (m, 30H), 1.57 (d, *J* = 33.8 Hz, 46H), 1.18 (t, *J* = 14.3 Hz, 12H).

### Example 7: synthesis of (S)-2,5-bis((2R,3S)-2-((E)-4,8-dimethylnona-3,7-dien-1-yl)-3-hydroxy-2-methyl-7-oxo-5-((thiomorpholino-4-c arbonyl)oxy)-3,4,7,9-tetrahydropyrano[2,3-E]isoindol-8(2H)-yl)pentanoic acid (2-7)

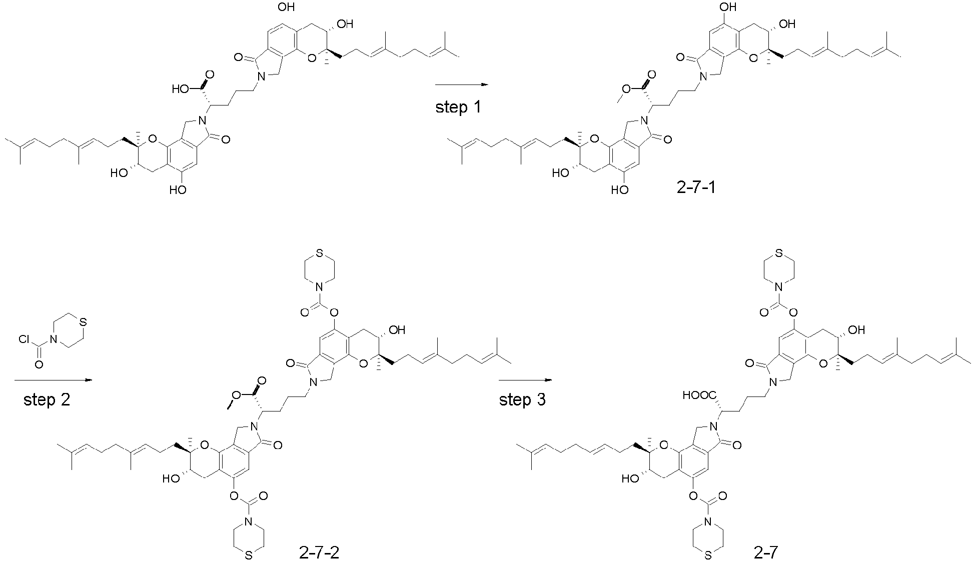

### Step 1: preparation of methyl (S)-2,5-bis((2R,3S)-2-((E)-4,8-dimethylnona-3,7-dien-1-yl)-3,5-dihydroxy-2-methyl-7-oxo-3,4,7,9-tetrahydrop yrano[2,3-E]isoindol-8(2H)-ylpentanoate (2-7-1)

(S)-5-((2R,3S)-2-((E)-4,8-dimethylnona-3,7-dien-1-yl)-3,5-dihydroxy-2-methyl-7-oxo-3,4,7,9-tetrahydropyran o[2,3-E]isoindol-8(2H)-yl)-2-((2S,3S)-2-(((E)-4,8-dimethylnona-3,7-dien-1-yl)-3,5-dihydroxy-2-methyl-7,9-tetrahy dropyrano[2,3-E]isoindol-8(2H)-pentanoic acid (1.00 g, 1.15 mmol) was dissolved in MeOH (20 mL), and cooled to -5 to -0 °C. SOCl₂ (1.37 g, 11.51 mmol) was slowly dropped, and the mixture was allowed to react at 0 ~ 5 °C for 4 hours. The reaction solution was added to a saturated aqueous solution of sodium bicarbonate (40 mL)/ethyl acetate (80 ml) at a temperature controlled at 0-10 °C. The organic phase was separated, washed with brine, dried with anhydrous sodium sulfate, and filtered, and the filtrate was concentrated to obtain the title compound (960 mg).

### Step 2: synthesis of methyl (S)-2,5-bis((2R,3S)-2-((E)-4,8-dimethylnona-3,7-dien-1-yl)-3-hydroxy-2-methyl-7-oxo-5-((thiomorpholino-4-c arbonyl)oxy)-3,4,7,9-tetrahydropyrano[2,3-E]isoindol-8(2H)-yl)pentanoate (2-7-2)

Compound 2-7-1 (50 mg, 0.057 mmol) and thiomorpholine-4-formyl chloride (28.13 mg, 0.170 mmol) were dissolved in N-methylpyrrolidone (2 mL), cesium carbonate (55.34 mg, 0.170 mmol) was added, and the reaction was stirred for 4 hours. The reaction was monitored by high performance liquid chromatography coupled with mass spectrometry. The reaction solution was quenched by pouring into cooled aqueous ammonium chloride solution, neutralized by dropping 0.1 N aqueous hydrochloric acid solution, and extracted with ethyl acetate. The organic phase was washed with saturated aqueous sodium bicarbonate solution and dried to obtain 60 mg of crude product.

### Step 3: synthesis of (S)-2,5-bis((2R,3S)-2-((E)-4,8-dimethylnona-3,7-dien-1-yl)-3-hydroxy-2-methyl-7-oxo-5-((thiomorpholino-4-c arbonyl)oxy)-3,4,7,9-tetrahydropyrano[2,3-E]isoindol-8(2H)-yl)pentanoic acid (2-7)

The crude compound 2-7-2 (60 mg, 0.053 mmol) was dissolved in methanol (2 mL) and water (0.5 mL), lithium hydroxide (8.82 mg, 0.210 mmol) was added under stirring, and the mixture was allowed to react for 4 hours. The reaction was monitored by high performance liquid chromatography coupled with mass spectrometry. 3N aqueous hydrochloric acid solution was dropped for neutralization, followed by purification via high performance liquid chromatography (HPLC) to obtain 22.24 mg of the title compound.

Column: SunFire Prep C18 OBD 19 mm × 150 mm × 5.0 µm.

Mobile phase A: acetonitrile; mobile phase B: water (containing 0.05% formic acid).

| Time [min] | Mobile phase A [%] | Mobile phase B [%] | Flow rate [mL/min] |
|---|---|---|---|
| 0.00 | 0 | 100 | 24 |
| 2.00 | 10 | 90 | 24 |
| 18.00 | 90 | 10 | 24 |

ESI-MS (m/z):1128.6[M+H]⁺.

¹H NMR (400 MHz, DMSO) δ 12.97 (s, 1H), 6.99 (d, *J* = 18.8 Hz, 2H), 5.29 (t, *J* = 4.4 Hz, 2H), 5.08 (dt, *J* = 28.3, 6.8 Hz, 4H), 4.83 - 4.74 (m, 1H), 4.32 (d, *J* = 10.7 Hz, 4H), 3.88 (s, 4H), 3.83 - 3.63 (m, 6H), 3.53 (t, *J* = 6.6 Hz, 2H), 3.31 (s, 2H), 2.80 (s, 2H), 2.77 - 2.61 (m, 8H), 2.52 (s, 3H), 2.33 (dd, *J* = 3.6, 1.8 Hz, 1H), 2.11 (d, *J=* 8.1 Hz, 4H), 1.95 (dt, *J* = 15.0, 7.2 Hz, 9H), 1.59 (d, *J* = 14.7 Hz, 10H), 1.53 (s, 11H), 1.20 (d, *J* = 15.7 Hz, 6H).

### Example 8: preparation of (S)-2,5-bis((2R,3S)-2-((E)-4,8-dimethylnona-3,7-dien-1-yl)-3-hydroxy-2-methyl-5-((methyl(tetrahydro-2H-py ran-4-yl)carbamoyl)oxy)-7-oxo-3,4,7,9-tetrahydropyrano[2,3-E]isoindol-8(2H)-yl)pentanoic acid (2-8)

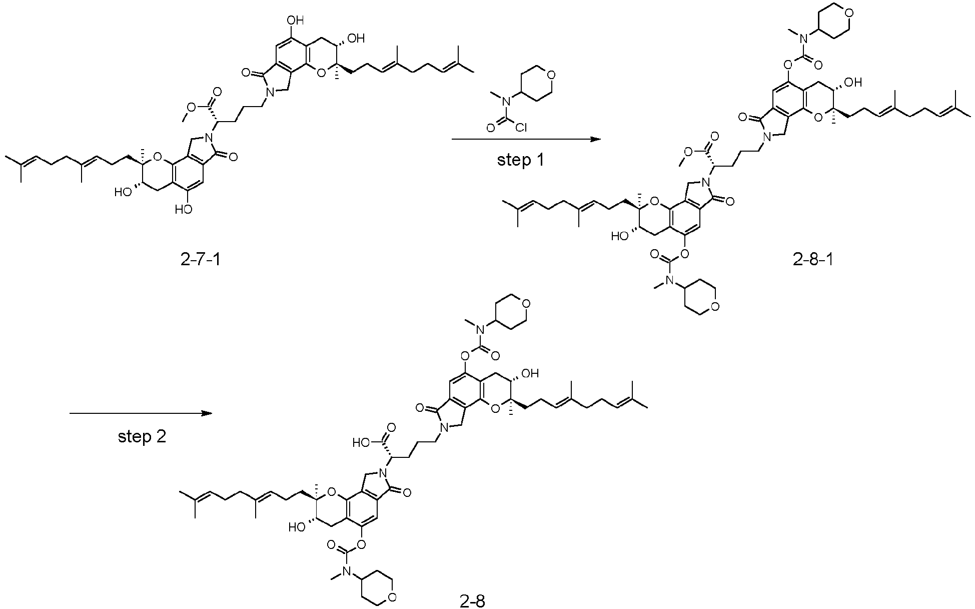

### Step 1: preparation of methyl (S)-2,5-bis((2R,3S)-2-((E)-4,8-dimethylnona-3,7-dien-1-yl)-3-hydroxy-2-methyl-5-(methyl(tetrahydro-2H-pyr an-4-yl)carbamoyl)oxy)-7-oxo-3,4,7,9-tetrahydropyrano[2,3-e]isoindol-8(2H)-yl)pentanoate (2-8-1)

Compound 2-7-1 (50 mg, 0.057 mmol) was added to NMP (2 mL), and was cooled to 0-5 °C with ice water. Cs₂CO₃ (55.34 mg, 0.17 mmol) was added, and the mixture was stirred for 10 minutes. N-methyl-N-tetrahydropyran-4-yl-carbamoyl chloride (30.17 mg, 0.17 mmol,) was added, and the mixture was allowed to react at 25 °C for 4 hours. The reaction solution was poured into ethyl acetate (20 ml)/0.1 N aqueous hydrochloric acid solution (6 ml). The organic phase was separated, washed with saturated aqueous sodium bicarbonate solution (5 ml), washed with brine (5 ml), dried, and concentrated to obtain a crude product of the title compound (82.0 mg), which was directly used in the next reaction.

### Step 2: preparation of (S)-2,5-bis((2R,3S)-2-((E)-4,8-dimethylnona-3,7-dien-1-yl) -3-hydroxy-2-methyl-5-(methyl(tetrahydro-2H-pyran-4-yl)carbamoyl)oxy)-7-oxo-3,4,7,9-tetrahydropyrano[2 ,3-e]isoindol-8(2H)-yl)pentanoic acid (2-8)

Compound 2-8-1 (80 mg, 0.055 mmol) was added to methanol (2 mL) and water (0.7 mL). LiOH.H₂O (9.22 mg, 0.22 mmol) was added, and the mixture was allowed to react at room temperature for 2 hours. To the system, 1 N hydrochloric acid was added to adjust to pH 3 ~ 4, ethyl acetate (20 ml)/water (50 ml) was added, and the mixture was stirred for 15 min. The organic phase was separated, washed with brine, dried and concentrated to obtain the crude product, which was purified with preparative high performance liquid chromatography to obtain 17 mg of the title compound.

Column: Waters SunFire Prep C18 OBD (5 µm * 19 mm * 150 mm).

Mobile phase A: acetonitrile; mobile phase B: water (containing 0.05% formic acid).

Retention time: 8-10 min.

| Time [min] | Mobile phase A [%] | Mobile phase B [%] | Flow rate [mL/min] |
|---|---|---|---|
| 0.00 | 50 | 50 | 24 |
| 2.00 | 50 | 50 | 24 |
| 18.00 | 95 | 5 | 24 |

Structural characterization data are presented below:

MS m/z (ESI): 1151.7 [M+H]⁺.

¹H NMR (400 MHz, DMSO-*d*₆) δ 6.96 (s, 1H), 6.92 (s, 1H), 5.28 (t, *J* = 4.7 Hz, 2H), 5.13 - 5.10 (m, 2H), 5.06 - 5.02 (m, 2H), 4.78 - 4.73 (m, 1H), 4.33 - 4.30 (m, 4H), 4.25 - 4.06 (m, 2H), 3.95 - 3.92 (m, 4H), 3.77 - 3.73 (m, 2H), 3.54 - 3.51 (m, 2H), 3.40 - 3.38 (m, 3H), 3.00 - 2.92 (m, 3H), 2.84 - 2.76 (m, 4H), 2.12 - 2.10 (m, 4H), 2.03 - 1.96 (m, 4H), 1.94 - 1.87 (m, 5H), 1.85 - 1.76 (m, 3H), 1.72 - 1.70 (m, 2H), 1.65 - 1.55 (m, 13H), 1.55 - 1.50 (m, 12H), 1.21 (s, 3H), 1.17 (s, 3H).

### Example 9: preparation of (S)-2,5-bis((2R,3S)-2-((E)-4,8-dimethylnona-3,7-dien-1-yl)-3-hydroxy-2-methyl-7-oxo-5-((piperidin-1-carbon yl)oxy)-3,4,7,9-tetrahydropyrano[2,3-E]isoindol-8(2H)-yl)pentanoic acid (2-9)

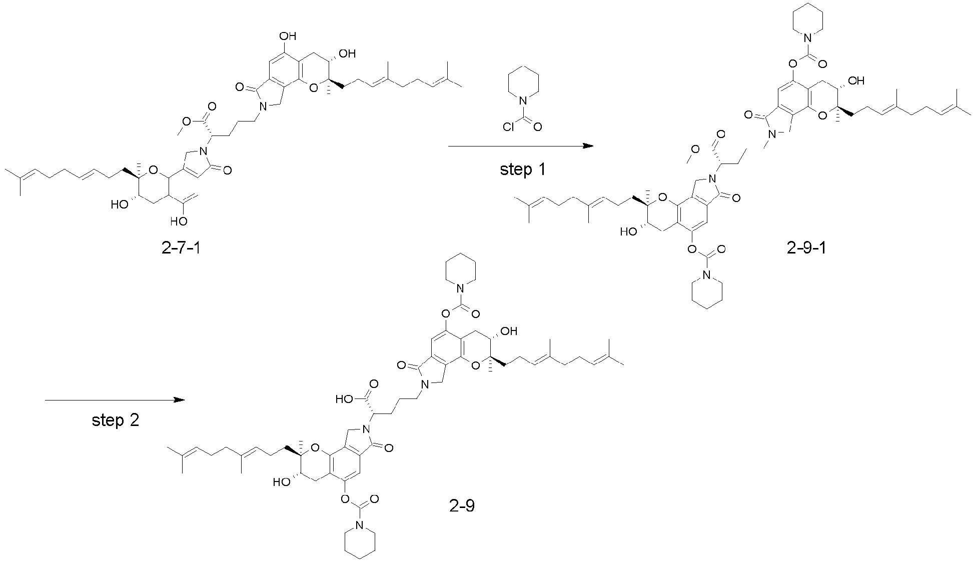

### Step 1: preparation of (2R,2'R,3S,3S)-((S)-5-methoxy-5-oxopentan-1,4-diyl)bis(2-((E)-4,8-dimethylnona-3,7-dien-1-yl)-3-hydroxy-2-methyl-3,4,7,9-tetrahydropyrano[2,3-E]isoindol-8,5-diyl)bis(piperidine-1-carboxylate) (2-9-1)

Compound 2-7-1 (50 mg, 0.057 mmol) was added to NMP (2 mL), and was cooled to 0-5 °C with ice water. Cs₂CO₃ (73.8 mg, 0.23 mmol) was added, and the mixture was stirred for 10 minutes. 1-piperidineformyl chloride (33.4 mg, 0.23 mmol) was added, and the mixture was allowed to react at 25 °C for 4 hours. The reaction solution was poured into ethyl acetate (20 ml)/0.1 N aqueous hydrochloric acid solution (6 ml). The organic phase was separated, washed with saturated aqueous sodium bicarbonate solution (5 ml), washed with brine (5 ml), dried, and concentrated to obtain a crude product of the title compound (62.0 mg), which was directly used in the next reaction.

### Step 2: preparation of (S)-2,5-bis((2R,3S)-2-((E)-4,8-dimethylnona-3,7-dien-1-yl)-3-hydroxy-2-methyl-7-oxo-5-((piperidin-1-carbon yl)oxy)-3,4,7,9-tetrahydropyrano[2,3-E]isoindol-8(2H)-yl)pentanoic acid (2-9)

Compound 2-9-1 (62.0 mg, 0.056 mmol) was added to methanol (3 mL) and water (1 mL). LiOH.H₂O (11.8 mg, 0.028 mol) was added, and the mixture was allowed to react at room temperature for 2 hours. To the system, 1 N hydrochloric acid was added to adjust to pH 3 ~ 4, ethyl acetate (30 ml)/water (10 ml) was added, and the mixture was stirred for 15 min. The organic phase was separated, washed with brine, dried and concentrated to obtain the crude product, which was purified by preparative high performance liquid chromatography to obtain 5.7 mg of the title compound.

Column: Waters SunFire Prep C18 OBD (5 µm * 19 mm * 150 mm).

Mobile phase A: acetonitrile; mobile phase B: water (containing 0.05% formic acid).

Retention time: 15.5-17.5 min.

| Time [min] | Mobile phase A [%] | Mobile phase B [%] | Flow rate [mL/min] |
|---|---|---|---|
| 0.00 | 70 | 30 | 24 |
| 2.00 | 70 | 30 | 24 |
| 18.00 | 100 | 0 | 24 |

### Structural characterization data are presented below:

MS m/z (ESI): 1091.6 [M+H]⁺.

¹H NMR (400 MHz, DMSO-*d*₆) δ 6.95 (s, 1H), 6.90 (s, 1H), 5.34 -5.29 (m, 2H), 5.16 - 5.00 (m, 4H), 4.81 - 4.73 (m, 1H), 4.38- 4.27 (m, 4H), 3.82 - 3.77 (m, 2H), 3.59 -3.51 (m, 6H), 2.82 - 2.80 (m, 2H), 2.17 - 2.10 (s, 4H), 2.04 - 1.86 (m, 10H), 1.69- 1.53 (m, 37H), 1.21 (s, 3H), 1.17 (s, 3H).

### Example 10: Preparation of (S)-2,5-bis((2R,3S)-2-((E)-4,8-dimethylnona-3,7-dien-1-yl)-3-hydroxy-2-methyl-5-((morpholinosulfonyl)oxy)-7-oxo-3,4,7,9-tetrahydropyrano[2,3-E]isoindol-8(2H)-yl)pentanoic acid (2-10); (S)-5-((2R,3S)-2-((E)-4,8-dimethylnona-3,7-dien-1-yl)-3,5-dihydroxy-2-methyl-7-oxo-3,4,7,9-tetrahydropyran o[2,3-E]isoindol-8(2H)-yl)-2-(2R,3S)-3-(((E)-4,7-dimethyldeca-3,7-dien-1-yl)-3-hydroxy-2-methyl-5-(((morph olinosulfonyl)oxy)-7-oxo-3,4,7,9-tetrahydropyrano[2,3-E]isoindol-8(2H)-yl)pentanoic acid (2-10-A); (S)-2-(2R,3S)-2-(E)-4,8-dimethylnona-3,7-dien-1-yl)-3,5-dihydroxy-2-methyl-7-oxo-3,4,7,9-tetrahydropyrano [2,3-E]isoindol-8(2H)-yl)-5-((2R,3S)-2-((E)-4.8-dimethylnona-4,7-dien-1-yl)-3-hydroxy-2-methyl-5-((morphol inosulfonyl)oxy)-7-oxo-3,6-tetrahydropyrano[2,3-E]isoindol-8(2H)-yl)pentanoic acid (2-10-B)

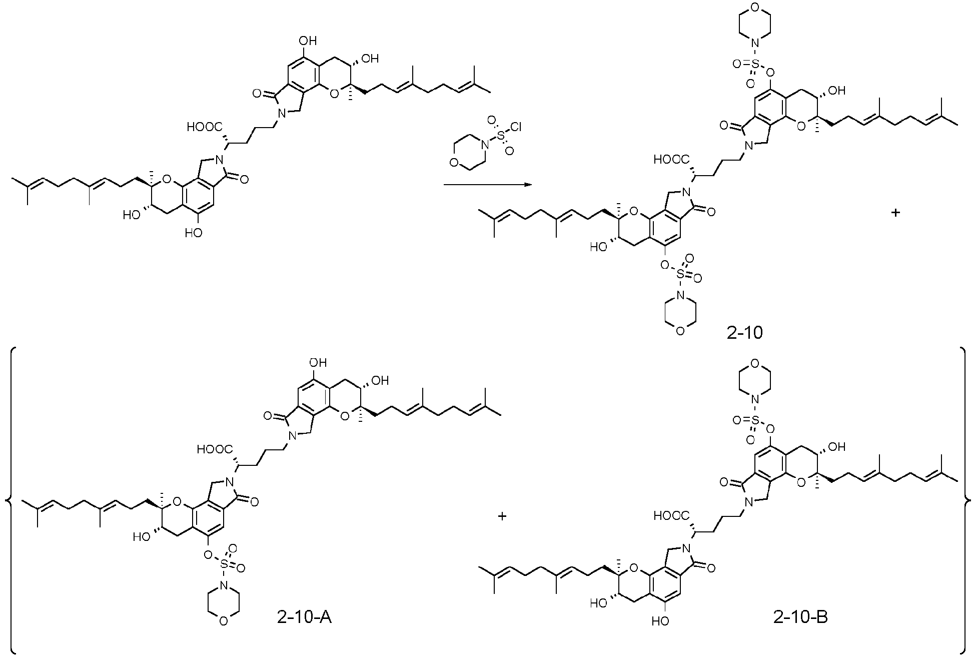

(S)-2,5-bis((2R,3S)-2-((E)-4,8-dimethylnona-3,7-dien-1-yl)-3,5-dihydroxy-2-methyl-7-oxo-3,4,7,9-tetrahydro pyrano[2,3-E]isoindol-8(2H)-yl)pentanoic acid (50 mg, 0.058 mmol) was dissolved **in** dry THF (2 mL), cooled to 0 °C with stiring. NaH (13.81 mg, 0.575 mmol, 60% purity) was added, and stirred for 0.5 h. 4-morpholinesulfonyl chloride (90.96 mg, 0.460 mmol) was added, and the temperature was slowly raised to room temperature for 4 hours. The reaction was monitored by high performance liquid chromatography coupled with mass spectrometry. The reaction was quenched by pouring into cooled aqueous ammonium chloride solution, neutralized by dropping 3N aqueous hydrochloric acid solution, and extracted with dichloromethane. The organic phase was dried, concentrated, and purified by high performance liquid chromatography to obtain a disubstituted compound 2-10 (11.74 mg) and a monosubstituted mixture of 2-10-A and 2-10-B (5.03 mg).

Column: SunFire Prep C18 OBD 19 mm × 150 mm × 5.0 µm.

Mobile phase A: acetonitrile; mobile phase B: water (containing 0.05% formic acid).

| Time [min] | Mobile phase A [%] | Mobile phase B [%] | Flow rate [mL/min] |
|---|---|---|---|
| 4.00 | 0 | 100 | 24 |
| 16.00 | 30 | 90 | 24 |
| 29.00 | 90 | 10 | 24 |

### Compounds 2-10:

ESI-MS (m/z): 1167.8[M+H]⁺.

¹H NMR (400 MHz, DMSO) δ 13.02 (s, 1H), 7.14 (d, *J* = 15.8 Hz, 2H), 5.36 (t, *J* = 4.9 Hz, 2H), 5.05 (dd, *J* = 21.2, 14.3 Hz, 4H), 4.80 - 4.74 (m, 1H), 4.35 (d, *J* = 8.8 Hz, 4H), 3.86 - 3.76 (m, 2H), 3.73 (dd, *J* = 5.7, 3.0 Hz, 8H), 3.53 (s, 2H), 3.41 (dd, *J=* 9.4, 4.7 Hz, 7H), 3.02 (d, *J* = 17.6 Hz, 2H), 2.72 (s, 2H), 2.11 (dd, *J* = 15.4, 7.3 Hz, 4H), 1.94 (dt, *J* = 14.8, 7.1 Hz, 11H), 1.56 (d, *J=* 33.5 Hz, 24H), 1.23 (d, *J* = 15.1 Hz, 6H).

### Mixture 2-10-A & 2-10-B:

ESI-MS (m/z): 1018.5[M+H]⁺.

¹H NMR (400 MHz, DMSO) δ 12.90 (s, 2H), 9.77 (d, *J* = 21.9 Hz, 2H), 7.13 (d, *J* = 16.1 Hz, 2H), 6.64 (d,*J* = 12.8 Hz, 2H), 5.35 (t, *J* = 4.7 Hz, 2H), 5.15 (t, *J =* 4.9 Hz, 2H), 5.11 (s, 4H), 5.03 (d, *J* = 6.1 Hz, 4H), 4.74 (s, 2H), 4.35 (d, *J=* 8.9 Hz, 4H), 4.19 (d, *J* = 10.1 Hz, 4H), 3.81 (s, 2H), 3.73 (d, *J* = 3.1 Hz, 8H), 3.54 - 3.45 (m, 4H), 3.41 (dd, *J* = 9.4, 4.8 Hz, 8H), 3.02 (d, *J* = 18.4 Hz, 3H), 2.77 (dd, *J* = 32.7, 13.8 Hz, 6H), 2.43 (d, *J* = 7.5 Hz, 2H), 2.11 (d, *J=* 6.3 Hz, 10H), 1.94 (dt, *J* = 14.7, 7.0 Hz, 20H), 1.56 (d, *J* = 34.0 Hz, 46H), 1.19 (dd, *J* = 24.6, 14.0 Hz, 12H).

### Example 11: preparation of (S)-2,5-bis((2R,3S)-5-([1,4'-bipiperidine]-1'-carbonyl)oxy)-2-(((E)-4,8-dimethylnona-3,7-dien-1-yl)-3-hydroxy -2-methyl-7-oxo-3,4,7,9-tetrahydropyrano[2,3-E]isoindol-8(2H)-yl)pentanoic acid (2-20)

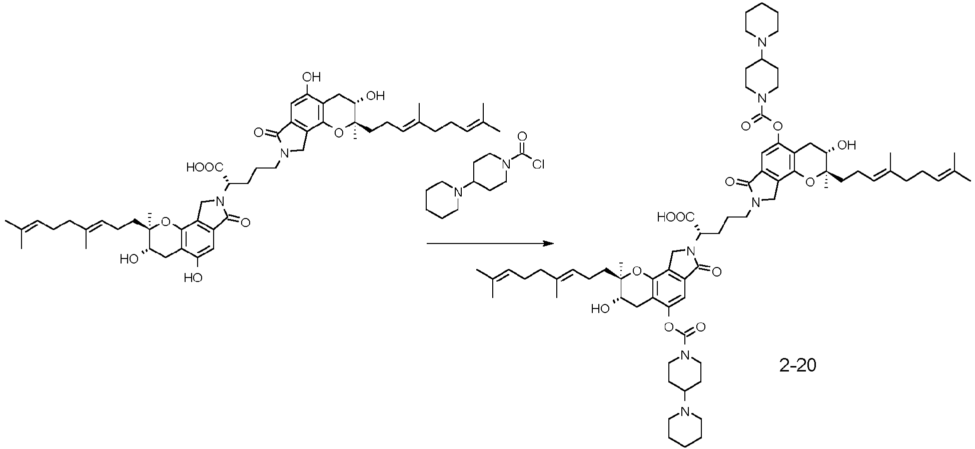

(S)-2,5-bis((2R,3S)-2-((E)-4,8-dimethylnona-3,7-dien-1-yl)-3,5-dihydroxy-2-methyl-7-oxo-3,4,7,9-tetrahydro pyrano[2,3-E]isoindol-8(2H)-yl)pentanoic acid (10 mL) was dissolved **in** dry THF (10 mL), and cooled to 0 °C with stiring. Then NaH (46.03 mg, 1.15 mmol, 60% content) was added, and the mixture was stirred at room temperature for 0.5 hours. 1-chloroformyl-4-piperidinopiperidine hydrochloride (122.51 mg, 0.460 mmol) was added, and the temperature was raised to 40 °C for 3 hours. The reaction was monitored by high performance liquid chromatography coupled with mass spectrometry. The reaction was quenched in ice water, neutralized by dropping 3N aqueous hydrochloric acid solution, concentrated under reduced pressure and purified by high performance liquid chromatography to obtain 33.15 mg of the title compound.

Column: SunFire Prep C18 OBD 19 mm × 150 mm × 5.0 µm.

Mobile phase A: acetonitrile; mobile phase B: water (containing 0.05% formic acid).

| Time [min] | Mobile phase A [%] | Mobile phase B [%] | Flow rate [mL/min] |
|---|---|---|---|
| 0.00 | 0 | 100 | 24 |
| 2.00 | 70 | 30 | 24 |
| 20.00 | 90 | 10 | 24 |

ESI-MS (m/z):1257.9 [M+H]⁺.

¹H NMR (400 MHz, DMSO) δ 8.21 (s, 1H), 6.93 (d, *J =* 11.2 Hz, 2H), 5.30 (s, 2H), 5.11 (t, *J =* 6.7 Hz, 2H), 5.04 (t, *J=* 6.8 Hz, 2H), 4.66 (dd, *J =* 10.9, 4.6 Hz, 1H), 4.41 (d, *J =* 17.4 Hz, 1H), 4.34 - 4.15 (m, 6H), 4.04 *(d, J=* 11.4 Hz, 2H), 3.80 - 3.74 (m, 3H), 3.51 (s, 3H), 3.02 (s, 3H), 2.91 - 2.74 (m, 5H), 2.55 (s, 10H), 2.10 (s, 5H), 2.05 - 1.95 (m, 6H), 1.95 - 1.86 (m, 5H), 1.80 (s, 5H), 1.53 (dd, J = 50.4, 31.9 Hz, 45H), 1.18 (d, *J =* 13.2 Hz, 6H).

### Example 12: preparation of (S)-2,5-bis((2R,3S)-2-((E)-4,8-dimethylnona-3,7-dien-1-yl)-3-hydroxy-2-methyl-7-oxo-5-((2S,3R,4S,5S,6R)-3, 4,5-trihydroxy-6-(hydroxymethyl)tetrahydro-2H-pyran-2-yl)oxy)-3,4,7,9-tetrahydropyrano[2,3-E]isoindol-8( 2H)-yl)pentanoic acid (2-22)

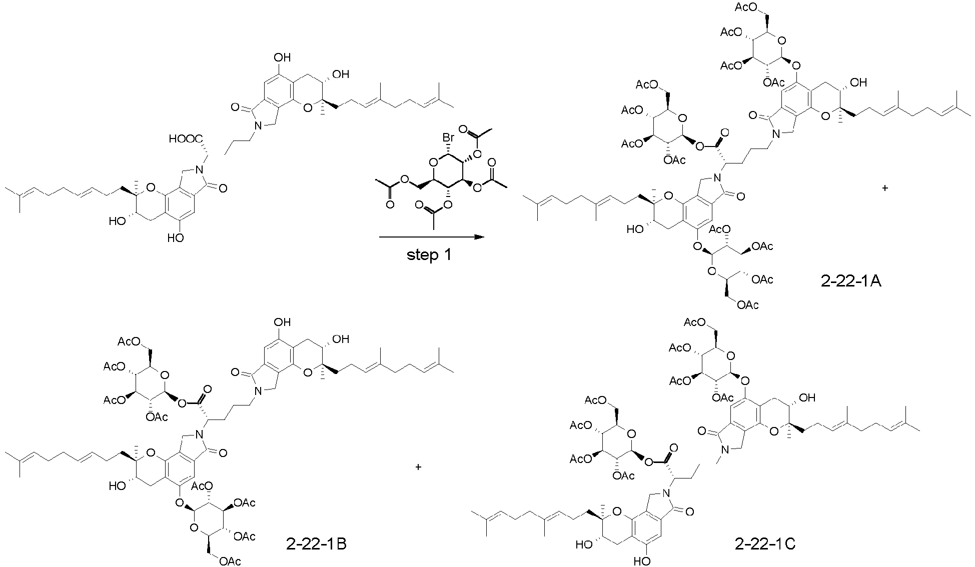

### Step 1:

(S)-2,5-bis((2R,3S)-2-((E)-4,8-dimethylnona-3,7-dien-1-yl)-3,5-dihydroxy-2-methyl-7-oxo-3,4,7,9-tetrahydro pyrano[2,3-E]isoindol-8(2H)-yl)pentanoic acid (200 mg, 0.230 mmol) and bromotetraacetylglucoside (378.51 mg, 0.920 mmol) were dissolved in acetonitrile (4 mL), then silver oxide (266.64 mg, 1.150 mmol) was added and the reaction was stirred at 25 °C for 16 hours. The reaction was monitored by high performance liquid chromatography coupled with mass spectrometry. The reaction solution was purified by HPLC to obtain 2-22-1A (8 mg), 2-22-1B (19 mg) and 2-22-1C (20 mg).

Column: SunFire Prep C18 OBD 19 mm × 150 mm × 5.0 µm.

Mobile phase A: acetonitrile; mobile phase B: water (containing 0.05% formic acid).

| Time [min] | Mobile phase A [%] | Mobile phase B [%] | Flow rate [mL/min] |
|---|---|---|---|
| 0.00 | 0 | 100 | 24 |
| 2.00 | 20 | 80 | 24 |
| 18.00 | 80 | 20 | 24 |

**2-22-1A:**
   ESI-MS (m/z): 1860.7[M+H]⁺.
**2-22-1B/2-22-1C:**
   ESI-MS (m/z): 1530.6[M+H]⁺.

### Step 2: synthesis of (S)-2,5-bis((2R,3S)-2-((E)-4,8-dimethylnona-3,7-dien-1-yl)-3-hydroxy-2-methyl-7-oxo-5-((2S,3R,4S,5S,6R)-3, 4,5-trihydroxy-6-(hydroxymethyl)tetrahydro-2H-pyran-2-yl)oxy)-3,4,7,9-tetrahydropyrano[2,3-E]isoindol-8( 2H)-yl)pentanoic acid (2-22)

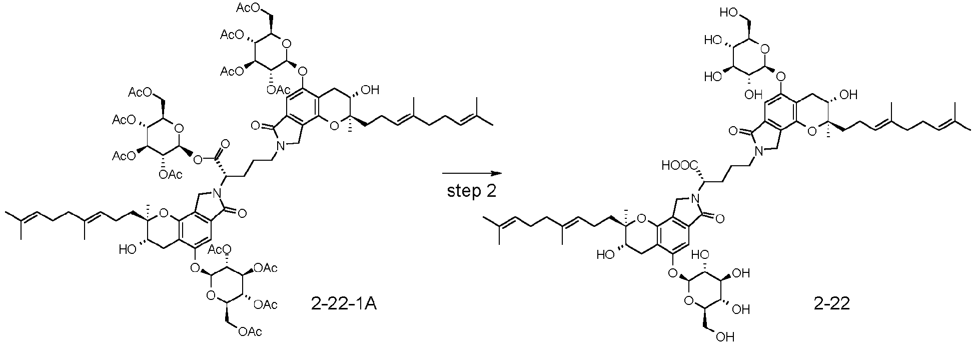

2-22-1A (8 mg, 0.004 mmol) was dissolved in tetrahydrofuran (1 mL) and (0.25 mL), lithium hydroxide (1.80 mg, 0.043 mmol) was added and the reaction was stirred for 1 hour. The reaction was monitored by high performance liquid chromatography coupled with mass spectrometry. The reaction solution was neutralized by dropping 3N aqueous hydrochloric acid solution, then evaporated under reduced pressure to remove the solvent, and purified by high performance liquid chromatography (HPLC) to obtain 2.12 mg of the title compound.

Mobile phase A: acetonitrile; mobile phase B: water (containing 0.05% formic acid).

| Time [min] | Mobile phase A [%] | Mobile phase B [%] | Flow rate [mL/min] |
|---|---|---|---|
| 0.00 | 0 | 100 | 24 |
| 2.00 | 30 | 70 | 24 |
| 18.00 | 90 | 10 | 24 |

ESI-MS (m/z):1193.6[M+H]⁺.

¹H NMR (400 MHz, DMSO) δ 6.89 (s, 1H), 6.86 (s, 1H), 5.28 (t, *J* = 5.1 Hz, 2H), 5.21 (d, *J =* 4.3 Hz, 2H), 5.16 - 5.09 (m, 4H), 5.03 (dd, *J =* 11.2, 6.1 Hz, 4H), 4.91 (d, *J =* 5.4 Hz, 2H), 4.54 (dd, *J =* 10.1, 5.7 Hz, 2H), 4.24 (s, 6H), 3.79 - 3.63 (m, 5H), 3.49 (d, *J =* 3.7 Hz, 5H), 3.24 (dd, *J =* 21.7, 7.8 Hz, 7H), 3.05 (d, *J =* 12.9 Hz, 3H), 2.53 (s, 1H), 2.46 - 2.44 (m, 1H), 2.11 (s, 5H), 1.95 (dt, *J =* 15.2, 7.2 Hz, 10H), 1.69 - 1.44 (m, 22H), 1.17 *(d, J=* 13.6 Hz, 6H).

### Example 13: Preparation of (S)-5-((2R,3S)-2-((E)-4,8-dimethylnona-3,7-dien-1-yl)-3,5-dihydroxy-2-methyl-7-oxo-3,4,7,9-tetrahydropyran o[2,3-E]isoindol-8(2H)-yl)-2-(2R,3S)-3-((E)-4,8D-dimethyldeca-3,7-dien-1-yl)-3-hydroxy-2-methyl-7-oxo-5-((2 S,3S,4S,6R)-3,4,5-trihydroxy-6-(hydroxymethyl)tetrahydro-2H-pyran-2-yl)oxy)-3,4,7,9-tetrahydropyrano[2, 3-E]isoindol-8(2H)-yl)pentanoic acid (2-22-A)

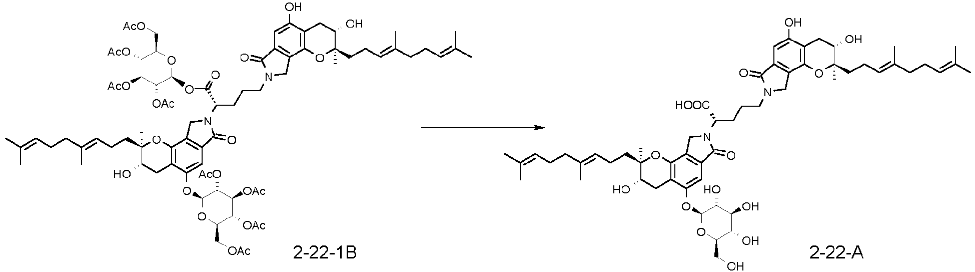

2-22-1B (19 mg, 0.012 mmol) was dissolved in tetrahydrofuran (1 mL) and (0.25 mL), lithium hydroxide (2.61 mg, 0.062 mmol) was added, and the reaction was stirred for 1 hour. The reaction was monitored by high performance liquid chromatography coupled with mass spectrometry. The reaction solution was neutralized by dropping 3N aqueous hydrochloric acid solution, then evaporated under reduced pressure to remove the solvent, and purified by high performance liquid chromatography to obtain 3.03 mg of the title compound.

Mobile phase A: acetonitrile; mobile phase B: water (containing 0.05% formic acid).

| Time [min] | Mobile phase A [%] | Mobile phase B [%] | Flow rate [mL/min] |
|---|---|---|---|
| 0.00 | 0 | 100 | 24 |
| 2.00 | 40 | 60 | 24 |
| 18.00 | 90 | 10 | 24 |

ESI-MS (m/z): 1031.6 [M+H]⁺.

¹H NMR (400 MHz, DMSO) δ 13.05 - 12.80 (m, 1H), 9.75 (s, 1H), 6.89 (s, 1H), 6.62 (s, 1H), 5.29 (d, *J* = 4.8 Hz, 1H), 5.21 (d, *J =* 5.2 Hz, 1H), 5.16 - 4.99 (m, 6H), 4.92 (d, *J =* 7.2 Hz, 1H), 4.71 (s, 1H), 4.54 (t, *J =* 5.8 Hz, 1H), 4.34 - 4.13 (m, 4H), 3.73 (d, *J =* 5.4 Hz, 3H), 3.48 (s, 3H), 3.27 (s, 4H), 3.05 (dd, *J =* 17.8, 5.2 Hz, 1H), 2.81 (dd, *J =* 17.7, 5.2 Hz, 1H), 2.59 - 2.52 (m, 1H), 2.43 (d, *J =* 7.4 Hz, 1H), 2.10 (s, 4H), 1.95 (dt, *J =* 14.8, 7.1 Hz, 10H), 1.55 (dd, *J* = 24.4, 14.4 Hz, 21H), 1.24 - 1.07 (m, 6H).

### Example 14: Preparation of (S)-2-(2R,3S)-2-(E)-4,8-dimethylnona-3,7-dien-1-yl)-3,5-dihydroxy-2-methyl-7-oxo-3,4,7,9-tetrahydropyrano [2,3-E]isoindol-8(2H)-yl)-5-((2R,3S)-2-((E)-4,8-dimethylnona-4,7-dien-1-yl)-3-hydroxy-2-methyl-7-oxo-5-(((2 S,3R,4S,SS,6R)-3,4,5-trihydroxy-6-(hydroxymethyl)tetrahydro-2H-pyran-2-yl)oxy)-3,4,7,9-tetrahydropyrano [2,3-E]isoindol-8(2H)-yl)pentanoic acid (2-22-B)

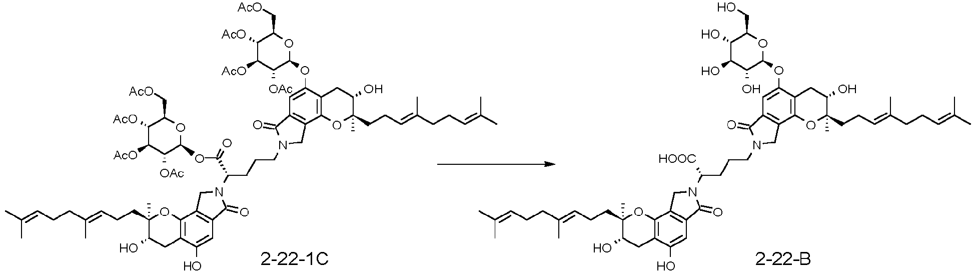

2-22-1C (20 mg, 0.013 mmol) was dissolved in tetrahydrofuran (1 mL) and (0.25 mL), lithium hydroxide (2.74 mg, 0.065 mmol) was added and the reaction was stirred for 1 hour. The reaction was monitored by high performance liquid chromatography coupled with mass spectrometry. The reaction solution was neutralized by dropping 3N aqueous hydrochloric acid solution, then evaporated under reduced pressure to remove the solvent, and purified by high performance liquid chromatography to obtain 3.73 mg of the title compound.

Mobile phase A: acetonitrile; mobile phase B: water (containing 0.05% formic acid.

| Time [min] | Mobile phase A [%] | Mobile phase B [%] | Flow rate [mL/min] |
|---|---|---|---|
| 0.00 | 0 | 100 | 24 |
| 2.00 | 40 | 60 | 24 |
| 18.00 | 90 | 10 | 24 |

ESI-MS (m/z):1031.6 [M+H]⁺.

¹H NMR (400 MHz, DMSO) δ 9.77 (s, 1H), 6.85 (s, 1H), 6.64 (s, 1H), 5.27 (d, *J =* 4.9 Hz, 1H), 5.21 (d, J = 5.2 Hz, 1H), 5.14 (dd, *J =* 10.9, 6.0 Hz, 4H), 5.07 - 5.00 (m, 3H), 4.91 (d, *J =* 7.3 Hz, 1H), 4.55 (t, *J =* 5.8 Hz, 1H), 4.20 (d, *J =* 20.4 Hz, 6H), 3.77 - 3.62 (m, 4H), 3.57 - 3.42 (m, 4H), 3.30 - 3.17 (m, 4H), 3.10 - 2.99 (m, 2H), 2.82 (d, *J* = 12.3 Hz, 2H), 2.47 - 2.40 (m, 1H), 2.11 (s, 5H), 1.95 (dt, *J =* 14.8, 6.8 Hz, 9H), 1.57 (d, *J =* 32.6 Hz, 22H), 1.16 *(d, J=* 11.5 Hz, 6H).

### Example 15: preparation of (S)-2,5-bis((2R,3S)-2-((E)-4,8-dimethylnona-3,7-dien-1-yl)-5-hydroxy-2-methyl-3-((4-methylpiperazin-1-carb onyl)oxy)-7-oxo-3,4,7,9-tetrahydropyrano[2,3-E]isoindol-8(2H)-yl)pentanoic acid (2-23); (S)-5-((2R,3S)-2-((E)-4,8-dimethylnona-3,7-dien-1-yl)-3,5-dihydroxy-2-methyl-7-oxo-3,4,7,9-tetrahydropyran o[2,3-E]isoindol-8(2H)-yl)-2-(2R,3S)-3-((E)-4,7-dimethyldec-3-yl)-5-hydroxy-2-methyl-3-(4-methylpiperazin-1-carbonyl)oxy)-7-oxo-3,4,7,9-tetrahydropyrano[2,3-E]isoindol-8(2H)-yl)pentanoic acid (2-23-A); (S)-2-(2R,3S)-2-(E)-4,8-dimethylnona-3,7-dien-1-yl)-3,5-dihydroxy-2-methyl-7-oxo-3,4,7,9-tetrahydropyrano [2,3-E]isoindol-8(2H)-yl)-5-((2R,3S)-2-((E)-4.8-dimethylnona-4,7-dien-1-yl)-5-hydroxy-2-methyl-3-((4-methyl piperazin-1-carbonyl)oxy)-7-oxo-3,4,7,9-tetrahydropyrano[2,3-E]isoindol-8(2H)-yl)pentanoic acid (2-23-B)

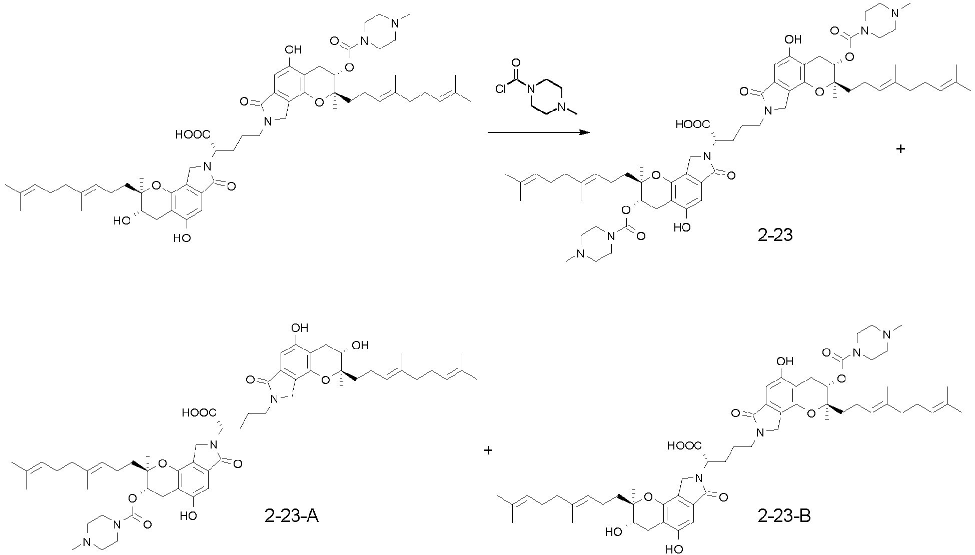

(S)-2,5-bis((2R,3S)-2-((E)-4,8-dimethylnona-3,7-dien-1-yl)-3,5-dihydroxy-2-methyl-7-oxo-3,4,7,9-tetrahydro pyrano[2,3-E]isoindol-8(2H)-yl)pentanoic acid (100 mg, 0.115 mmol) was dissolved in dry THF (10 mL), and cooled to 0 °C with stiring. NaH (46.03 mg, 1.15 mmol, 60% content) was added, and stirred at room temperature for 0.5 hours. 1-chloroformyl-4-methylpiperazine (91.16 mg, 0.460 mmol) was added, and the temperature was raised to 40 °C for 3 hours. The reaction was monitored by high performance liquid chromatography coupled with mass spectrometry. The reaction was quenched by pouring into ice water, neutralized by dropping 3N aqueous hydrochloric acid solution, concentrated under reduced pressure, and purified by high performance liquid chromatography to obtain disubstituted compound 2-23 (8.07 mg) and monosubstituted compounds 2-23-A (3.32 mg) and 2-23-B (14.16 mg).

Column: SunFire Prep C18 OBD 19 mm × 150 mm × 5.0 µm.

Mobile phase A: acetonitrile; mobile phase B: water (containing 0.05% formic acid).

| Time [min] | Mobile phase A [%] | Mobile phase B [%] | Flow rate [mL/min] |
|---|---|---|---|
| 0.00 | 0 | 100 | 24 |
| 2.00 | 70 | 30 | 24 |
| 20.00 | 90 | 10 | 24 |

**2-23:**
   ESI-MS (m/z): 1122.7[M+H]⁺.
   ¹H NMR (400 MHz, DMSO) δ 9.94 (d*, J=* 19.3 Hz, 2H), 6.67 (d, *J* = 13.5 Hz, 2H), 5.04 (dd, *J =* 15.1, 7.7 Hz, 5H), 4.95 - 4.87 (m, 2H), 4.70 (dd, *J =* 10.2, 5.1 Hz, 1H), 4.30 - 4.15 (m, 4H), 3.50 (d, *J =* 7.0 Hz, 2H), 2.99 - 2.90 (m, 2H), 2.64 (dd, *J =* 15.6, 9.7 Hz, 2H), 2.18 (d, *J =* 28.9 Hz, 15H), 2.10 - 1.85 (m, 15H), 1.65 - 1.43 (m, 25H), 1.27 (d, *J =* 8.4 Hz, 6H).
**2-23-A:**
   ESI-MS (m/z): 996.6[M+H]⁺.
   ¹H NMR (400 MHz, DMSO) δ 9.93 (s, 1H), 9.76 (s, 1H), 6.65 (d, *J =* 22.3 Hz, 2H), 5.20 - 4.97 (m, 5H), 4.89 (d, *J* = 4.9 Hz, 1H), 4.56 (s, 2H), 4.37 (s, 3H), 4.19 *(*d, *J =* 16.6 Hz, 4H), 3.73 (s, 3H), 3.47 (s, 2H), 2.96 *(d, J=* 13.4 Hz, 2H), 2.86 - 2.76 (m, 2H), 2.65 (d, *J =* 13.0 Hz, 1H), 2.44 (s, 1H), 2.33 (s, 1H), 2.28 - 1.85 (m, 18H), 1.76 (s, 2H), 1.54 (dd, *J =* 26.6, 19.5 Hz, 18H), 1.21 (d, *J =* 50.8 Hz, 6H).
**2-23-B:**
   ESI-MS (m/z): 996.6[M+H]⁺.
   ¹H NMR (400 MHz, DMSO) δ 9.90 (s, 1H), 9.80 (s, 1H), 6.65 (s, 2H), 5.12 (dd, *J =* 14.5, 6.7 Hz, 2H), 5.08 - 4.97 (m, 3H), 4.89 (t, *J* = 5.3 Hz, 1H), 4.72 (dd, *J* = 10.2, 5.3 Hz, 1H), 4.21 (d, *J =* 7.9 Hz, 4H), 3.74 (s, 1H), 3.48 (s, 2H), 2.97 - 2.89 (m, 1H), 2.86 - 2.78 (m, 1H), 2.66 - 2.58 (m, 1H), 2.46 (s, 1H), 2.23 (s, 3H), 2.15 (s, 3H), 2.12 - 1.81 (m, 14H), 1.64 - 1.44 (m, 22H), 1.20 (t, *J =* 20.8 Hz, 6H).

### Example 16: Preparation of (S)-5-((2R,3S)-2-((E)-4,8-dimethylnona-3,7-dien-1-yl)-3,5-dihydroxy-2-methyl-7-oxo-3,4,7,9-tetrahydropyran o[2,3-E]isoindol-8(2H)-yl)-2-((2R,3S)-2-((E)-4,8-dimethylnona-3,7-dien-1-yl)-5-hydroxy-2-methyl-7-oxo-3-(su lfamoyloxy)-3,4,7,9-tetrahydropyrano[2,3-E]isoindol-8(2H)-yl)pentanoic acid (2-28-A); (S)-2-((2R,3S)-2-(((E)-4,8-dimethylnona-3,7-dien-1-yl)-3,5-dihydroxy-2-methyl-7-oxo-3,4,7,9-tetrahydropyra no[2,3-E]isoindol-8(2H)-yl)-5-((2R,3S)-2-((E)-4,8-dimethylnona-3,7-dien-1-yl)-5-hydroxy-2-methyl-7-oxo-3-(s ulfamoyloxy)-3,4,7,9-tetrahydropyrano[2,3-E]isoindol-8(2H)-yl)pentanoic acid (2-28-B)

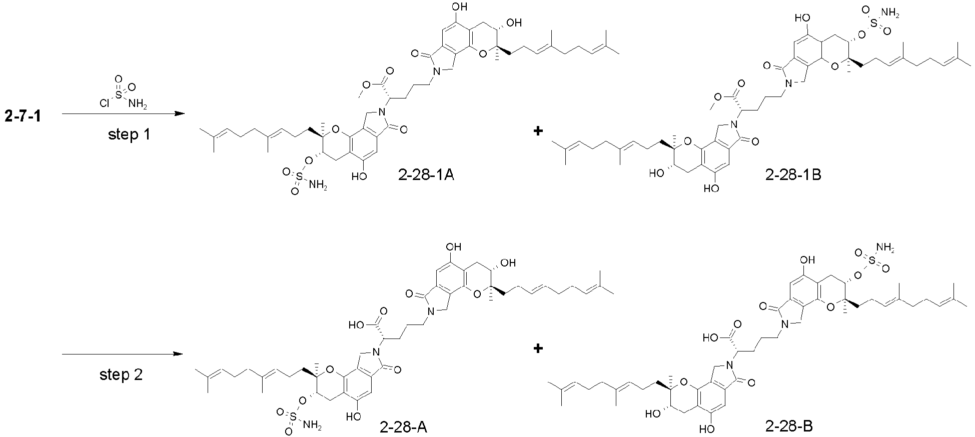

### Step 1: Preparation of a mixture of methyl (S)-5-((2R,3S)-2-((E)-4,8-dimethylnona-3,7-dien-1-yl)-3,5-dihydroxy-2-methyl-7-oxo-3,4,7,9-tetrahydropyran o[2,3-E]isoindol-8(2H)-yl)-2-((2R,3S)-2-((E)-4,8-dimethylnona-3,7-dien-1-yl)-5-hydroxy-2-methyl-7-oxo-3-(su lfamoyloxy)-3,4,7,9-tetrahydropyrano[2,3-E]isoindol-8(2H)-yl)pentanoate (2-28-1A) and (S)-2-((2R,3S)-2-((E)-4,8-dimethylnona-3,7-dien-1-yl)-3,5-dihydroxy-2-methyl-7-oxo-3,4,7,9-tetrahydropyrano[2,3-E]isoind ol-8(2H)-yl)-5-((2R,3S)-2-((E)-4,8-dimethylnona-3,7-dien-1-yl)-5-hydroxy-2-methyl-7-oxo-3-(sulfamoyloxy)-3 ,4,7,9-tetrahydropyrano[2,3-E]isoindol-8(2H)-yl)pentanoate (2-28-1B)

Compound 2-7-1 (200.0 mg, 0.23 mmol) and sulfamoyl chloride (157.0 mg, 1.36 mmol) were added to DMA (6 mL). NaH (136.0 mg, 3.40 mmol, assay 60%) was added, the temperature was raised to 30 °C, and the mixture was allowed to react for 2 hours. The reaction solution was cooled to room temperature, poured into a saturated aqueous ammonium chloride solution, and extracted with ethyl acetate. The organic phase was concentrated to give a crude product of the title compound (218 mg), which was used directly for the next reaction.

### Step 2: Preparation of a mixture of (S)-5-((2R,3S)-2-((E)-4,8-dimethylnona-3,7-dien-1-yl)-3,5-dihydroxy-2-methyl-7-oxo-3,4,7,9-tetrahydropyran o[2,3-E]isoindol-8(2H)-yl)-2-((2R,3S)-2-((E)-4,8-dimethylnona-3,7-dien-1-yl)-5-hydroxy-2-methyl-7-oxo-3-(su lfamoyloxy)-3,4,7,9-tetrahydropyrano[2,3-E]isoindol-8(2H)-yl)pentanoic acid (2-28-A) and (S)-2-((2R,3S)-2-((E)-4,8-dimethylnona-3,7-dien-1-yl)-3,5-dihydroxy-2-methyl-7-oxo-3,4,7,9-tetrahydropyran o[2,3-E]isoindol-8(2H)-yl)-5-((2R,3S)-2-((E)-4,8-dimethylnona-3,7-dien-1-yl)-5-hydroxy-2-methyl-7-oxo-3-(su lfamoyloxy)-3,4,7,9-tetrahydropyrano[2,3-E]isoindol-8(2H)-yl)pentanoic acid (2-28-B)

A mixture of 2-28-1A and 2-28-1B (250.00 mg, 0.26 mmol) was added to tetrahydrofuran (5 mL) and water (5 ml), lithium hydroxide (93.34 mg, 3.90 mmol) was added and the mixture was allowed to react at room temperature for 1 hour. The reaction solution was adjusted to pH 4 with an aqueous citric acid solution, and extracted with ethyl acetate (20 ml). The organic phase was concentrated to give the crude product, which was purified by preparative high performance liquid chromatography to obtain the title compound (24.0 mg).

Column: Waters SunFire Prep C18 OBD (5 µm * 19 mm * 150 mm).

Mobile phase A: acetonitrile; mobile phase B: water (0.05% formic acid in water).

Retention time: 5.3-6.2 min.

| Time [min] | Mobile phase A [%] | Mobile phase B [%] | Flow rate [mL/min] |
|---|---|---|---|
| 0.00 | 30 | 70 | 28 |
| 2.00 | 30 | 70 | 28 |
| 18.00 | 90 | 10 | 28 |

### Structural characterization data are presented below:

MS m/z (ESI): 948.3 [M+H]⁺.

¹H NMR (400 MHz, DMSO-d₆) δ 10.04 (s, 1H), 10.00 (s, 1H), 9.80 (s, 1H), 9.77 (s, 1H), 7.66 (br, 4H), 6.71 (s, 1H), 6.68(s, 1H), 6.65 (s, 1H), 6.62 (s, 1H), 5.15 - 5.11 (m, 4H), 5.05 - 4.97 (m, 5H), 4.74 - 4.63 (m, 4H), 4.32 - 4.14 (m, 8H), 3.76 - 3.71 (m, 2H), 3.50 - 3.44 (m, 4H), 3.09 - 2.90 (m, 4H), 2.85 - 2.78 (m, 2H), 2.47 - 2.43 (m, 2H), 2.12 - 2.03 (m, 8H), 2.02 - 1.79 (m, 18H), 1.64 - 1.45 (m, 42H), 1.38 (s, 3H), 1.35 (s, 3H), 1.18 (s, 3H), 1.15 (s, 3H).

### Example 17: preparation of (S)-2,5-bis((2R,3S)-2-((E)-4,8-dimethylnona-3,7-dien-1-yl)-3,5-dihydroxy-2-methyl-7-oxo-3,4,7,9-tetrahydrop yrano[2,3-E]isoind ol-8(2H)-yl)-N-methylpentanamide (2-29)

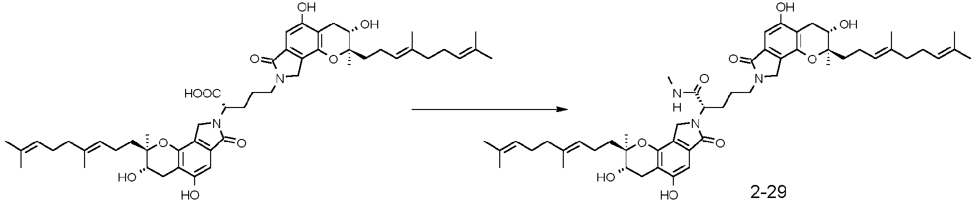

(S)-5-((2R,3S)-2-((E)-4,8-dimethylnona-3,7-dien-1-yl)-3,5-dihydroxy-2-methyl-7-oxo-3,4,7,9-tetrahydropyran o[2,3-E]isoindol-8(2H)-yl)-2-((25,3S)-2-((E)-4,8-dimethylnona-3,7-dien-1-yl)-3,5-dihydroxy-2-methyl-7,9-tetrahy dropyrano[2,3-E]isoindol-8(2H)-pentanoic acid (100 mg, 115.06 µmol) was added to DMF (3 mL), methylamine hydrochloride (15.54 mg, 230.13 µmol), HATU (131.25 mg, 345.19 µmo) and DIPEA (74.35 mg, 575.31 µmol) were added, and the mixture was allowed to react at 25 °C for 12 hours. The reaction solution was purified directly with preparative high performance liquid chromatography to obtain the title compound 2-29 (56.0 mg). )

Column: Waters SunFire Prep C18 OBD (5 µm * 19 mm * 150 mm).

Mobile phase A: acetonitrile; mobile phase B: water (0.05% formic acid in water).

Retention time: 8.5-9.5 min.

| Time [min] | Mobile phase A [%] | Mobile phase B [%] | Flow rate [mL/min] |
|---|---|---|---|
| 0.00 | 50 | 50 | 28 |
| 10.00 | 90 | 10 | 28 |
| 20.00 | 90 | 10 | 28 |

The structural characterization data are presented below:
MS m/z (ESI): 882.5 [M+H]⁺.

¹H NMR (400 MHz, DMSO-d₆) δ 9.76 (s, 1H), 9.74 (s, 1H), 8.02 - 7.97 (m, 1H), 6.64 (s, 1H), 6.62 (s, 1H), 5.17 - 5.08 (m, 4H), 5.06 - 5.02 (m, 2H), 4.70 - 4.65 (m, 1H), 4.38 - 4.34 (m, 1H), 4.19 - 4.15 (m, 3H), 3.75 - 3.70 (m, 2H), 3.52 - 3.41 (m, 2H), 2.85 - 2.78 (m, 2H), 2.54 (d, *J* = 4.4 Hz, 2H), 2.46 - 2.42 (m, 1H), 2.13 - 2.05 (m, 4H), 2.03 - 1.95 (m, 4H), 1.92 - 1.89 (m, 4H), 1.83 - 1.72 (m, 2H), 1.60 - 1.55 (m, 9H), 1.55 - 1.49 (m, 11H), 1.48 - 1.41 (m, 2H), 1.17 (s, 3H), 1.15 (s, 3H).

### Example 18: preparation of (S)-2,5-bis((2R,3S)-2-((E)-4,8-dimethylnona-3,7-dien-1-yl)-3,5-dihydroxy-2-methyl-7-oxo-3,4,7,9-tetrahydrop yrano[2,3-e]isoindol-8(2H)-yl)-N-(2-methoxyethyl)pentanamide (2-30)

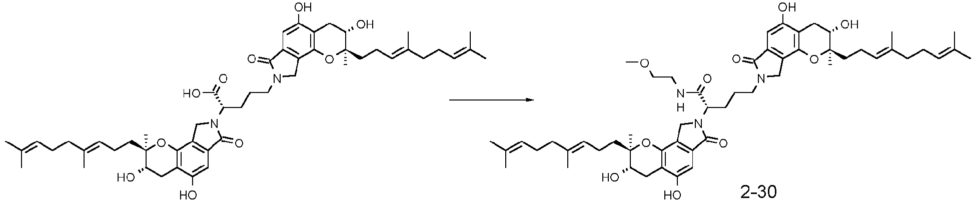

(S)-5-((2R,3S)-2-((E)-4,8-dimethylnona-3,7-dien-1-yl)-3,5-dihydroxy-2-methyl-7-oxo-3,4,7,9-tetrahydropyran o[2,3-E]isoindol-8(2H)-yl)-2-((2S,3S)-2-(((E)-4,8-dimethylnona-3,7-dien-1-yl)-3,5-dihydroxy-2-methyl-7,9-tetrahy dropyrano[2,3-E]isoindol-8(2H)-pentanoic acid (52.63 mg, 57.53 µmol) was dissolved in THF (30 mL), and the solution was cooled to 0 °C. NaH (23.01 mg, 575.31 µmol) was added, the temperature was raised to 25 °C, and the mixture was stirred for 0.5 hours. N-(2-methoxyethyl)carbamoyl chloride (41.65 mg, 287.66 µmol) was added dropwise to the reaction solution, the temperature was raised to 40 °C, and the mixture was allowed to react for 3 h. The reaction solution was concentrated directly, and purified by preparative high performance liquid chromatography to obtain the title compound (10.0 mg).

Column: Waters SunFire Prep C18 OBD (5 µm * 19 mm * 150 mm).

Mobile phase A; acetonitrile; mobile phase B: water (containing 0.05% formic acid).

Retention time: 8.5-9.5 min.

| Time [min] | Mobile phase A [%] | Mobile phase B [%] | Flow rate [mL/min] |
|---|---|---|---|
| 0.00 | 50 | 50 | 30 |
| 2.00 | 50 | 50 | 30 |
| 18.00 | 95 | 5 | 30 |

The structural characterization data are presented below:
MS m/z (ESI): 926.5 [M+H+H₂O]⁺.

¹H NMR (400 MHz, DMSO) δ 9.75 (d, *J =* 6.5 Hz, 1H), 8.15 (t, *J=* 5.5 Hz, 1H), 6.63 (d, *J* = 8.7 Hz, 1H), 5.20 - 5.08 (m, 2H), 5.04 (s, 1H), 4.74 (dd, *J* = 9.2, 6.1 Hz, 1H), 4.41 (d, *J* = 17.0 Hz, 1H), 4.17 (t, *J* = 8.5 Hz, 1H), 3.72 (d, *J* = 10.6 Hz, 1H), 3.47 (s, 1H), 3.38 - 3.25 (m, 4H), 3.22 - 3.13 (m, 2H), 2.82 (dd, *J =* 12.3, 3.6 Hz, 1H), 2.49 - 2.39 (m, 1H), 2.10 (d, *J =* 6.9 Hz, 1H), 2.00 (dd, *J =* 14.3, 6.9 Hz, 1H), 1.95 - 1.86 (m, 2H), 1.77 (dd, *J =* 14.9, 8.7 Hz, 1H), 1.59 *(d, J=* 16.1 Hz, 4H), 1.53 (s, 5H), 1.36 (s, 1H), 1.16 *(d, J=* 12.0 Hz, 2H).

### Example 19: preparation of 2-morpholinylethyl (S)-2,5-bis((2R,3S)-2-((E)-4,8-dimethylnona-3,7-dien-1-yl)-3,5-dihydroxy-2-methyl-7-oxo-3,4,7,9-tetrahydrop yrano[2,3-E]isoindol-8(2H)-yl) pentanoate (2-31)

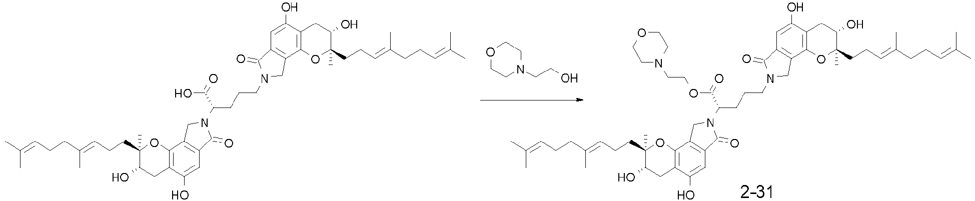

(S)-2,5-bis((2R,3S)-2-((E)-4,8-dimethylnona-3,7-dien-1-yl)-3,5-dihydroxy-2-methyl-7-oxo-3,4,7,9-tetrahydro pyrano[2,3-E]isoindol-8(2H)-yl)pentanoic acid (50 mg, 0.058 mmol) was dissolved in pyridine (1 mL), then 2-morpholinylethanol (15.09 mg, 0.115 mmol) and DCC (17.81 mg, 0.086 mmol) were added and the reaction was stirred at 25 °C for 12 hours. The reaction was monitored by high performance liquid chromatography coupled with mass spectrometry. The reaction solution was purified by high performance liquid chromatography to obtain 1.18 mg of the title compound.

Column: SunFire Prep C18 OBD 19 mm × 150 mm × 5.0 µm.

Mobile phase A: acetonitrile; mobile phase B: water (containing 0.05% formic acid).

| Time [min] | Mobile phase A [%] | Mobile phase B [%] | Flow rate [mL/min] |
|---|---|---|---|
| 0.00 | 0 | 100 | 28 |
| 12.00 | 60 | 40 | 28 |
| 18.00 | 90 | 10 | 28 |

ESI-MS (m/z):983.7[M+H]⁺.

¹H NMR (400 MHz, DMSO) δ 9.81 (d, *J =* 30.4 Hz, 2H), 8.44 (s, 1H), 6.64 (d, *J =* 14.0 Hz, 2H), 5.58 (d, *J=* 8.0 Hz, 2H), 5.08 (d, *J* = 28.1 Hz, 6H), 4.83 (d, *J* = 5.7 Hz, 1H), 4.19 (d, *J =* 14.0 Hz, 6H), 3.72 (s, 2H), 3.48 (s, 2H), 2.82 (dd, *J* = 12.0, 5.7 Hz, 2H), 2.43 (d, *J =* 8.2 Hz, 4H), 2.22 (d, *J =* 3.9 Hz, 4H), 2.09 (s, 3H), 2.03 - 1.97 (m, 3H), 1.94 - 1.85 (m, 4H), 1.71 (d, *J =* 12.8 Hz, 3H), 1.56 (dd, *J* = 24.2, 9.9 Hz, 21H), 1.28 - 1.02 (m, 12H).

### Example 20: preparation of 35-hydroxy-3,6,9,12,15,18,21,24,27,30,33-undecaoxapentatriacontyl (S)-2,5-bis((2R,3S)-2-((E)-4,8-dimethylnona-3,7-dien-1-yl)-3,5-dihydroxy-2-methyl-7-oxo-3,4,7,9-tetrahydrop yrano[2,3-E]isoindol-8(2H)-yl)pentanoate (2-32)

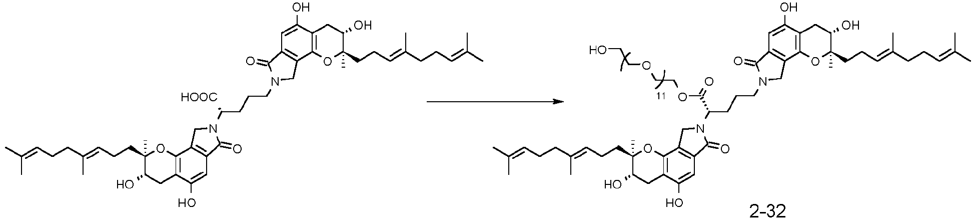

(S)-5-((2R,3S)-2-((E)-4,8-dimethylnona-3,7-dien-1-yl)-3,5-dihydroxy-2-methyl-7-oxo-3,4,7,9-tetrahydropyran o[2,3-E]isoindol-8(2H)-yl)-2-((2S,3S)-2-((E)-4,8-dimethylnona-3,7-dien-1-yl)-3,5-dihydroxy-2-methyl-7,9-tetrahy dropyrano[2,3-E]isoindol-8(2H)-pentanoic acid (120.0 mg, 0.14 mmol) was dissolved in DMF (2 mL). Dodecylglycol (754.8 mg, 1.38 mmol), HATU (105.0 mg, 0.27 mmol), and DIPEA(89.2 mg, 0.69 mmol) were added and the mixture was allowed to react at 25 °C for 12 hours. The reaction solution was purified directly by preparative high performance liquid chromatography to give the title compound 2-32 (48.0 mg).

Column: Waters SunFire Prep C18 OBD (5 µm * 19 mm * 150 mm).

Mobile phase A: acetonitrile; mobile phase B: water (containing 0.05% formic acid).

Retention time: 8.5-9.5 min.

| Time [min] | Mobile phase A [%] | Mobile phase B [%] | Flow rate [mL/min] |
|---|---|---|---|
| 0.00 | 50 | 50 | 28 |
| 12.00 | 90 | 10 | 28 |
| 18.00 | 90 | 10 | 28 |

The structural characterization data are presented below:
MS m/z (ESI): 1414.7 [M +H+ H₂O]⁺.

¹H NMR (400 MHz, DMSO-d₆) δ 9.82 (s, 1H), 9.75 (s, 1H), 6.66 (s, 1H), 6.62 (s, 1H), 5.18 - 5.15 (m, 2H), 5.13 -5.09 (m, 2H), 5.06 - 5.02 (m, 2H), 4.88 - 4.82 (m, 2H), 4.58 (t, *J =* 5.4 Hz, 2H), 4.20 - 4.14 (m, 7H), 3.77 - 3.70 (m, 3H), 3.56 - 3.52 (m, 2H), 3.50 - 3.48 (m, 30H), 3.47 - 3.38 (m, 10H), 3.37 - 3.35 (m, 2H), 2.85 - 2.78 (m, 3H), 2.47 - 2.42 (m, 2H), 2.13 - 2.07 (m, 4H), 2.04 - 1.97 (m, 4H), 1.94 - 1.86 (m, 6H), 1.63 - 1.54 (m, 10H), 1.54 - 1.50 (m, 12H), 1.17 (s, 3H), 1.14 (s, 3H).

### Example 21: preparation of 2-(S)-2,5-bis((2R,3S)-2-((E)-4,8-dimethylnona-3,7-dien-1-yl)-3,5-dihydroxy-2-methyl-7-oxo-3,4,7,9-tetrahydr opyrano[2,3-E]isoindol-8(2H)-yl)pentanoyl)oxy)-N,N,N-trimethylethane-1-amine hydrochloride (2-33)

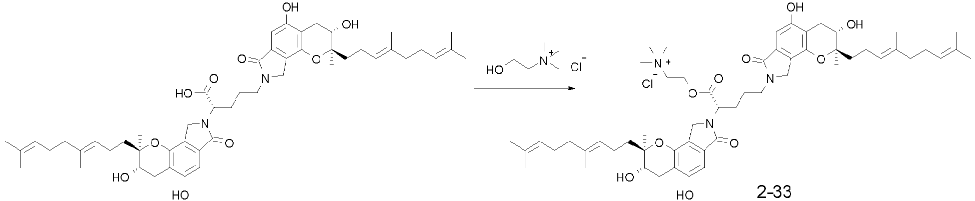

(S)-2,5-bis((2R,3S)-2-((E)-4,8-dimethylnona-3,7-dien-1-yl)-3,5-dihydroxy-2-methyl-7-oxo-3,4,7,9-tetrahydro pyrano[2,3-E]isoindol-8(2H)-yl)pentanoic acid (300 mg, 0.345 mmol) was dissolved in DMF (3 mL), then choline hydrochloride (97.09 mg, 0.69 mmol) and DCC (106.83 mg, 0.518 mmol) were added, and the reaction was stirred at 25 °C for 12 hours. The reaction was monitored by high performance liquid chromatography coupled with mass spectrometry. The reaction solution was acidified by dropping hydrochloric acid, followed by purification via high performance liquid chromatography to obtain 8.70 mg of the title compound.

Column: SunFire Prep C18 OBD 19 mm × 150 mm × 5.0 µm.

Mobile phase A: acetonitrile; mobile phase B: water (containing 0.05% formic acid).

| Time [min] | Mobile phase A [%] | Mobile phase B [%] | Flow rate [mL/min] |
|---|---|---|---|
| 0.00 | 0 | 100 | 28 |
| 12.00 | 60 | 40 | 28 |
| 18.00 | 90 | 10 | 28 |

ESI-MS (m/z): 954.7[M+H]⁺.

'H NMR (400 MHz, DMSO) δ 6.55 (d, *J =* 3.9 Hz, 2H), 5.09 (d, *J =* 30.2 Hz, 4H), 4.89 (d, *J =* 8.0 Hz, 1H), 4.43 (s, 2H), 4.14 (d, *J* = 18.0 Hz, 3H), 3.72 (d, *J =* 4.6 Hz, 2H), 3.57 (s, 3H), 3.36 (s, 6H), 3.04 (d, *J =* 47.3 Hz, 9H), 2.83 (d, *J =* 16.0 Hz, 2H), 2.45 (s, 2H), 2.21 - 1.81 (m, 12H), 1.76 - 1.38 (m, 20H), 1.14 (d, *J =* 7.7 Hz, 6H).

### Example 22:

### (S)-2,5-bis((2R,3S)-2-((E)-4,8-dimethylnona-3,7-dien-1-yl)-5-((1-oxothiomorpholino-4-carbonyl)oxy)-3-hydro xy-2-methyl-7-oxo-3,4,7,9-tetrahydropyrano[2,3-E]isoindol-8(2H)-yl)pentanoic acid (2-6); (S)-5-((2R,3S)-2-((E)-4,8-dimethylnona-3,7-dien-1-yl)-3,5-dihydroxy-2-methyl-7-oxo-3,4,7,9-tetrahydropyran o[2,3-E]isoindol-8(2H)-yl)-2-(2R,3S)-3-((E)-4,6-dimethylhept-3,7-dien-1-yl)-5-((1-oxothiomorpholino-4-carbo nyl)oxy)-3-hydroxy-2-methyl-7-oxo-3,4,7,9-tetrahydropyrano[2,3-E]isoindol-8(2H)-yl)pentanoic acid (2-6-A) and

### (S)-2-((2R,3S)-2-((E)-4,8-dimethylnona-3,7-dien-1-yl)-3,5-dihydroxy-2-methyl-7-oxo-3,4,7,9-tetrahydropyran o[2,3-E]isoindol-8(2H)-yl)-5-(2R,3S)-2-((E)-4,8-dimethylnona-2,7-dien-1-yl)-5-(1-oxothiomorpholino-4-carbo nyl)oxy)-3-hydroxy-2-methyl-7-oxo-3,4,7,9-tetrahydropyrano[2,3-E]isoindol-8(2H)-yl)pentanoic acid (2-6-B)

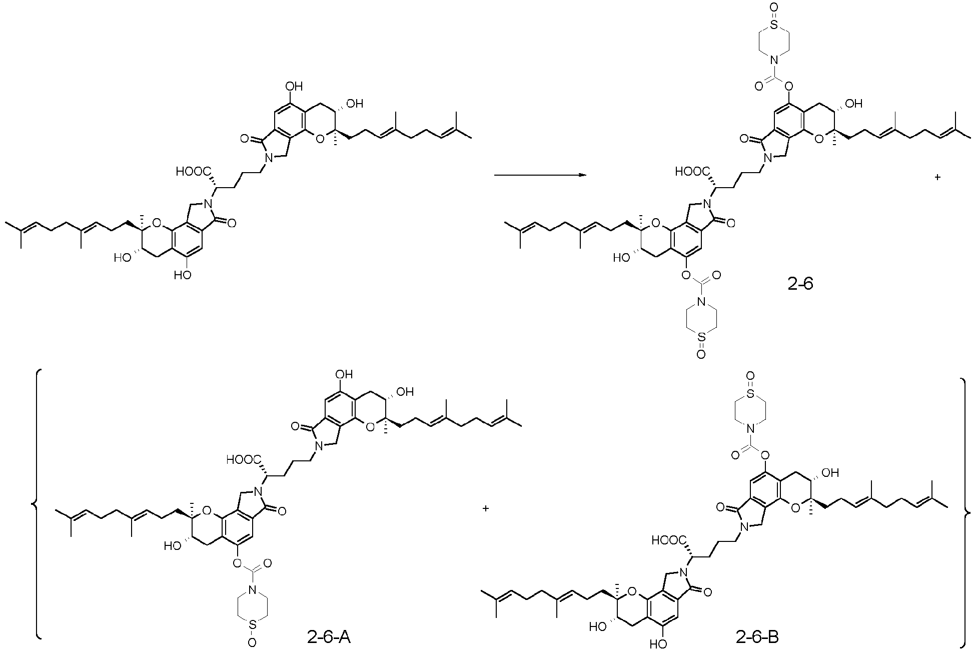

A solution of (S)-2,5-bis((2R,3S)-2-((E)-4,8-dimethylnona-3,7-dien-1-yl)-3,5-dihydroxy-2-methyl-7-oxo-3,4,7,9-tetrahydropyran o[2,3-E]isoindol-8(2H)-yl)pentanoic acid (500 mg, 0.575 mmol) was dissolved in dry tetrahydrofuran (50 mL), triethylamine was added dropwise, then p-nitrophenyl chloroformate (406 mg, 2.06 mmol, dissolved in 5 mL of tetrahydrofuran) was added dropwise, and the reaction was stirred for 2 hours. 1-oxothiomorpholine hydrochloride (447 mg, 2.87 mmol) was added and stirring was continued for 2 hours. The reaction was monitored by high performance liquid chromatography coupled with mass spectrometry. The reaction solution was concentrated under reduced pressure to obtain a crude product, which was purified by high performance liquid chromatography to obtain a disubstituted compound 2-6 (27.33 mg) and a monosubstituted mixture of 2-6-A and 2-6-B (17.64 mg).

### Compounds 2-6:

### Purification Condition 1:

Column: SunFire Prep C18 OBD 19 mm × 150 mm × 5.0 µm.

Mobile phase A: acetonitrile; mobile phase B: water (0.05% ammonium bicarbonate).

| Time [min] | Mobile phase A [%] | Mobile phase B [%] | Flow rate [mL/min] |
|---|---|---|---|
| 0.00 | 20 | 80 | 28 |
| 2.00 | 20 | 80 | 28 |
| 22.00 | 80 | 20 | 28 |

ESI-MS (m/z): 1159.5[M+H]⁺.

¹H NMR (400 MHz, DMSO): δ 7.06 (d, *J =* 12.0 Hz, 2H), 5.29 (s, 2H), 5.14 - 4.99 (m, 4H), 4.70 (s, 2H), 4.34 (d, *J =* 26.4 Hz, 4H), 4.10 (s, 2H), 3.93 (d, *J =* 12.3 Hz, 4H), 3.77 (dd, *J =* 11.5, 6.3 Hz, 4H), 3.52 (s, 2H), 3.12 - 2.94 (m, 4H), 2.83 (s, 5H), 2.54 (s, 1H), 2.44 - 2.35 (m, 1H), 2.11 (d, *J* = 7.8 Hz, 4H), 2.05 - 1.83 (m, 10H), 1.66 - 1.47 (m, 20H), 1.30 - 1.14 (m, 8H).

### Mixture 2-6-A & 2-6-B:

### Purification Condition 2:

Column: SunFire Prep C18 OBD 19 mm × 150 mm × 5.0 µm.

Mobile phase A: acetonitrile; mobile phase B: water (0.05% ammonium bicarbonate).

| Time [min] | Mobile phase A [%] | Mobile phase B [%] | Flow rate [mL/min] |
|---|---|---|---|
| 0.00 | 35 | 65 | 28 |
| 2.00 | 35 | 65 | 28 |
| 22.00 | 90 | 10 | 28 |

ESI-MS (m/z): 1014.3[M+H]⁺.

¹H NMR (400 MHz, DMSO): δ 9.79 (d, *J* = 11.8 Hz, 2H), 7.05 (d, *J* = 11.0 Hz, 2H), 6.63 (d, *J =* 10.7 Hz, 2H), 5.29 (s, 2H), 5.16 (s, 2H), 5.14 - 5.00 (m, 8H), 4.67 (s, 2H), 4.41 (d, *J =* 17.7 Hz, 2H), 4.28 (d, *J =* 22.5 Hz, 4H), 4.15 (d, *J* = 15.6 Hz, 6H), 3.92 (s, 4H), 3.76 (d, *J* = 10.2 Hz, 6H), 3.57 - 3.44 (m, 4H), 3.03 (d, J = 41.1 Hz, 6H), 2.92 - 2.75 (m, 8H), 2.46 (s, 2H), 2.10 (s, 8H), 2.04 - 1.76 (m, 20H), 1.57 (d, *J =* 33.5 Hz, 42H), 1.19 (dd, J = 24.8, 11.1 Hz, 14H).

The remaining compounds shown in the following table were prepared in a similar manner as described above:

| Compound Number | Mass spectrometric data (ESI-MS (m/z): [M + H]⁺.) |
|---|---|
| 1-1 | 619.3 |
| 1-2 | 635.3 |
| 1-4 | 633.3 |
| 1-5 | 633.3 |
| 1-6 | 663.3 |
| 2-3-A | 954.5 |
| 2-3-B | 954.5 |
| 2-4-A | 927.5 |
| 2-4-B | 927.5 |
| 2-7-A | 998.5 |
| 2-7-B | 998.5 |
| 2-8-A | 1010.6 |
| 2-8-B | 1010.6 |
| 2-9-A | 980.6 |
| 2-9-B | 980.6 |
| 2-11 | 1179.6 |
| 2-11-A | 1024.6 |
| 2-11-B | 1024.6 |
| 2-12 | 1209.6 |
| 2-12-A | 1039.6 |
| 2-12-B | 1039.6 |
| 2-13 | 1177.6 |
| 2-13-A | 1023.6 |
| 2-13-B | 1023.6 |
| 2-14 | 1121.6 |
| 2-14-A | 995.5 |
| 2-14-B | 995.5 |
| 2-15 | 1151.6 |
| 2-15-A | 1010.6 |
| 2-15-B | 1010.6 |
| 2-16 | 1123.6 |
| 2-16-A | 996.6 |
| 2-16-B | 996.6 |
| 2-17 | 1123.5 |
| 2-17-A | 996.5 |
| 2-17-B | 996.5 |
| 2-18 | 1095.6 |
| 2-18-A | 982.5 |
| 2-18-B | 982.5 |
| 2-19 | 1067.6 |
| 2-19-A | 968.5 |
| 2-19-B | 968.5 |
| 2-20-A | 1063.6 |
| 2-20-B | 1063.6 |
| 2-21 | 1261.7 |
| 2-21-A | 1065.6 |
| 2-21-B | 1065.6 |
| 2-24 | 1069.6 |
| 2-24-A | 969.5 |
| 2-24-B | 969.5 |
| 2-25 | 1029.42 |
| 2-25-A | 949.5 |
| 2-25-B | 949.5 |
| 2-26 | 1089.4 |
| 2-26-A | 979.5 |
| 2-26-B | 979.5 |
| 2-27 | 1029.4 |
| 2-27-A | 949.4 |
| 2-27-B | 949.4 |
| 2-28 | 1027.4 |
| 2-34 | 946.5 |
| 2-35 | 975.5 |
| 2-36 | 893.5 |
| 2-37 | 1119.6 |
| 2-37-A | 994.5 |
| 2-37-B | 994.5 |
| 2-38 | 1175.6 |
| 2-38-A | 1022.6 |
| 2-38-B | 1022.6 |
| 2-39 | 1147.6 |
| 2-39-A | 1008.5 |
| 2-39-B | 1008.5 |
| 2-40 | 1163.6 |
| 2-40-A | 1108.5 |
| 2-40-B | 1108.5 |
| 2-41 | 885.5 |
| 2-42 | 885.5 |
| 2-43 | 885.5 |
| 2-44 | 901.4 |
| 2-45 | 1111.6 |
| 2-46 | 1207.5 |
| 2-47 | 1175.5 |
| 2-48 | 1093.6 |
| 2-49 | 1189.5 |
| 2-50 | 1157.5 |
| 2-51 | 1079.5 |
| 2-52 | 1175.4 |
| 2-53 | 1143.4 |
| 2-54 | 1071.6 |
| 2-55 | 1167.5 |
| 2-56 | 1135.5 |
| 2-57 | 1071.6 |
| 2-58 | 1167.5 |
| 2-59 | 1135.5 |

### Biological data

### Experimental example 1: the activation effect of the compounds on Plasminogen (Glu-Typ)

### 1. Test Method

### (1) Formulation of compounds

The test compounds of the present invention and the positive compound are all set at three concentration points, i.e., 200 µM, 150 µM and 100 µM, respectively; the final concentration in DMSO was 2.5%.

### (2) Information of main reagents and instruments

Plasminogen (Glu-Typ) (sigma); urokinase-type plasminogen activator (u-PA) (Aladdin); vLK-pNA (absin); Multifunctional microplate reader (Thermo).

### (3) Test system and signal detection

Plasminogen, the urokinase-type Plasminogen activator (u-PA) and VLK-pNA were formulated in a buffer [Tris-HCl (50 mM, pH 7.4) + NaCl (100 mM) + Tween-80 (0.01%)] to obtain mother solutions at concentrations of 100 nM (5x), 100 IU/ml (5x), and 5 mM (5x), respectively. 20 µl of 5 × Plasminogen (Glu-Typ), 20 µl of 5 × urokinase-type Plasminogen activator (u-PA) and 20 µl of 5 × VLK-pNA were added to a 96-well plate. After mixing uniformly, 40 µl of the test compounds or positive compound were added to the 96-well plate, and a control group (no compound) was set up. The mixture was uniformly mixed well. The kinetic detection was performed using a Thermo multifunctional microplate reader, the detection wavelength was 405 nm, and the activation factor was calculated.

### 2. Test Results

The following results were obtained by plotting the absorbance value at 405 nm (A405) versus time squared (t²), using the slope of the curve for analysis, and calculating the activation multiples by dividing the slope of the compound group by the slope of the control group (see Table 1-1 and Table 1-2).

**Table 1-1. Activation potency of the compounds on plasminogen**

| **Compound** | **Activation multiples (5 min)** | | |
|---|---|---|---|
| | 200 µM | 150 µM | 100 µM |
| Positive compound | 8.62 | 2.62 | 1.71 |
| 2-1 | 42.97 | 15.93 | 5.22 |
| 2-2 | 42.23 | 21.12 | 6.66 |

**Table 1-2. Activation potency of the compounds on plasminogen**

| **Compound** | **Activation multiples (5 min)** | | |
|---|---|---|---|
| | 200 µM | 150 µM | 100 µM |
| Positive compound | 12.49 | 4.54 | 3.02 |
| 2-5 | 90.00 | 52.96 | 4.65 |

The results show that at the same concentration, compounds 2-1, 2-2 and 2-5 have better activation potency on plasminogen than the positive compounds under the test system disclosed by the present invention.

### Experimental example 2: the fibrinolysis effect of the compounds shown by thromboelastography

**Main Instruments and Reagents:** thromboelastography TEG (Udibio); urokinase-type plasminogen activator (u-PA) (Aladdin); reagent 1 (the main component is kaolin); reagent 2 (main component is calcium chloride).

### 1.Test Method:

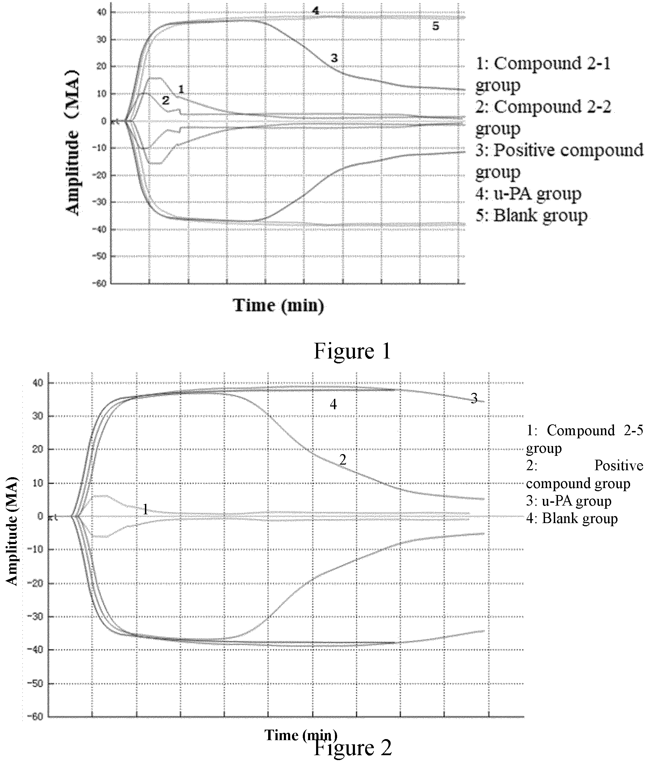

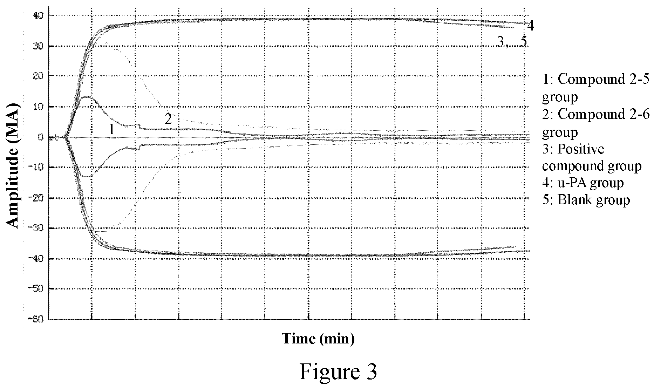

(1) Test group setting: uPA group: urokinase-type plasminogen activator (u-PA) (1000 U/ml); positive compound/compound 2-1/compound 2-2 group: test compound (200 µM) + u-PA (1000 U/ml); blank group: vehicle.
(2) Formulation of compound solution: the compound was formulated using a 0.5 mg/ml trometamol solution to obtain a 4 mM mother solution, adjusted to pH 9, and diluted to a 2 mM 10X mother solution. The final test concentration was 200 µM. u-PA was formulated as a 50,000 U/ml mother solution using normal saline, and diluted to a 10,000 U/ml 10X mother solution. The final test concentration was 1000 U/ml.
(3) Test: 100 µL of u-PA was added to a sample cup (containing reagent 1) containing 800 µL of the whole blood of rat (anticoagulated with sodium citrate), 100 µL of the test compound was added, and the cup was inverted to mix well. 340 µL of the sample from the sample cup was added to the reaction cup containing 20 µL of reagent 2, and the instrument was allowed to run. The results are shown in Table 2-1, Table 2-2, Table 2-3, Figure 1, Figure 2 and Figure 3. The compound concentration as shown in Table 2-1, Table 2-2, Figure 1 and Figure 2 was 200 µM; and the compound concentration as shown in Table 2-3 and Figure 3 was 100 µM.

**Table 2-1 Thromboelastographic Data**

| **Compound** | **Ma(mm)** |
|---|---|
| Positive compound | 72.9 |
| 2-1 | 4.7 |
| 2-2 | 4.7 |

**Table 2-2 Thromboelastographic Data**

| **Compound** | **Ma(mm)** |
|---|---|
| Positive compound | 73.1 |
| 2-5 | 1.9 |

**Table 2-3 Thromboelastographic Data**

| **Compound** | **Ma(mm)** |
|---|---|
| Positive compound | 76.4 |
| 2-6 | 8.8 |

In this test system, according to the Ma values in combination with Figures 1 and 2, it can be seen that compounds 2-1, 2-2, 2-5 and 2-6 can directly affect the strength (Ma) of the blood clot formed and prevent thrombosis, and have superior thrombolytic activity to the positive compound.

The structure of the positive compound is as follows:

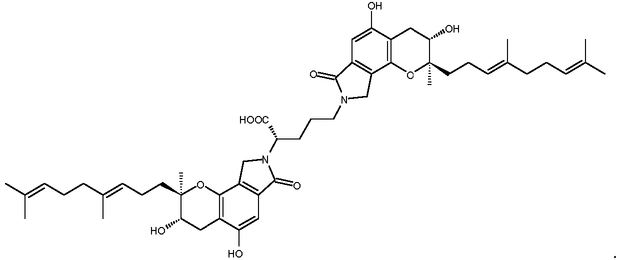

## Claims

1. A compound or a pharmaceutically acceptable salt, an ester, a solvate, a stereoisomer, a tautomer, a polymorph, an isotopically labeled product, a metabolite, a prodrug, or a mixture thereof, the compound being: 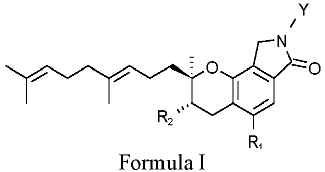 wherein,
Y is selected from the group consisting of 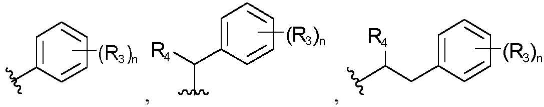 and 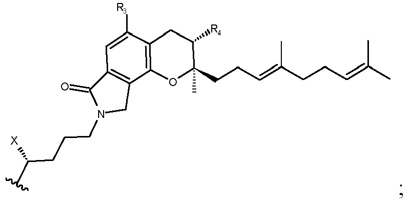
R₁ and R₃ are each independently selected from the group consisting of carboxyl, hydroxy, C₁₋₆ alkyl, C₁₋₆ alkoxy, C₁₋₆ alkoxyacyloxy, C₁₋₆ alkylacyloxy, C₁₋₆ alkoxyalkylaminoacyloxy, C₁₋₆ alkylaminoacyloxy, C₄₋₁₀ heterocyclylacyloxy, -O-C₄₋₁₀ heterocycyl, C₁₋₆ alkylsulfamoyloxy, C₄₋₁₀ heterocycylsulfonyloxy, glycosyl, -O-P(O)(OH)₂, -O-P(O)₂OH, -O-S(O)₂OH, amino, C₁₋₆ alkylamino, C₁₋₆ alkylamido, C₄₋₁₀ heterocyclylamido, halogen, cyano, C₂₋₆ alkenyl, and C₂₋₆ alkynyl; the C₄₋₁₀ heterocyclylacyloxy, -O-C₄₋₁₀ heterocycyl, C₁₋₆ alkylacyloxy, C₁₋₆ alkyl, C₁₋₆ alkoxy, or C₁₋₆ alkylaminoacyloxy being optionally substituted with one or more substituents selected from the group consisting of hydroxy, carboxyl, C₁₋₆ alkyl, C₁₋₆ alkoxy, C₁₋₆ alkylcarbonyl, C₁₋₆ alkoxycarbonyl, C₁₋₆ haloalkyl, C₁₋₆ alkoxyacyloxy, 4-10-membered heterocyclyl, C₁₋₆ hydroxyalkyl, and -O-P(O)(OH)₂;
R₂ and R₄ are each independently selected from the group consisting of hydrogen, hydroxyl, carboxyl, ester group, C₁₋₆ alkoxy, carboxyl-substituted C₁₋₆ alkylacyloxy, -OC(=O)-C₄₋₁₀ heterocyclyl, -O-P(O)₂OH, -O-S(O)₂OH, and -O-S(O)₂NH₂, the C₄₋₁₀ heterocycyl being optionally substituted with one or more substituents selected from C₁₋₆ alkyl;
R₁ and R₂ are not simultaneously hydroxyl when Y is selected from 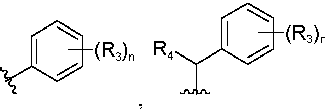 or 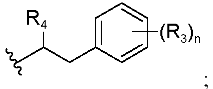
n = 0, 1, 2, 3, 4, or 5;
X is selected from the group consisting of -CO₂H and its carboxylic acid isosteres, -CO₂C₁₋₆ alkyl, -C(=O)SH, 4-10-membered heteroaryl, choline carboxylate, -C(=O)NHC₁₋₆ alkyl, -C(=O)NHS(=O)₂C₁₋₆ alkylamino and -C(=O)NHS(=O)₂C₁₋₆ alkyl, the -CO₂C₁₋₆ alkyl, 4-10 membered heteroaryl, choline carboxylate, -C(=O)NHC₁₋₆ alkyl, -C(=O)NHS(=O)₂C₁₋₆ alkylamino and -C(=O)NHS(=O)₂C₁₋₆ alkyl being optionally substituted with one or more substituents selected from the group consisting of C₄₋₁₀ heterocyclyl, C₁₋₆ alkoxy, 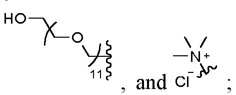 and
when X is -CO₂H, R₁, R₂, R₃ and R₄ are not simultaneously hydroxyl;
where the wavy " 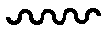 " represents the attachment point to the remainder of the molecule.

2. The compound according to claim 1, or a pharmaceutically acceptable salt, an ester, a solvate, a stereoisomer, a tautomer, a polymorph, an isotopically labeled product, a metabolite, a prodrug, or a mixture thereof, wherein:
R₁ and R₃ are each independently selected from the group consisting of carboxyl, hydroxy, C₁₋₆ alkoxy, C₁₋₆ alkoxyacyloxy, C₁₋₆ alkylaminoacyloxy, C₄₋₁₀ heterocyclylacyloxy, -O-C₄₋₁₀ heterocycyl, C₄₋₁₀ heterocycylsulfonyloxy, -O-P(O)(OH)₂, -O-S(O)₂OH, and C₄₋₁₀ heterocyclylamido; the C₄₋₁₀ heterocyclylacyloxy, -O-C₄₋₁₀ heterocycyl, or C₁₋₆ alkylaminoacyloxy being optionally substituted with one or more substituents selected from the group consisting of hydroxy, carboxyl, C₁₋₆ alkyl, C₁₋₆ alkoxy, C₁₋₆ alkoxyacyloxy, C₁₋₆ alkoxycarbonyl, C₁₋₆ alkyl-C(=O)-, 4-10-membered heterocyclyl, C₁₋₆ hydroxyalkyl, and -O-P(O)(OH)₂.

3. The compound according to any one of claims 1-2, or a pharmaceutically acceptable salt, an ester, a solvate, a stereoisomer, a tautomer, a polymorph, an isotopically labeled product, a metabolite, a prodrug, or a mixture thereof, wherein:
R₁ and R₃ are each independently selected from the group consisting of carboxyl, hydroxy, C₁₋₃ alkoxy, C₁₋₃ alkoxyacyloxy, C₁₋₃ alkylaminoacyloxy, C₄₋₈ heterocyclylacyloxy, -O-C₄₋₈ heterocyclyl, C₄₋₈ heterocycylsulfonyloxy, -O-P(=O)(OH)₂, -O-S(O)₂OH, and C₄₋₈ heterocyclylamido; the C₄₋₈ heterocyclylacyloxy, -O-C₄₋₈ heterocyclyl, or C₁₋₃ alkylaminoacyloxy being optionally substituted with one or more substituents selected from the group consisting of hydroxy, carboxyl, C₁₋₃ alkyl, C₁₋₃ alkoxy, C₁₋₃ alkoxyacyloxy, C₁₋₃ alkoxycarbonyl, C₁₋₃ alkyl-C(=O)-, 4-8-membered heterocyclyl, C₁₋₃ hydroxyalkyl, and -O-P(=O)(OH)₂.

4. The compound according to any one of claims 1-3, or a pharmaceutically acceptable salt, an ester, a solvate, a stereoisomer, a tautomer, a polymorph, an isotopically labeled product, a metabolite, a prodrug, or a mixture thereof, wherein:
R₁ and R₃ are each independently selected from the group consisting of carboxyl, hydroxy, -OC(=O)N(CH₃)₂, -OC(=O)-morpholinyl, -OC(=O)NHCH(CH₃)₂, -OC(=O)OCH₃, -OC(=O)-thiomorpholine dioxide, -OC(=O)-thiomorpholine monoxide, -OC(=O)-thiomorpholine, -OC(=O)N(CH₃)-tetrahydropyran, -OC(=O)-piperidine, -OSO₂-morpholine, -OC(=O)-piperazine, 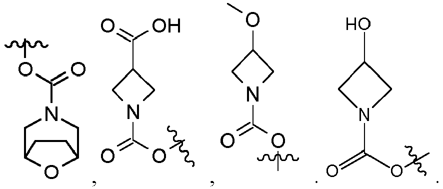 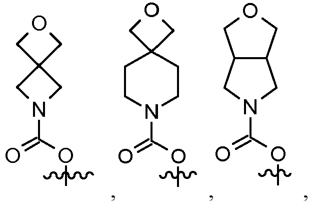 -O-tetrahydropyran, -O-P(=O)(OH)₂, methoxy, glucopyranosyl and -O-S(O)₂OH, the -OC(=O)-piperidine, -OC(=O)-piperazine, -O-tetrahydropyran, or methoxy is optionally substituted with one or more substituents selected from the group consisting of hydroxyl, carboxyl, CH₃OC(=O)-, -C(=O)CH₃, -OCH₃, piperidinyl, morpholinyl, hydroxy, -CH₂OH, -O-P(=O)(OH)₂, and oxo,
where the wavy line " 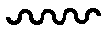 " represents the attachment point to the remainder of the molecule.

5. The compound according to any one of claims 1-4, or a pharmaceutically acceptable salt, an ester, a solvate, a stereoisomer, a tautomer, a polymorph, an isotopically labeled product, a metabolite, a prodrug, or a mixture thereof, wherein:
R₁ and R₃ are each independently selected from the group consisting of carboxyl, hydroxy, -OC(=O)N(CH₃)₂, -OC(=O)NHCH(CH₃)₂, -OC(=O)OCH₃, -O-P(=O)(OH)₂, methoxy, -O-S(O)₂OH, 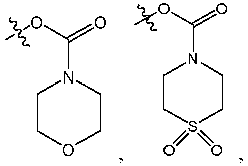 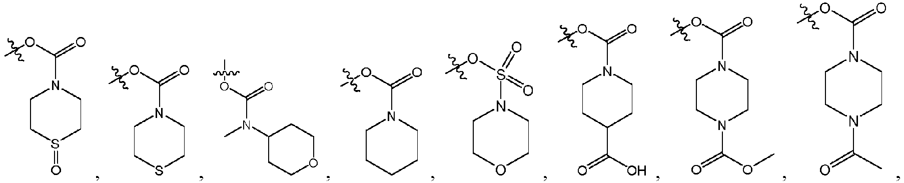 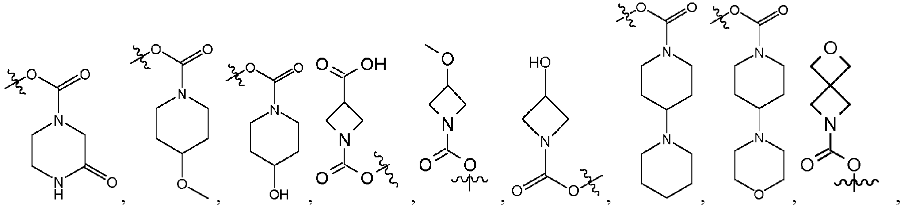 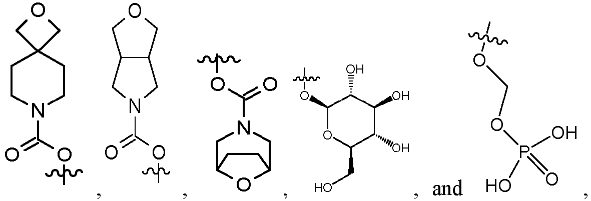 where the wavy line " 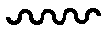 " represents the attachment point to the remainder of the molecule.

6. The compound according to any one of claims 1-5, or a pharmaceutically acceptable salt, an ester, a solvate, a stereoisomer, a tautomer, a polymorph, an isotopically labeled product, a metabolite, a prodrug, or a mixture thereof, wherein:
R₂ and R₄ are each independently selected from the group consisting of hydrogen, carboxyl, hydroxy, C₁₋₆ alkoxy, carboxyl-substituted C₁₋₆ alkylacyloxy, -OC(=O)-C₄₋₁₀ heterocyclyl, -O-P(O)₂OH, -O-S(O)₂OH, and -O-S(O)₂NH₂, the C₄₋₁₀ heterocyclyl being optionally substituted with one or more substituents selected from C₁₋₆ alkyl.

7. The compound according to any one of claims 1-6, or a pharmaceutically acceptable salt, an ester, a solvate, a stereoisomer, a tautomer, a polymorph, an isotopically labeled product, a metabolite, a prodrug, or a mixture thereof, wherein:
R₂ and R₄ are each independently selected from the group consisting of carboxyl, hydroxy, C₁₋₆ alkoxy, carboxyl-substituted C₁₋₆ alkylacyloxy, -OC(=O)-piperazinyl, -O-P(O)₂OH, -O-S(O)₂OH, and -O-S(O)₂NH₂, the piperazine being optionally substituted with one or more substituents selected from C₁₋₆ alkyl.

8. The compound according to any one of claims 1-7, or a pharmaceutically acceptable salt, an ester, a solvate, a stereoisomer, a tautomer, a polymorph, an isotopically labeled product, a metabolite, a prodrug, or a mixture thereof, wherein:
R₂ and R₄ are each independently selected from the group consisting of carboxyl, hydroxy, 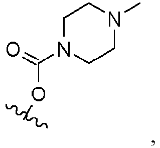 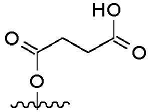 , **and** -O-S(O)₂NH₂,
where the wavy line 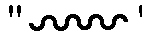 represents the attachment point to the remainder of the molecule.

9. The compound according to any one of claims 1-8, or a pharmaceutically acceptable salt, an ester, a solvate, a stereoisomer, a tautomer, a polymorph, an isotopically labeled product, a metabolite, a prodrug, or a mixture thereof, wherein:
n = 1, 2, or 3.

10. The compound according to any one of claims 1-9, or a pharmaceutically acceptable salt, an ester, a solvate, a stereoisomer, a tautomer, a polymorph, an isotopically labeled product, a metabolite, a prodrug, or a mixture thereof, wherein:
X is selected from the group consisting of -CO₂H and its carboxylic acid isosteres, -CO₂C₁₋₆ alkyl, -C(=O)SH, 4-10-membered heteroaryl, choline carboxylate, -C(=O)NHC₁₋₆ alkyl, -C(=O)NHS(=O)₂C₁₋₆ alkylamino and -C(=O)NHS(=O)₂C₁₋₆ alkyl, the -CO₂C₁₋₆ alkyl, 4-10-membered heteroaryl, choline carboxylate, -C(=O)NHC₁₋₆ alkyl, -C(=O)NHS(=O)₂C₁₋₆ alkylamino and -C(=O)NHS(=O)₂C₁₋₆ alkyl being optionally substituted with one or more substituents selected from the group consisting of 4-10 membered heterocyclyl, 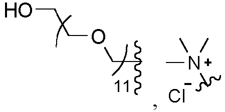 and -C₁₋₆ alkoxy,
where the wavy line" 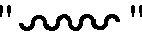 "represents the attachment point to the remainder of the molecule.

11. The compound according to any one of claims 1-10, or a pharmaceutically acceptable salt, an ester, a solvate, a stereoisomer, a tautomer, a polymorph, an isotopically labeled product, a metabolite, a prodrug, or a mixture thereof, wherein:
X is selected from the group consisting of -CO₂H, -CO₂C₁₋₆ alkyl, -C(=O)SH, tetrazolyl, -C(=O)NHC₁₋₆ alkyl, -C(=O)NHS(=O)₂C₁₋₆ alkyl and -C(=O)NHS(=O)₂N(C₁₋₆ alkyl)₂, the -CO₂C₁₋₆ alkyl and -C(=O)NHC₁₋₆ alkyl being each optionally substituted with a substituent selected from the group consisting of morpholinyl, 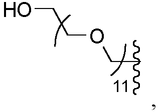 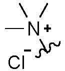 and -C₁₋₆ alkoxy,
where the wavy line " 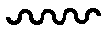 " represents the attachment point to the remainder of the molecule.

12. The compound according to any one of claims 1-11, or a pharmaceutically acceptable salt, an ester, a solvate, a stereoisomer, a tautomer, a polymorph, an isotopically labeled product, a metabolite, a prodrug, or a mixture thereof, wherein:
X is selected from the group consisting of -CO₂H, -C(=O)NHCH₃, 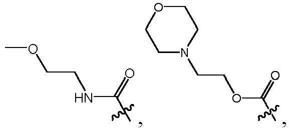 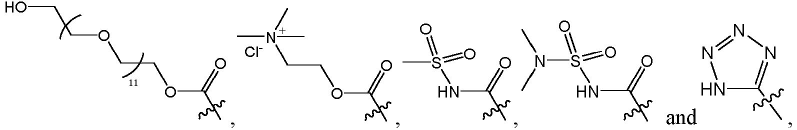
where the wavy line 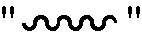 represents the attachment point to the remainder of the molecule.

13. The compound according to any one of claims 1-12, or a pharmaceutically acceptable salt, an ester, a solvate, a stereoisomer, a tautomer, a polymorph, an isotopically labeled product, a metabolite, a prodrug, or a mixture thereof,
the compound being selected from the group consisting of:

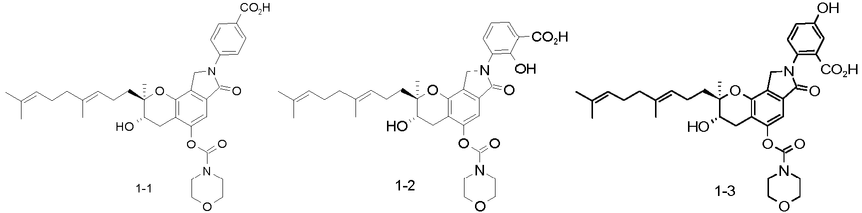

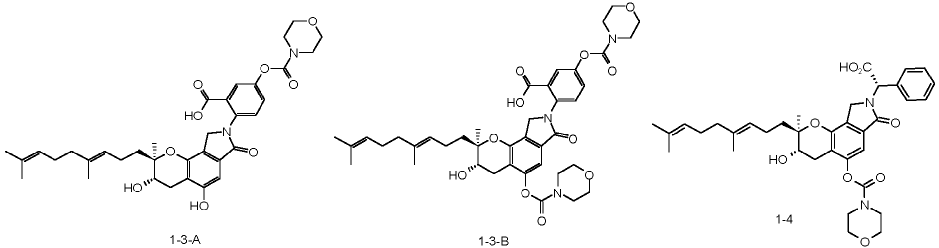

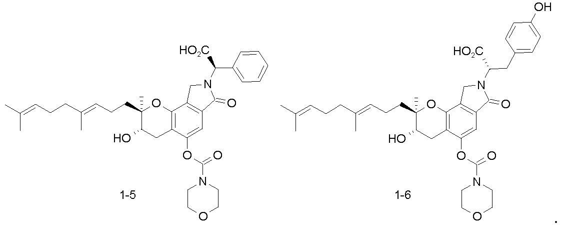

14. The compound according to any one of claims 1-12, or a pharmaceutically acceptable salt, an ester, a solvate, a stereoisomer, a tautomer, a polymorph, an isotopically labeled product, a metabolite, a prodrug, or a mixture thereof, wherein the compound is: 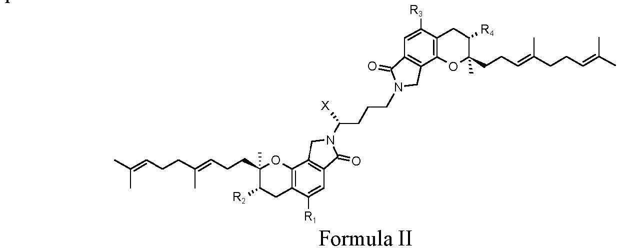 wherein,
R₁, R₂, R₃, R₄, and X are as defined in any one of claims 1-12, and
when X is -CO₂H, R₁, R₂, R₃ and R₄ are not simultaneously hydroxyl.

15. The compound according to claim 14, or a pharmaceutically acceptable salt, an ester, a stereoisomer, a polymorph, a solvate, an N-oxide, an isotopically labeled product, a metabolite, and a prodrug, the compound being selected from the group consisting of:

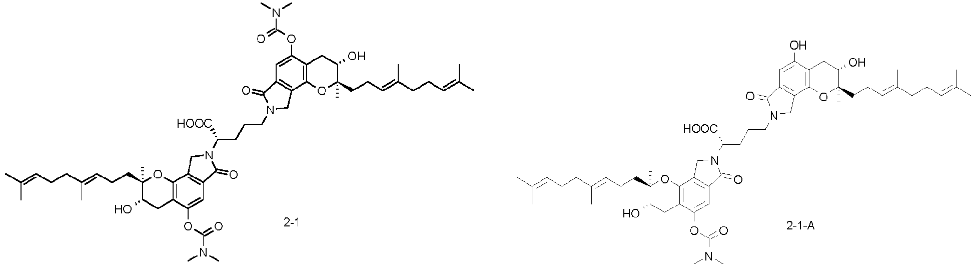

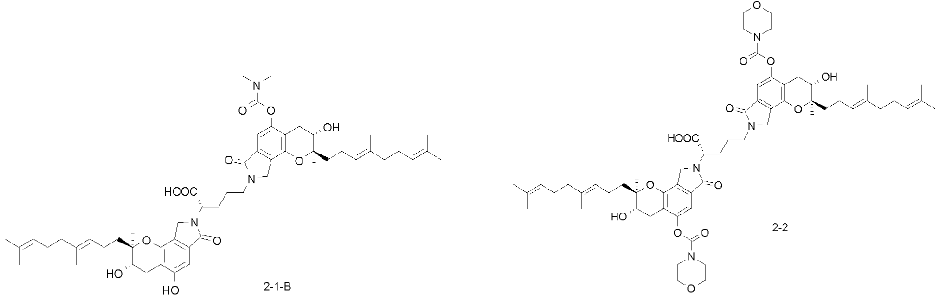

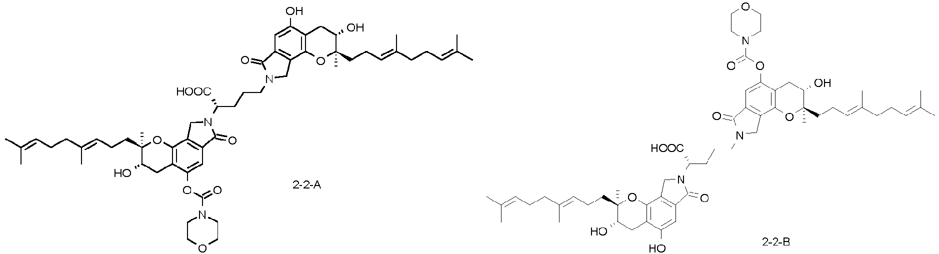

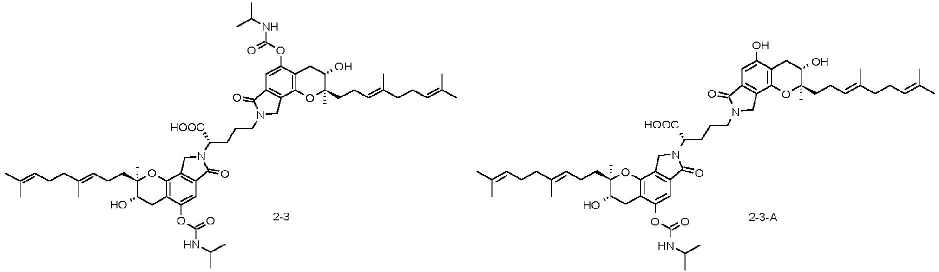

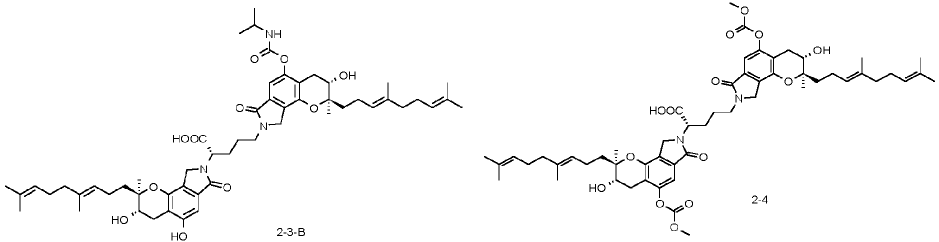

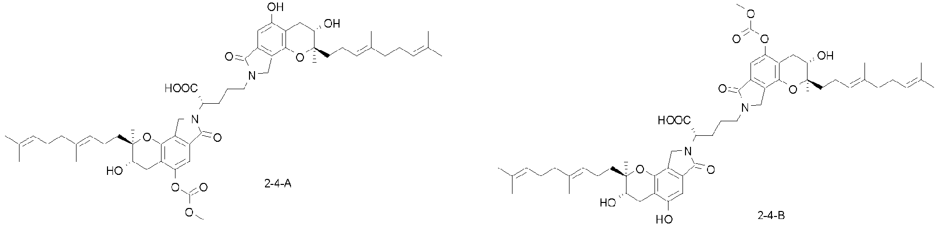

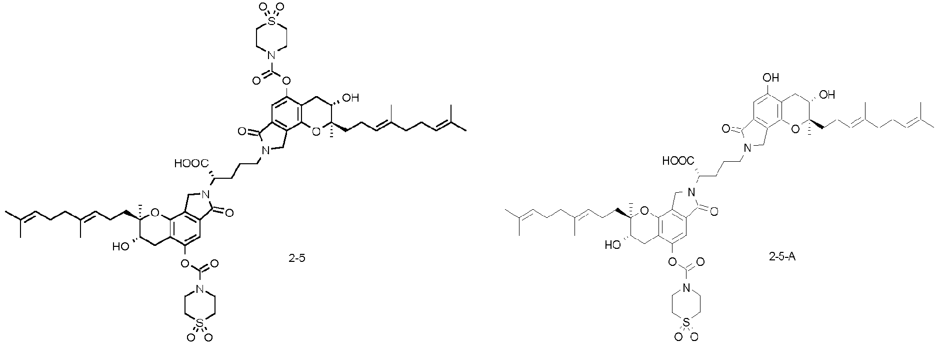

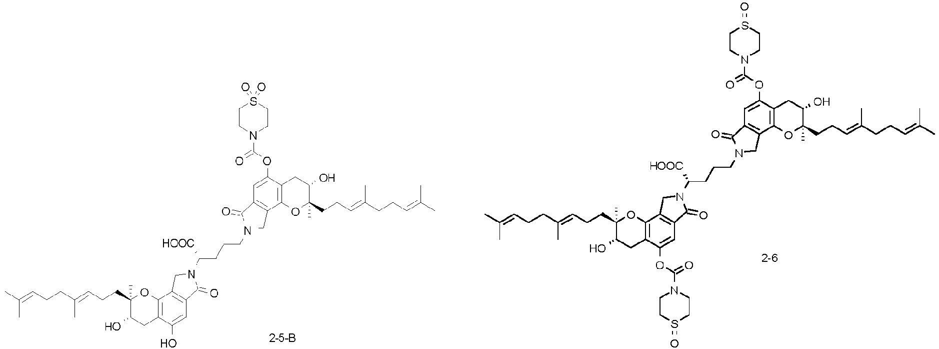

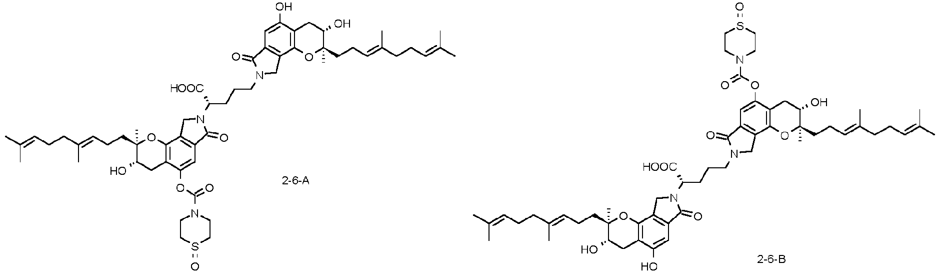

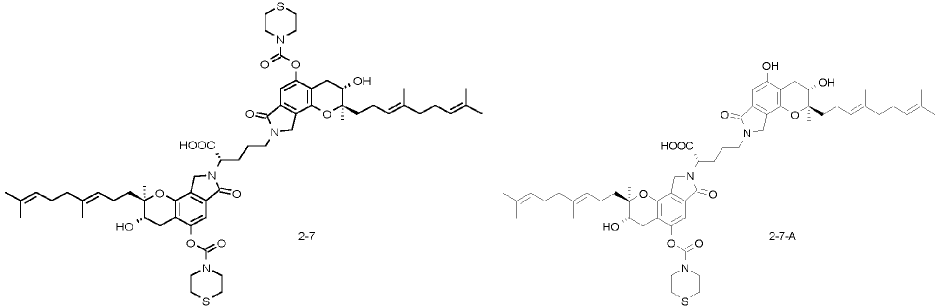

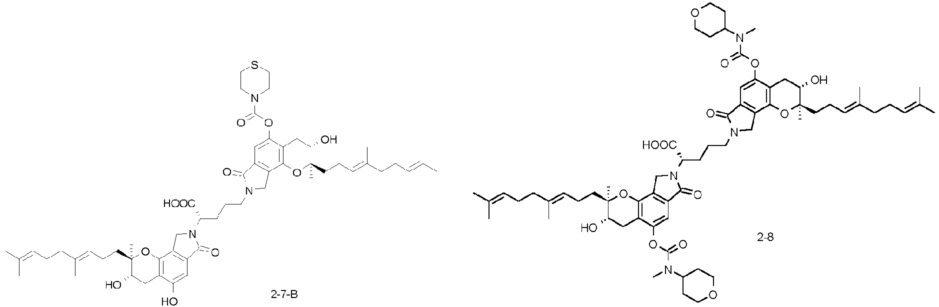

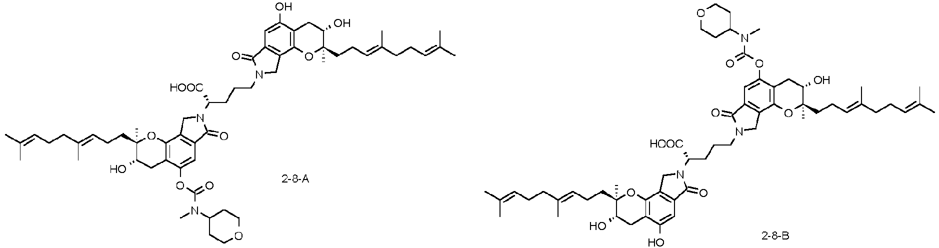

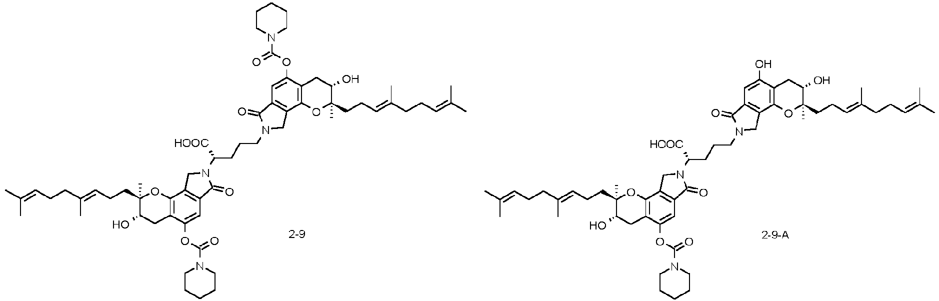

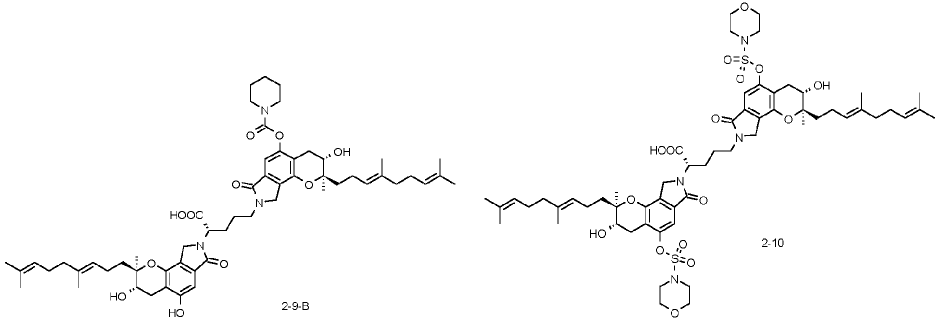

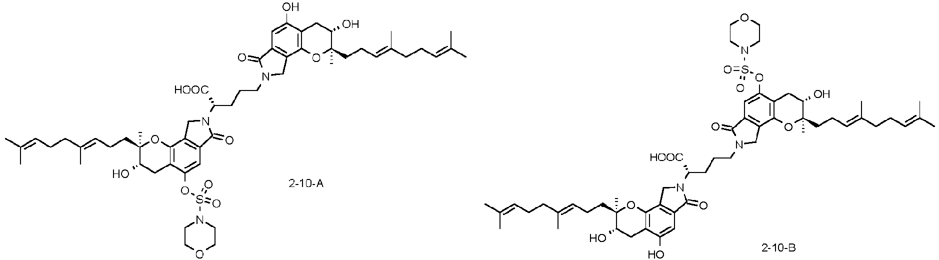

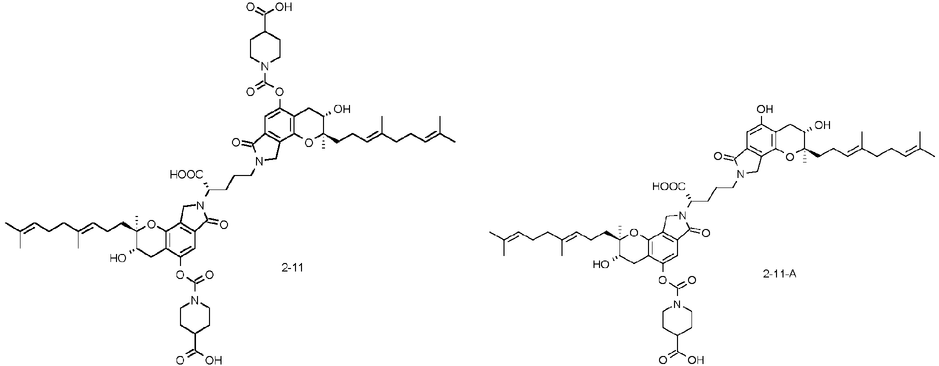

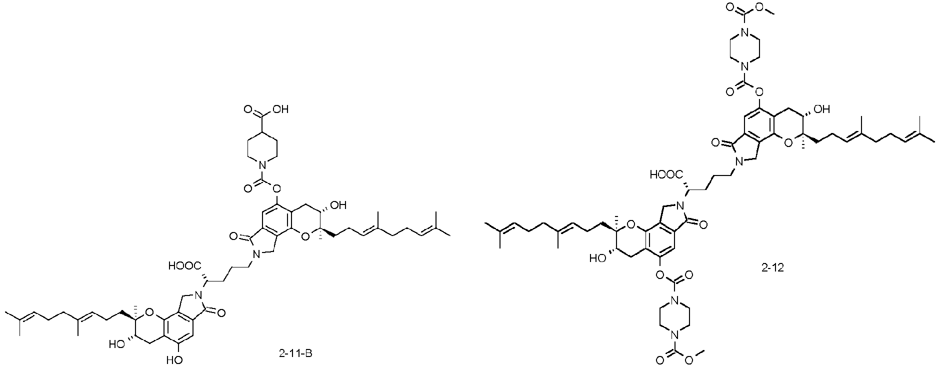

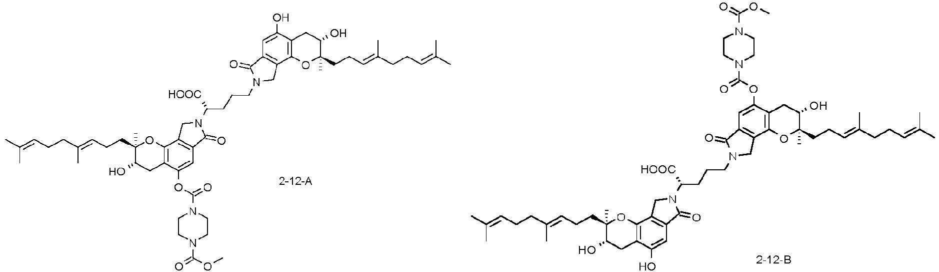

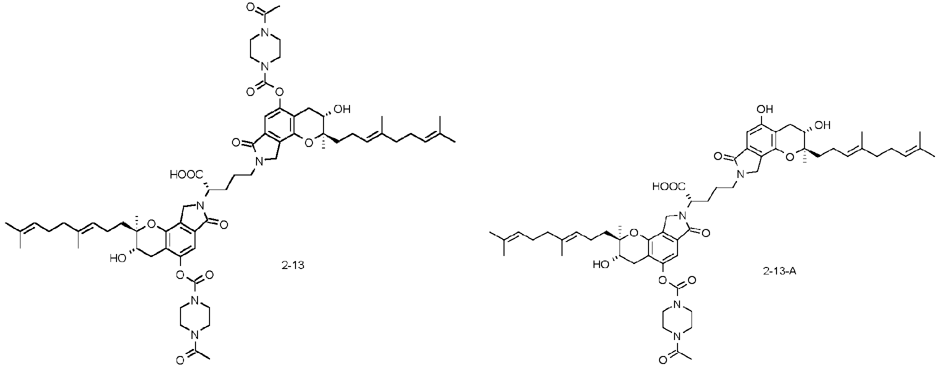

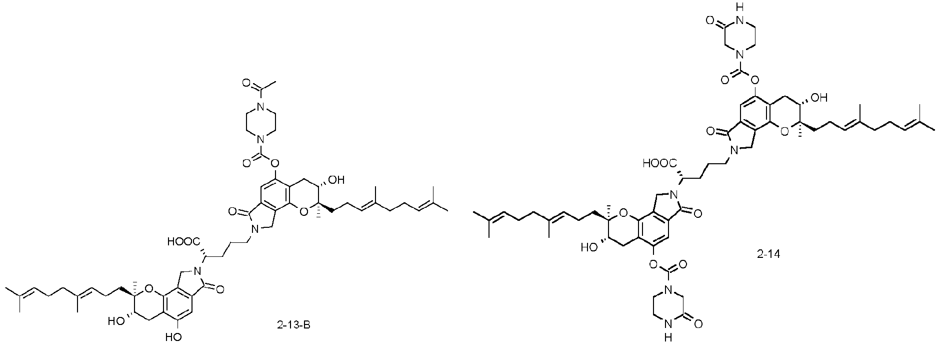

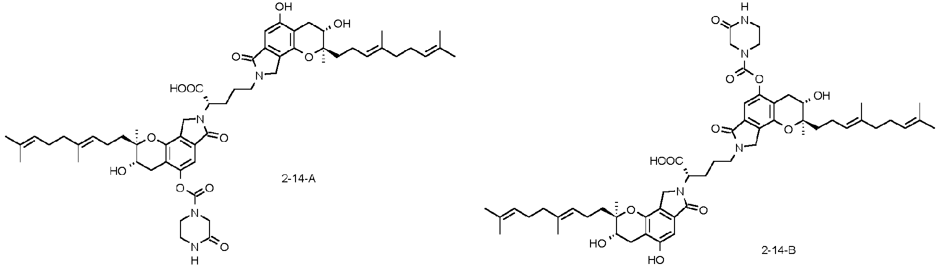

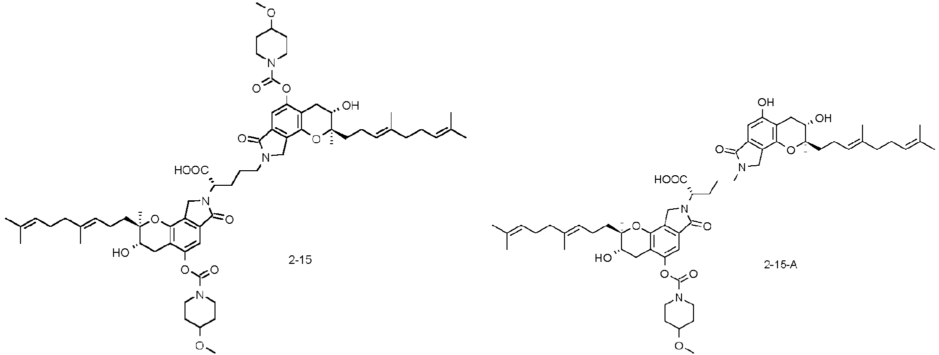

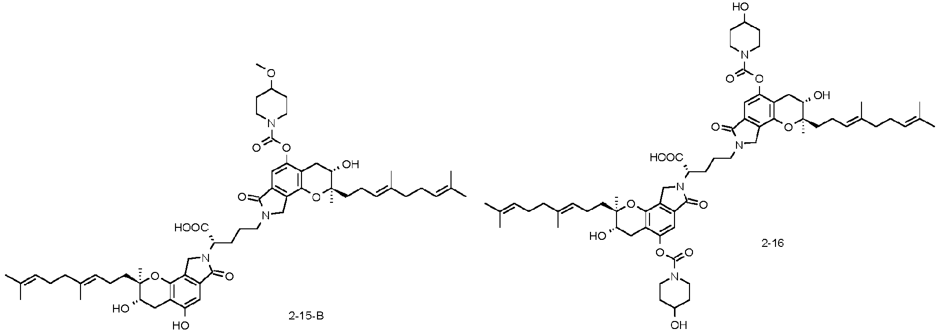

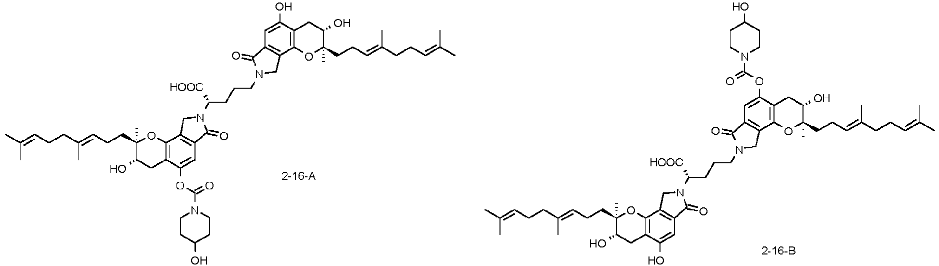

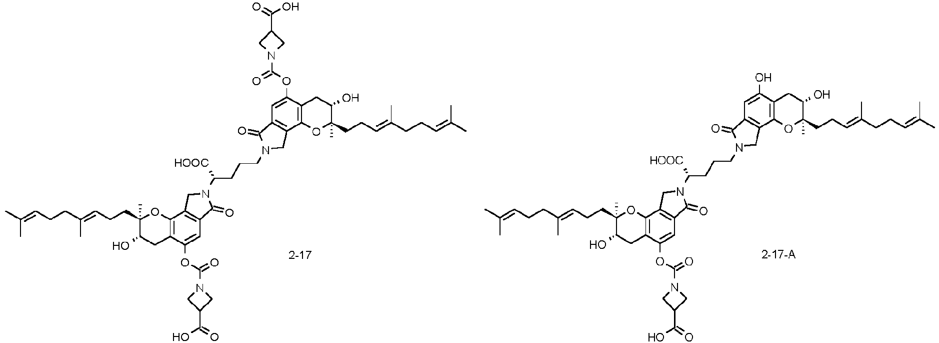

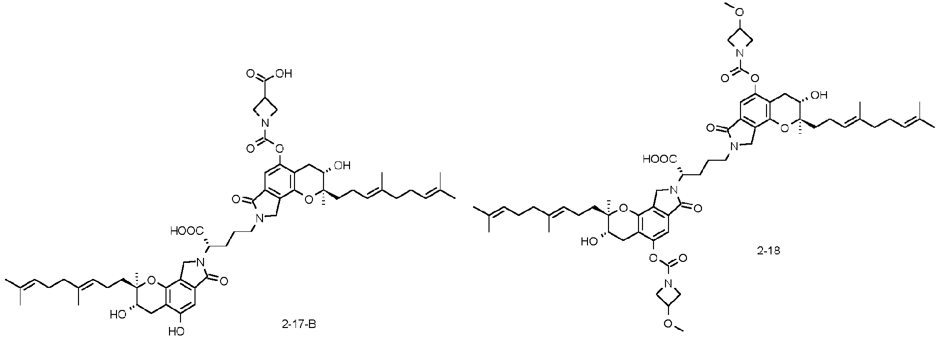

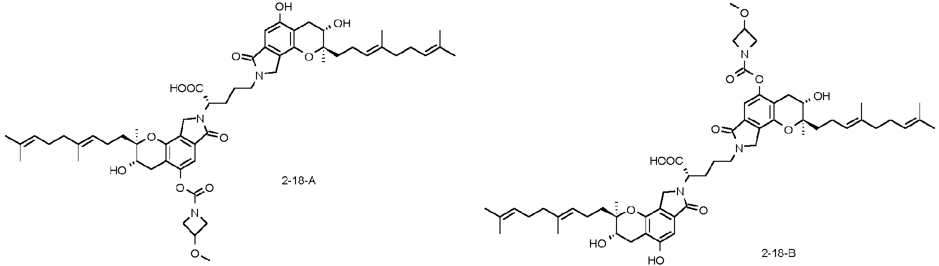

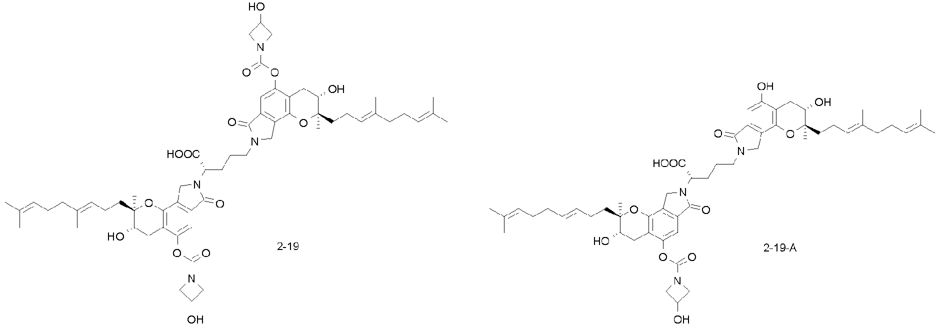

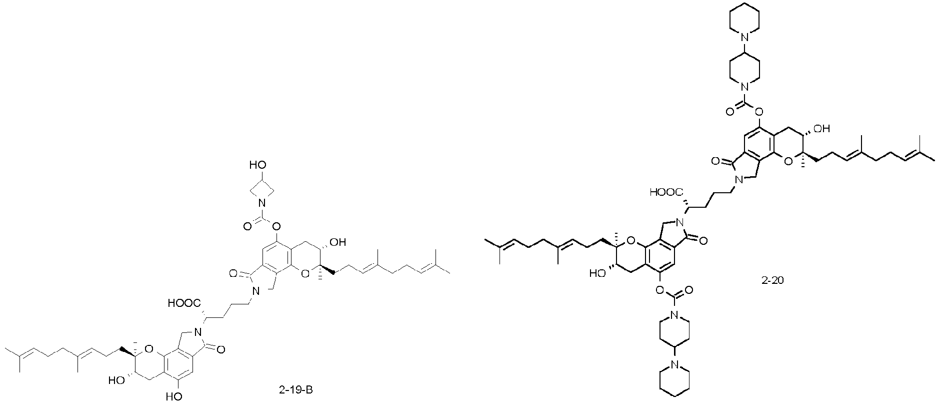

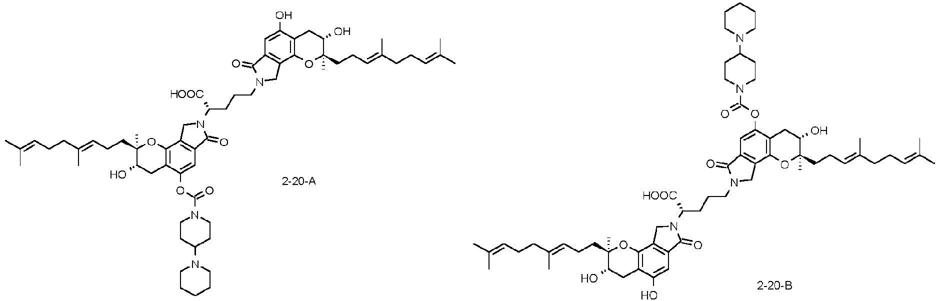

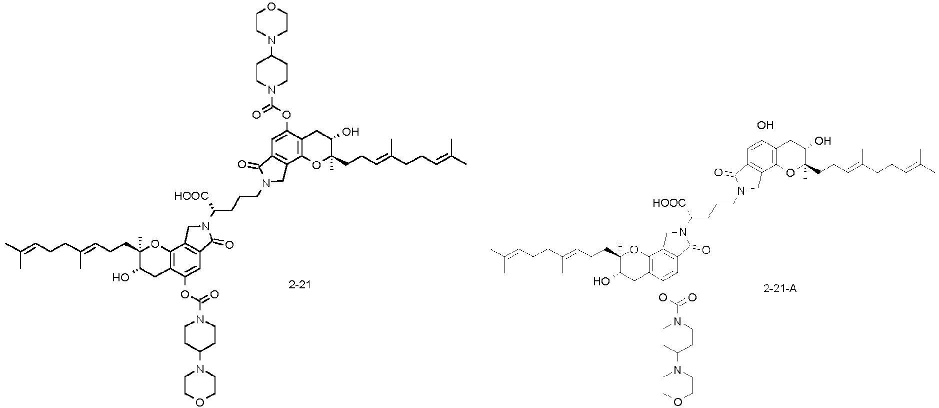

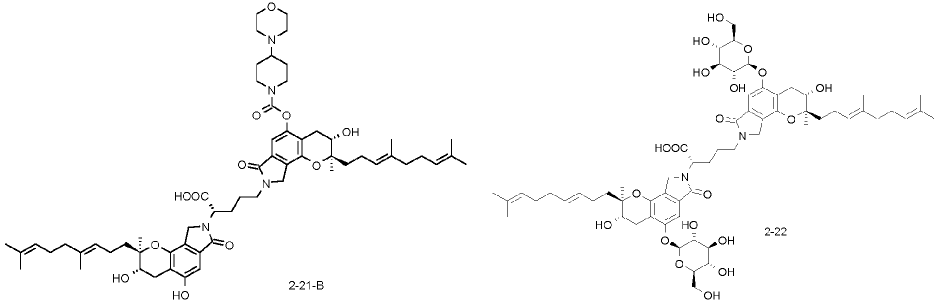

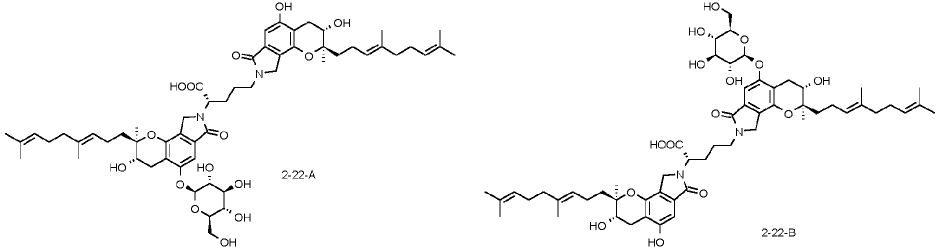

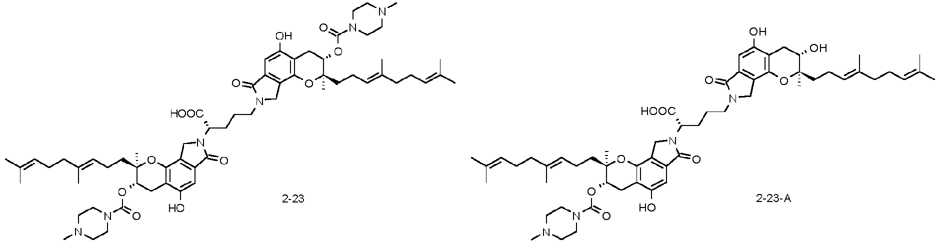

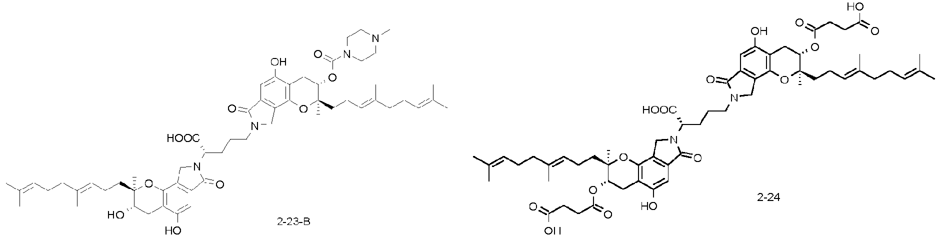

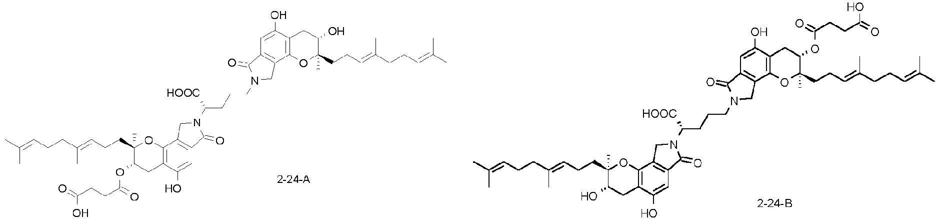

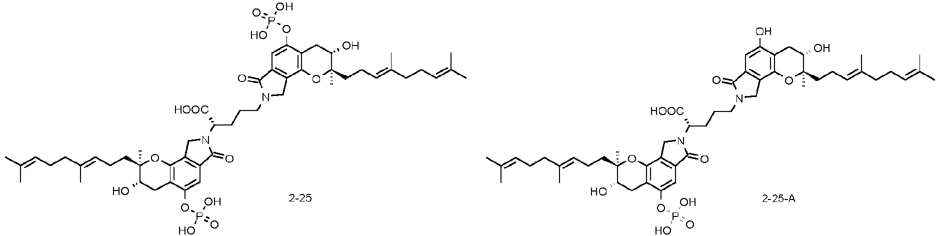

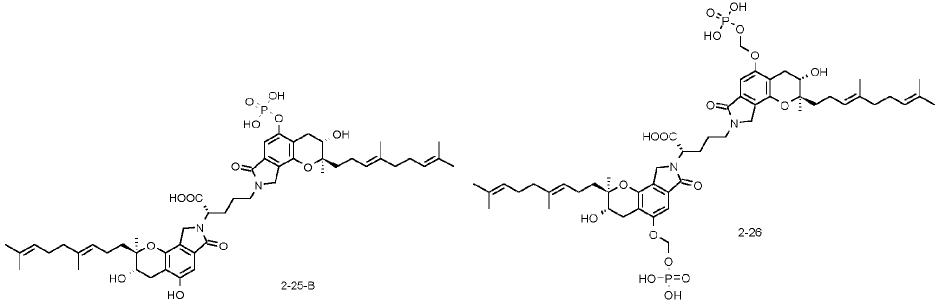

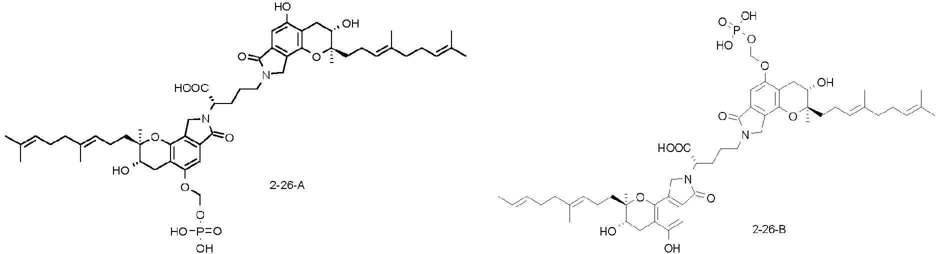

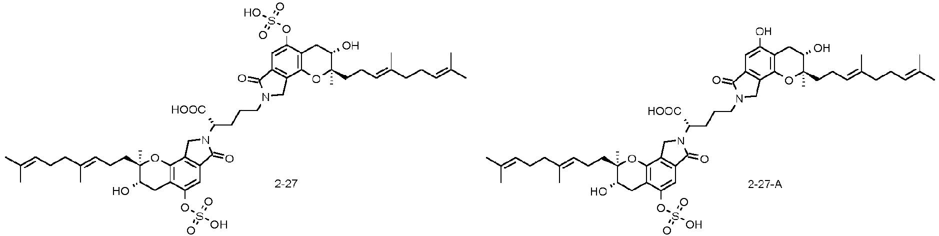

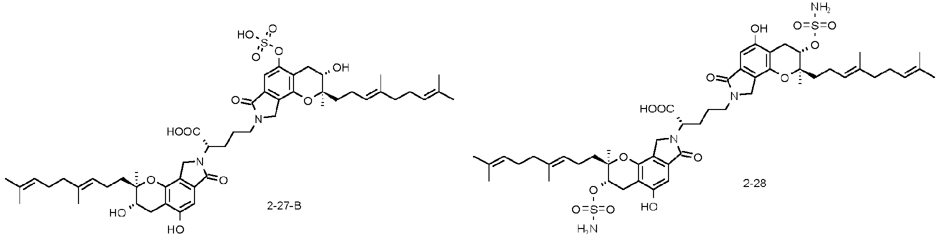

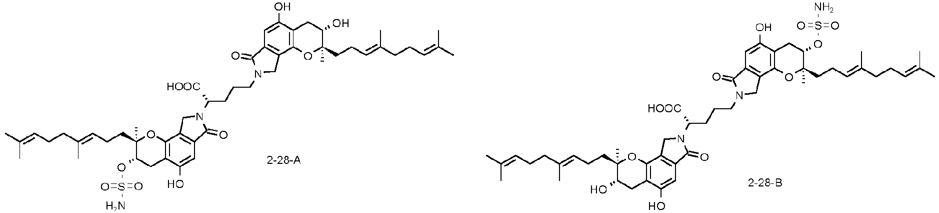

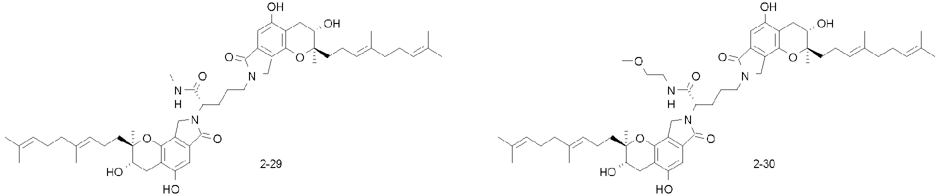

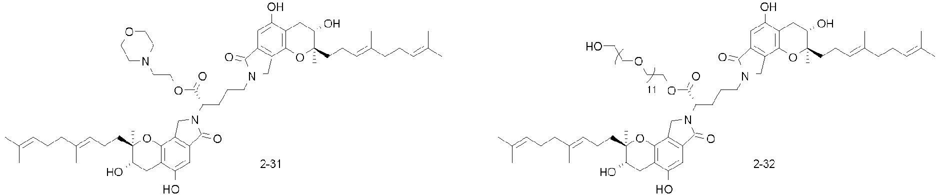

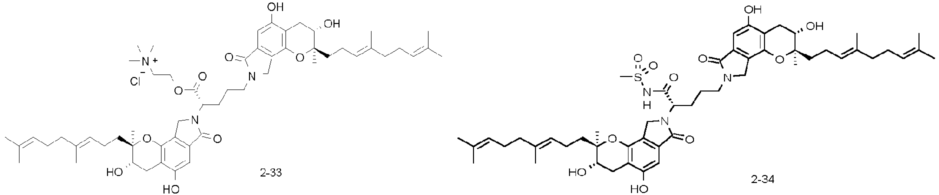

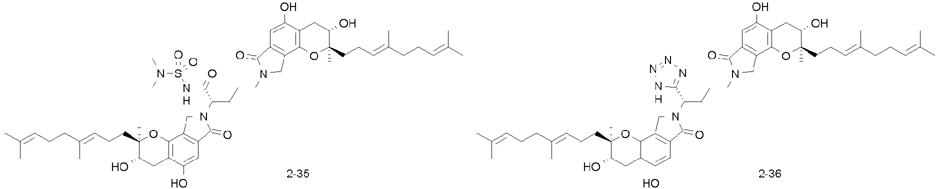

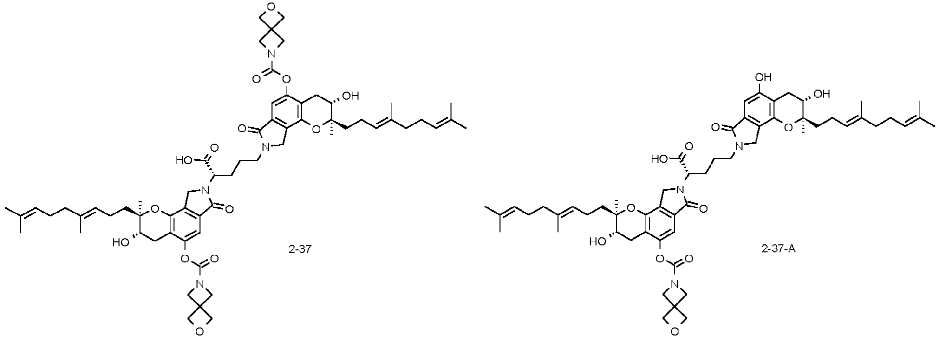

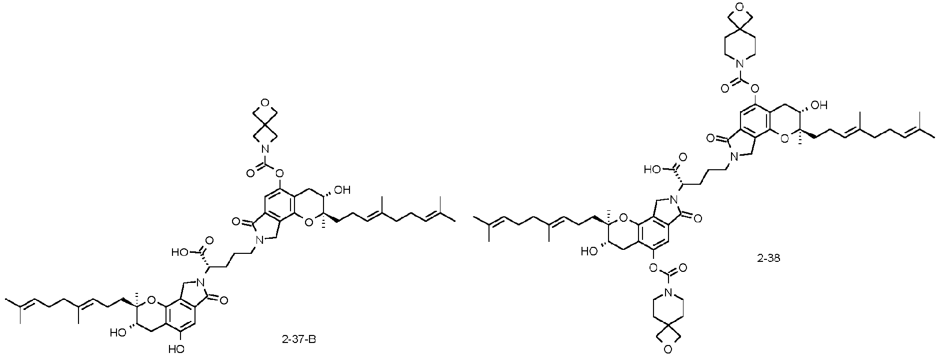

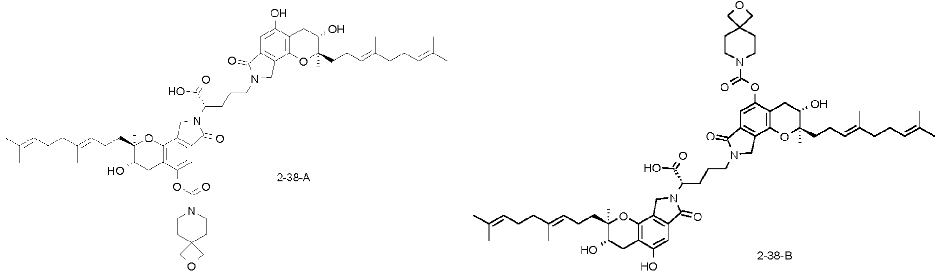

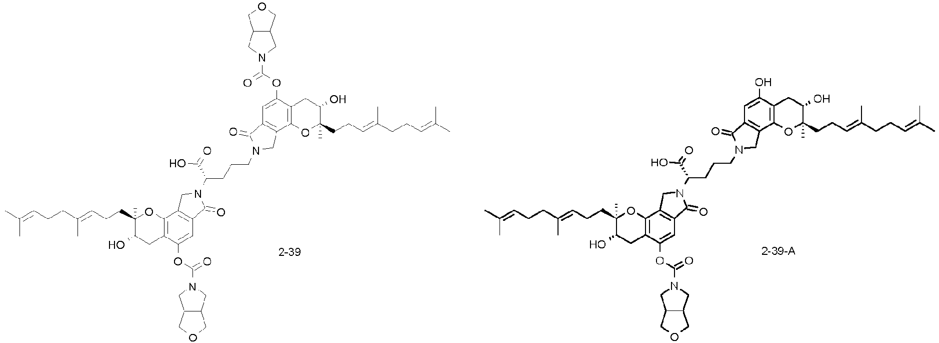

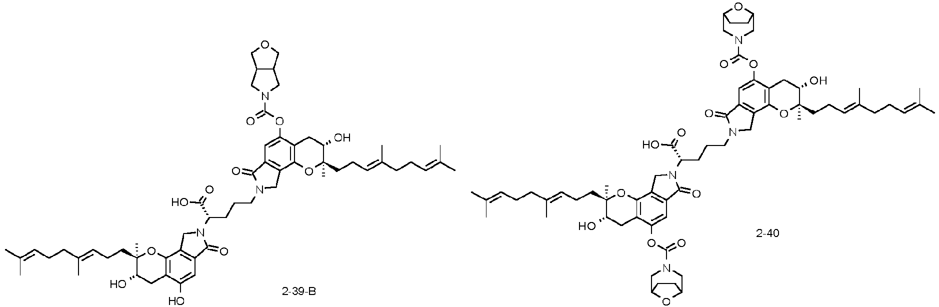

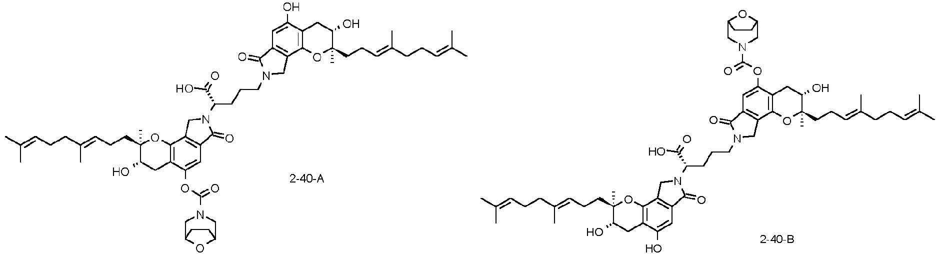

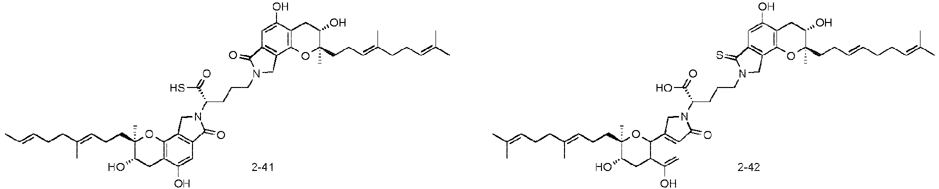

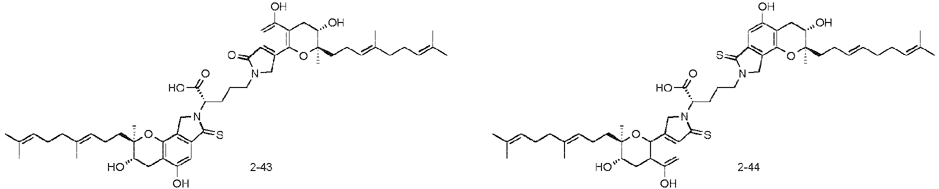

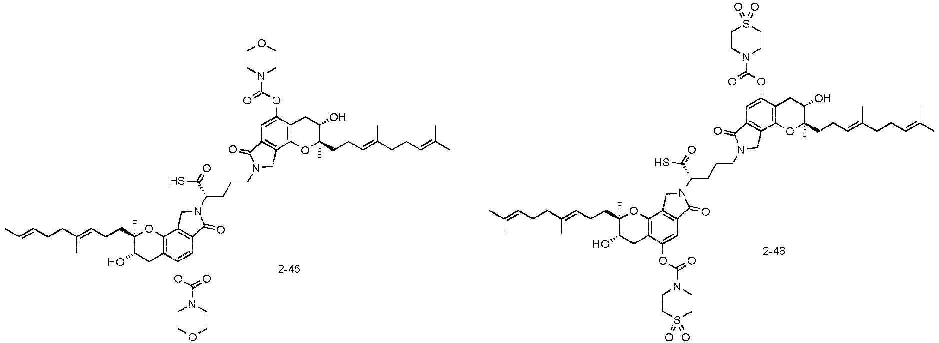

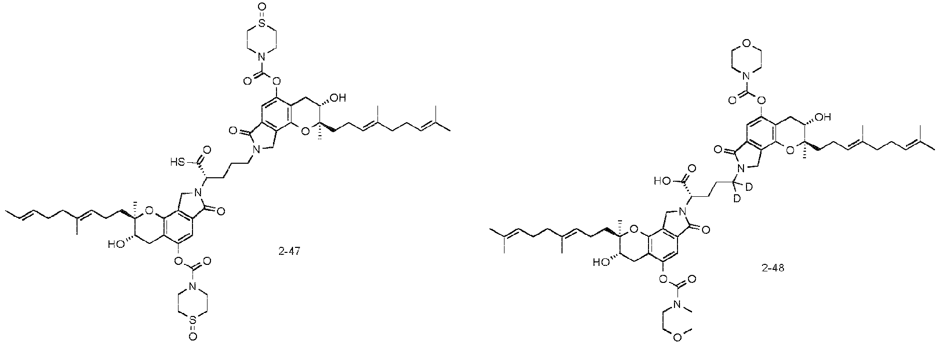

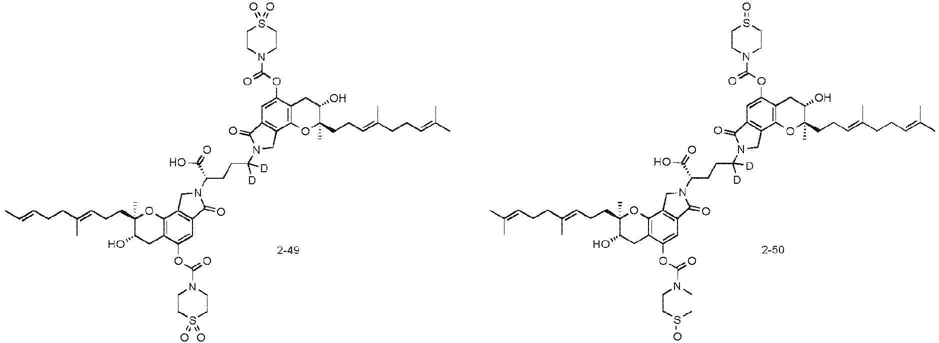

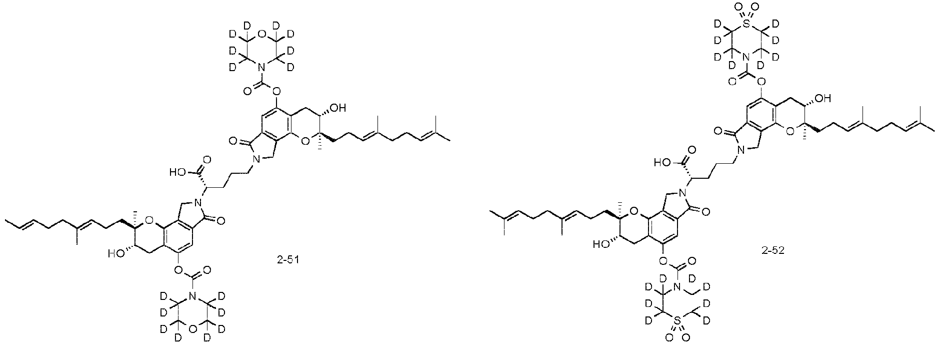

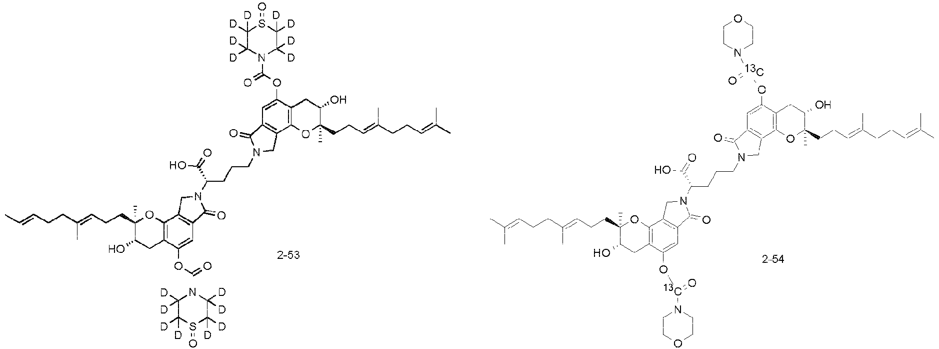

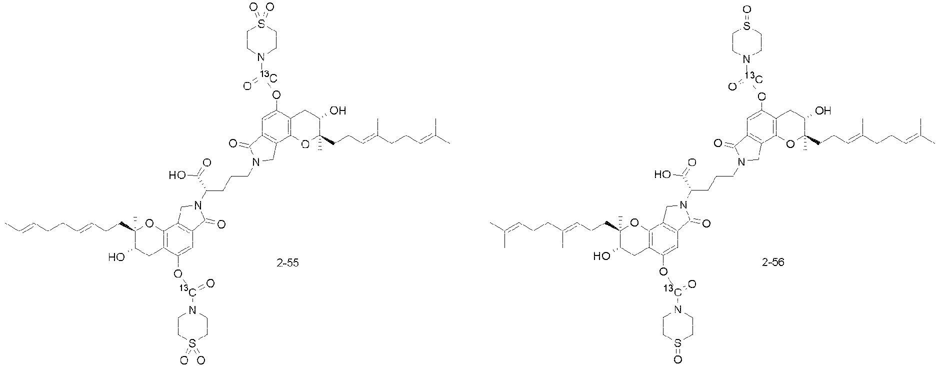

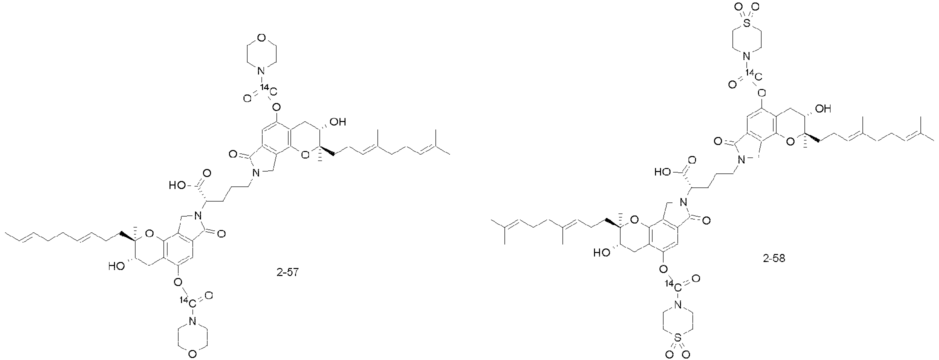

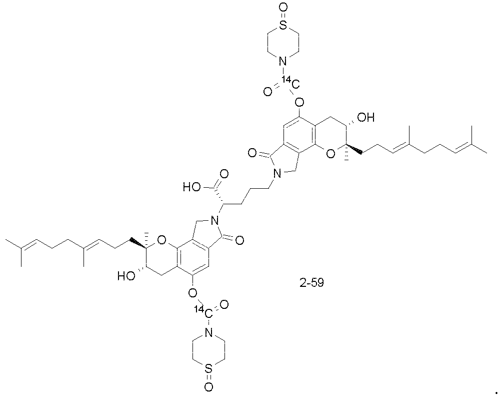

16. A pharmaceutical composition comprising a therapeutically effective amount of the compound according to any one of claims 1-15, or a pharmaceutically acceptable salt, an ester, a solvate, a stereoisomer, a tautomer, a polymorph, an isotopically labeled product, a metabolite, a prodrug, or a mixture thereof, and one or more pharmaceutically acceptable carriers thereof, and optionally further comprising one or more additional drugs for the treatment of thromboembolic diseases.

17. Use of the compound according to claims 1-15, or a pharmaceutically acceptable salt, an ester, a solvate, a stereoisomer, a tautomer, a polymorph, an isotopically labeled product, a metabolite, a prodrug, or a mixture thereof, or the pharmaceutical composition according to claim 16 in the manufacture of a medicament for the treatment of thromboembolic diseases.

18. The compound according to claims 1-15, or a pharmaceutically acceptable salt, an ester, a solvate, a stereoisomer, a tautomer, a polymorph, an isotopically labeled product, a metabolite, a prodrug, or a mixture thereof, or the pharmaceutical composition according to claim 16 for use in the treatment of thromboembolic diseasess.

19. A method of treating thromboembolic diseases comprising administering to a subject in need thereof a therapeutically effective amount of the compound according to claims 1-15, or a pharmaceutically acceptable salt, an ester, a solvate, a stereoisomer, a tautomer, a polymorph, an isotopically labeled product, a metabolite, a prodrug, or a mixture thereof, or the pharmaceutical composition according to claim 16.

20. A process for preparing the compound of formula I comprising the steps as shown in the following scheme: 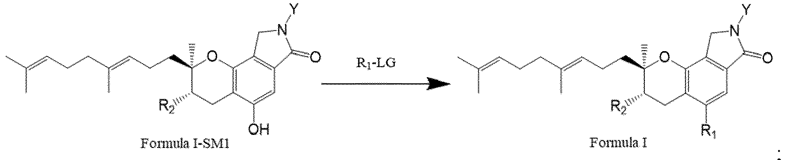 wherein:
R₁, R₂ and Y are as defined in any one of claims 1-15; and
LG is a leaving group.
